# EUROPEAN PATENT APPLICATION

(11) **EP 3 998 255 A1**
(43) Date of publication of application: **18.05.2022**
(21) Application number: 20835516.4
(22) Date of filing: 22.06.2020
(51) Int. Cl.: C07D 249/12, C07D 233/68, C07D 233/90, A61K 31/4164, A61K 31/4196, A61P 29/00, A61P 25/28, A61P 37/00

(54) **SMALL-MOLECULE REGULATOR OF TLR8**

(30) Priority: 01.07.2019 CN 201910585582
(71) Applicant: Tsinghua University, Haidian District, Beijing 100084 (CN)
(72) Inventor: YIN, Hang Hubert, Beijing 100084 (CN); JIANG, Shuangshuang, Beijing 100084 (CN)
(74) Representative: Algemeen Octrooi- en Merkenbureau B.V.
(86) International application number: PCT/CN2020/097340
(87) International publication number: WO 2021/000755

(57) **Abstract**

Provided in the present disclosure is a small-molecule regulator of TLR8; a compound of formula (I) or the pharmaceutically acceptable salt thereof provided in the present disclosure has good TLR8 regulatory activity and can be used to prevent or treat diseases mediated by TLR8.

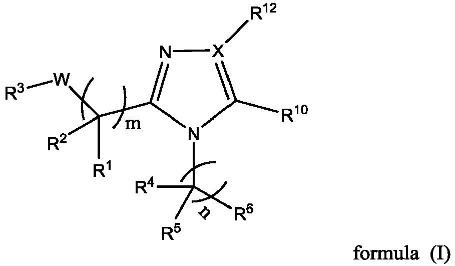

## Description

### TECHNICAL FIELD

The present disclosure relates to a small-molecule regulator of TLR8, which belongs to the field of medicinal chemistry.

### BACKGROUND

Toll-like receptor (TLR) family is a class of highly conserved transmembrane proteins, which play a very important role in innate immunity and acquired immunity and are the first barrier of the body resisting the invasion of pathogen(s) and eliminating the waste of the body. Currently, 10 homologous receptors have been found in the human body, namely TLR1 to TLR10. Among them, TLR1, TLR2, TLR4, TLR5, TLR6 and TLR10 are distributed on the surface of cell membrane, and TLR3, TLR7, TLR8 and TLR9 are distributed in the endosomes of cells. TLRs recognize ligands via homodimerization or heterodimerization. For example, after TLR8 recognizes the single-stranded RNA of a virus or a bacterium, the downstream signaling pathways would be activated to produce pro-inflammatory factors and chemokines such as tumor necrosis factor, interleukin-6 and interleukin-1β, thereby regulating the immune response of the body.

Researches have indicated that various types of diseases may be resulted from the dysregulation of TLR signaling pathway. For example, the dysregulation of Toll-like receptor 8 signaling pathway may cause rheumatoid arthritis, inflammatory bowel disease, Alzheimer's syndrome, systemic lupus erythematosus, Adult Still's disease, and the like. Therefore, Toll-like receptor 8 is an important target for the treatment of many diseases. The development of specific regulators of TLR8, especially small-molecule regulators, faces greater challenge due to the following reasons, i.e., TLR7 and TLR8 are both present in endosomes, belong to the same subfamily and have high degree of similarity in structure and function; both of them are capable of recognizing the single-stranded RNA of a virus or a bacterium, imidazoquinoline compounds and nucleotide analogs; and the interface for protein-protein interaction is relatively large and flexible.

Prior art such as the patent application WO2019089648A1 of UNIV COLORADO REGENTS, the patent application WO2018089695A1 of DYNAVAX TECH CORPORATION, the patent application WO2018233648A1 of MEDSHINE DISCOVERY INC, the patent application AU2017202755C1 of GILEAD SCIENCES, the patent application US20180258045A1 of UNIVERSITY OF KANSAS all disclose a series of compounds that may act as regulators of TLR8. In view of the fact that the small-molecule regulators of TLR8 have great potential medicinal value, there is an urgent need to develop compounds capable of specifically regulating TLR8.

### SUMMARY

The present disclosure provides a compound of formula (I) or a pharmaceutically acceptable salt thereof, which may be used as a small-molecule regulator of TLR8, 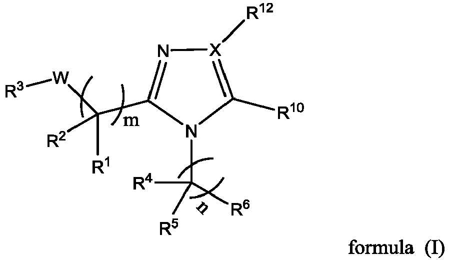 wherein,
X is CH or N; W is O or CH₂; m is 0, 1, 2, 3, 4, 5 or 6; n is 0, 1, 2, 3, 4, 5 or 6;
R¹ and R² are each independently H, C₁-C₆ alkyl, or halogen, wherein said C₁-C₆ alkyl is optionally substituted by one or two or three substituents selected from halogen, hydroxyl, cyano, amino, or nitro;
R³ is H, C₁-C₆ alkyl, C₁-C₆ alkoxy, or C₁-C₆ alkylacyl, wherein said C₁-C₆ alkyl, C₁-C₆ alkoxy or C₁-C₆ alkylacyl is optionally substituted by one or two or three substituents selected from halogen, hydroxyl, cyano, amino, or nitro;
R⁴ and R⁵ are each independently H, C₁-C₆ alkyl, or 3-6 membered cycloalkyl, wherein said C₁-C₆ alkyl or 3-6 membered cycloalkyl is optionally substituted by one or two or three substituents selected from halogen, hydroxyl, cyano, amino, or nitro;
R⁶ is phenyl, pyridyl, naphthyl, quinolyl, isoquinolyl, 3-6 membered cycloalkyl, or CR⁷R⁸R⁹, said phenyl, pyridyl, naphthyl, quinolyl, isoquinolyl or 3-6 membered cycloalkyl is optionally substituted by one or two or three substituents selected from H, C₁-C₆ alkyl, C₁-C₆ alkenyl, C₁-C₆ alkynyl, C₁-C₆ alkoxy, 3-6 membered cycloalkyl, trifluoromethyl, halogen, hydroxyl, cyano, amino, or nitro;
R⁷ and R⁸ are each independently H, C₁-C₆ alkyl, or 3-6 membered cycloalkyl, wherein said C₁-C₆ alkyl or 3-6 membered cycloalkyl is optionally substituted by one or two or three substituents selected from halogen, hydroxyl, cyano, amino, or nitro;
R⁹ is OH or H;
R¹⁰ is phenyl, pyridyl, naphthyl, quinolyl, biphenyl, 3-6 membered cycloalkyl, or SR¹¹, said phenyl, pyridyl, naphthyl, quinolyl, biphenyl or 3-6 membered cycloalkyl is optionally substituted by one or two or three substituents selected from H, C₁-C₆ alkyl, C₁-C₆ alkenyl, C₁-C₆ alkynyl, C₁-C₆ alkoxy, 3-6 membered cycloalkyl, halogen, hydroxyl, cyano, amino, or nitro;
R¹¹ is H, C₁-C₆ alkyl, C₁-C₆ alkenyl, C₁-C₆ alkynyl, C₁-C₆ alkoxy, 3-6 membered cycloalkyl, benzyl, or amide group, wherein said C₁-C₆ alkyl, C₁-C₆ alkenyl, C₁-C₆ alkynyl, C₁-C₆ alkoxy, 3-6 membered cycloalkyl or benzyl is optionally substituted by one or two or three substituents selected from C₁-C₆ alkyl, C₁-C₆ alkenyl, C₁-C₆ alkynyl, C₁-C₆ alkoxy, 3-6 membered cycloalkyl, phenyl, halogen, hydroxyl, cyano, amino, amide group, or nitro;
R¹² is H, C₁-C₆ alkyl, C₁-C₆ alkenyl, C₁-C₆ alkynyl, C₁-C₆ alkoxy, C₁-C₆ alkylacyl, 3-6 membered cycloalkyl, phenyl, halogen, hydroxyl, cyano, amino, nitro, amidino, amide group, aldehyde group, ester group, or carboxyl group, wherein said C₁-C₆ alkyl, C₁-C₆ alkenyl, C₁-C₆ alkynyl, C₁-C₆ alkoxy, C₁-C₆ alkylacyl, 3-6 membered cycloalkyl or phenyl is optionally substituted by one or two or three substituents selected from C₁-C₆ alkyl, C₁-C₆ alkenyl, C₁-C₆ alkynyl, C₁-C₆ alkoxy, C₁-C₆ alkylacyl, 3-6 membered cycloalkyl, halogen, hydroxyl, cyano, amino, or nitro.

In some embodiments, X is CH. In some embodiments, X is N. In some embodiments, W is O. In some embodiments, W is CH₂. In some embodiments, m is 0. In some embodiments, m is 1. In some embodiments, m is 2. In some embodiments, m is 3. In some embodiments, m is 4. In some embodiments, m is 5. In some embodiments, n is 0. In some embodiments, n is 1.

In some embodiments, R¹ and R² are each independently H, C₁-C₃ alkyl, fluorine, chlorine, bromine, or iodine, wherein said C₁-C₃ alkyl is optionally substituted by one or two or three substituents selected from fluorine, chlorine, bromine, iodine, hydroxyl, cyano, amino, or nitro.

In some embodiments, R¹ and R² are each independently H, methyl, ethyl, n-propyl, isopropyl, fluorine, chlorine, bromine, or iodine, wherein said methyl, ethyl, n-propyl or isopropyl is optionally substituted by one or two or three substituents selected from fluorine, chlorine, bromine, iodine, hydroxyl, cyano, amino, or nitro.

In some embodiments, R¹ and R² are each independently H, methyl, ethyl, n-propyl, isopropyl, fluorine, or chlorine.

In some typical embodiments, R¹ is H, R² is H.

In some embodiments, R³ is H, C₁-C₃ alkyl, C₁-C₃ alkoxy, or C₁-C₃ alkylacyl, wherein said C₁-C₃ alkyl, C₁-C₃ alkoxy or C₁-C₃ alkylacyl is optionally substituted by one or two or three substituents selected from fluorine, chlorine, bromine, iodine, hydroxyl, cyano, amino, or nitro.

In some embodiments, R³ is H, methyl, ethyl, n-propyl, isopropyl, acetyl, or propionyl, wherein said methyl, ethyl, n-propyl, isopropyl, acetyl or propionyl is optionally substituted by one or two or three substituents selected from fluorine, chlorine, bromine, iodine, hydroxyl, cyano, amino, or nitro.

In some embodiments, R³ is H, methyl, ethyl, n-propyl, isopropyl, acetyl, or propionyl. In some typical embodiments, R³ is H. In some typical embodiments, R³ is methyl. In some typical embodiments, R³ is ethyl.

In some typical embodiments, R³ is acetyl.

In some embodiments, R⁴ and R⁵ are each independently H, C₁-C₃ alkyl, or 3-6 membered cycloalkyl, wherein said C₁-C₃ alkyl or 3-6 membered cycloalkyl is optionally substituted by one or two or three substituents selected from fluorine, chlorine, bromine, iodine, hydroxyl, cyano, amino, or nitro.

In some embodiments, R⁴ and R⁵ are each independently H, methyl, ethyl, n-propyl, isopropyl, cyclopropyl, cyclobutyl, cyclopentyl, or cyclohexyl, wherein said methyl, ethyl, n-propyl, isopropyl, cyclopropyl, cyclobutyl, cyclopentyl, or cyclohexyl is optionally substituted by one or two or three substituents selected from fluorine, chlorine, bromine, iodine, hydroxyl, cyano, amino, or nitro.

In some embodiments, R⁴ and R⁵ are each independently H, methyl, ethyl, n-propyl, isopropyl, cyclopentyl, or cyclohexyl.

In some typical embodiments, R⁴ is H, R⁵ is H.

In some embodiments, R⁶ is phenyl, pyridyl, naphthyl, quinolyl, isoquinolyl, 3-6 membered cycloalkyl, or CR⁷R⁸R⁹, said phenyl, pyridyl, naphthyl, quinolyl, isoquinolyl or 3-6 membered cycloalkyl is optionally substituted by one or two or three substituents selected from H, C₁-C₃ alkyl, C₁-C₃ alkenyl, C₁-C₃ alkynyl, C₁-C₃ alkoxy, 3-6 membered cycloalkyl, trifluoromethyl, fluorine, chlorine, bromine, iodine, hydroxyl, cyano, amino, or nitro.

In some typical embodiments, R⁶ is phenyl, 2-chlorophenyl, 3-chlorophenyl, 4-chlorophenyl, 3-fluorophenyl, 2,4-difluorophenyl, 2,4-dichlorophenyl, 2,3-dichlorophenyl, 3,4-dichlorophenyl, 2,4,5-trichlorophenyl, 2,3,4-trichlorophenyl, 3,4,5-trichlorophenyl, 3,4,5-trimethylphenyl, 4-methoxyphenyl, 3-methylphenyl, 3-trifluoromethylphenyl, 4-trifluoromethylphenyl, 3-pyridyl, cyclohexyl, 1-naphthyl, 3-quinolyl, 6-quinolyl, 2-hydroxyprop-2-yl, or isopropyl.

In some more typical embodiments, R⁶ is 3,4,5-trimethylphenyl. In some more typical embodiments, R⁶ is 2-hydroxyprop-2-yl. In some more typical embodiments, R⁶ is isopropyl.

In some embodiments, R⁷ and R⁸ are each independently H, C₁-C₃ alkyl, or 3-6 membered cycloalkyl, wherein said C₁-C₃ alkyl or 3-6 membered cycloalkyl is optionally substituted by one or two or three substituents selected from fluorine, chlorine, bromine, iodine, hydroxyl, cyano, amino, or nitro.

In some embodiments, R⁷ and R⁸ are each independently H, methyl, ethyl, n-propyl, isopropyl, cyclopentyl, or cyclohexyl.

In some typical embodiments, R⁷ is methyl, R⁸ is methyl. In some embodiments, R⁹ is OH. In some embodiments, R⁹ is H.

In some embodiments, R¹⁰ is phenyl, pyridyl, naphthyl, quinolyl, biphenyl, 3-6 membered cycloalkyl, or SR¹¹, said phenyl, pyridyl, naphthyl, quinolyl, biphenyl or 3-6 membered cycloalkyl is optionally substituted by one or two or three substituents selected from H, C₁-C₃ alkyl, C₁-C₃ alkenyl, C₁-C₃ alkynyl, C₁-C₃ alkoxy, 3-6 membered cycloalkyl, fluorine, chlorine, bromine, iodine, hydroxyl, cyano, amino, or nitro.

In some embodiments, R¹⁰ is phenyl, p-methylphenyl, pyridyl, naphthyl, quinolyl, biphenyl, mercapto, methylthio, ethylthio, isopropylthio, (cyclopropylmethyl)thio, 2,6-dichlorobenzylthio, formamidethio, or acetamidethio.

In some typical embodiments, R¹⁰ is phenyl. In some typical embodiments, R¹⁰ is methylthio.

In some embodiments, R¹¹ is H, C₁-C₃ alkyl, C₁-C₃ alkenyl, C₁-C₃ alkynyl, C₁-C₃ alkoxy, 3-6 membered cycloalkyl, benzyl, or amide group, wherein said C₁-C₃ alkyl, C₁-C₃ alkenyl, C₁-C₃ alkynyl, C₁-C₃ alkoxy, 3-6 membered cycloalkyl or benzyl is optionally substituted by one or two or three substituents selected from C₁-C₃ alkyl, C₁-C₃ alkenyl, C₁-C₃ alkynyl, C₁-C₃ alkoxy, 3-6 membered cycloalkyl, fluorine, chlorine, bromine, iodine, hydroxyl, cyano, amino, amide group, or nitro.

In some typical embodiments, R¹¹ is H, methyl, ethyl, isopropyl, cyclopropylmethyl, carboxamide group, acetamide group, or 2,6-dichlorobenzyl. In some more typical embodiments, R¹¹ is methyl.

In some embodiments, R¹² is H, C₁-C₃ alkyl, C₁-C₃ alkenyl, C₁-C₃ alkynyl, C₁-C₃ alkoxy, C₁-C₃ alkylacyl, 3-6 membered cycloalkyl, phenyl, fluorine, chlorine, bromine, iodine, hydroxyl, cyano, amino, nitro, amidino, amide group, aldehyde group, ester group, or carboxyl group, wherein said C₁-C₃ alkyl, C₁-C₃ alkenyl, C₁-C₃ alkynyl, C₁-C₃ alkoxy, C₁-C₃ alkylacyl, 3-6 membered cycloalkyl or phenyl is optionally substituted by one or two or three substituents selected from C₁-C₃ alkyl, C₁-C₃ alkenyl, C₁-C₃ alkynyl, C₁-C₃ alkoxy, C₁-C₃ alkylacyl, 3-6 membered cycloalkyl, fluorine, chlorine, bromine, iodine, hydroxyl, cyano, amino, or nitro.

In some typical embodiments, R¹² is H, cyano, amidino, amide group, aldehyde group, methoxycarbonyl, hydroxymethyl, methoxymethyl, methyl, fluoromethyl, 2-hydroxyprop-2-yl, isopropyl, phenyl, chlorine, bromine, iodine, or carboxyl group.

In some more typical embodiments, R¹² is iodine.

In some embodiments, the above-mentioned formula (I) has the structure represented by formula (II), 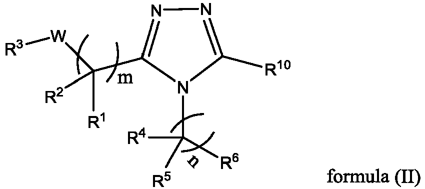 wherein R¹, R², R³, R⁴, R⁵, R⁶, R¹⁰, W, m and n are as defined in the above-mentioned compound of formula (I).

In some embodiments, the above-mentioned formula (I) has the structure represented by formula (III), 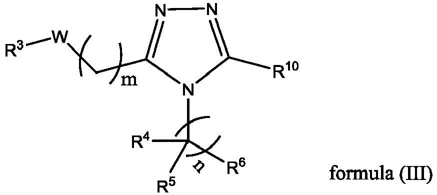 wherein R³, R⁴, R⁵, R⁶, R¹⁰, W, m and n are as defined in the above-mentioned compound of formula (I).

In some embodiments, the above-mentioned formula (I) has the structure represented by formula (IV), 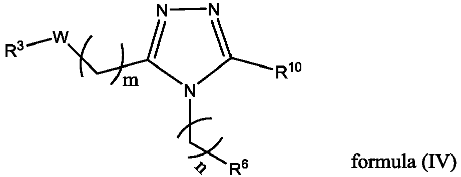 wherein R³, R⁶, R¹⁰, W, m and n are as defined in the above-mentioned compound of formula (I).

In some embodiments, the above-mentioned formula (I) has the structure represented by formula (V), 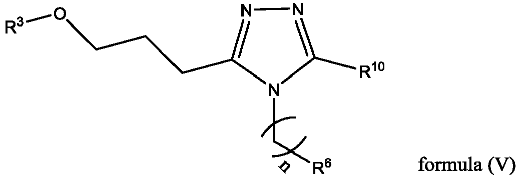 wherein R³, R⁶, R¹⁰ and n are as defined in the above-mentioned compound of formula (I).

In some embodiments, the above-mentioned formula (I) has the structure represented by formula (VI), 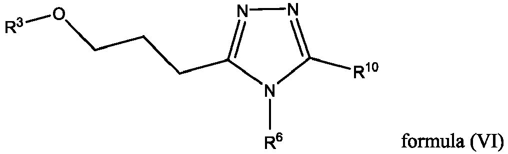 wherein R³, R⁶ and R¹⁰ are as defined in the above-mentioned compound of formula (I).

In some embodiments, the above-mentioned formula (I) has the structure represented by formula (VII), 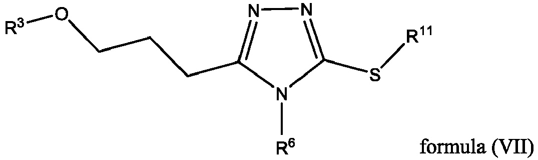 wherein R³, R⁶ and R¹¹ are as defined in the above-mentioned compound of formula (I).

In some embodiments, the above-mentioned formula (I) has the structure represented by formula (VIII), 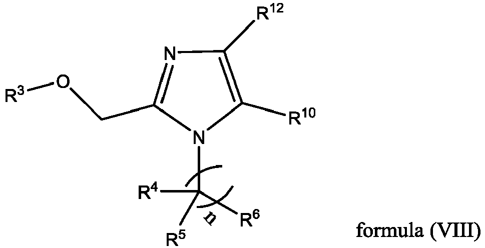 wherein R³, R⁴, R⁵, R⁶, R¹⁰, R¹² and n are as defined in the above-mentioned compound of formula (I).

In some embodiments, the above-mentioned formula (I) has the structure represented by formula (IX), 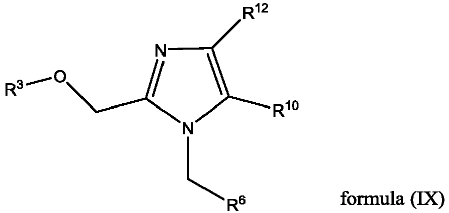 wherein R³, R⁶, R¹⁰ and R¹² are as defined in the above-mentioned compound of formula (I).

In some embodiments, the above-mentioned formula (I) has the structure represented by formula (X), 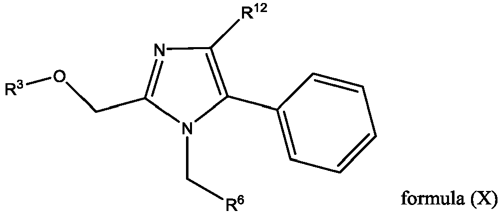 wherein R³, R⁶ and R¹² are as defined in the above-mentioned compound of formula (I).

In some embodiments, the compounds involved in the present disclosure are selected from the following compounds or the pharmaceutically acceptable salts thereof.

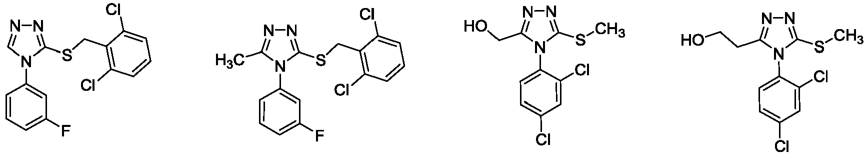

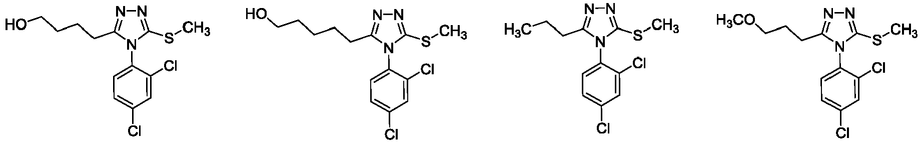

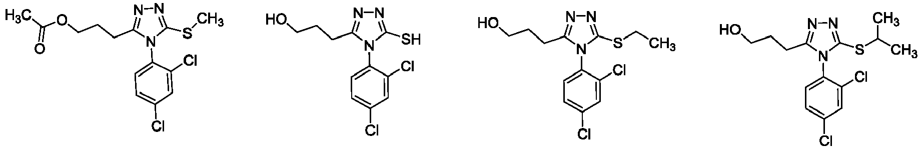

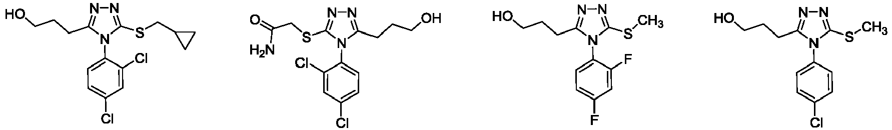

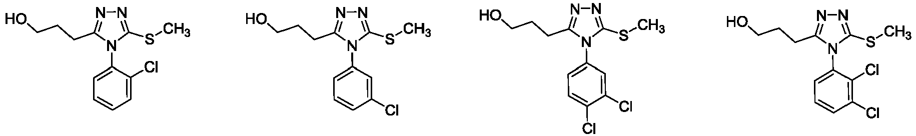

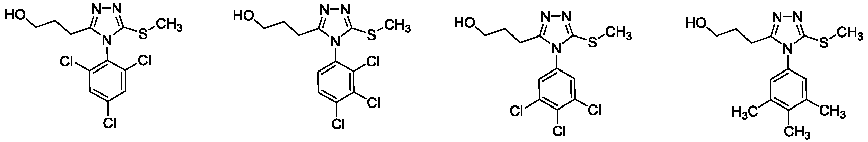

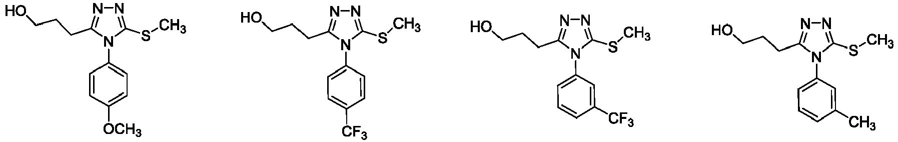

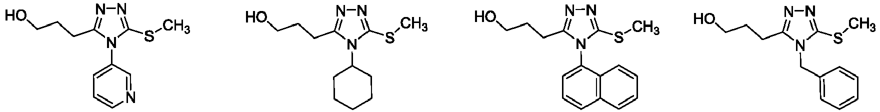

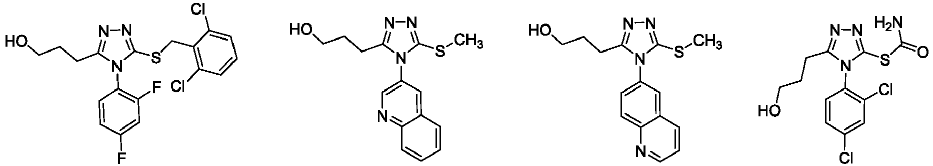

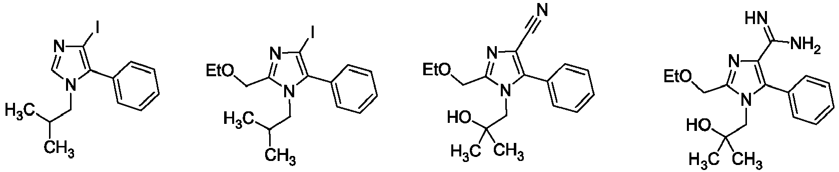

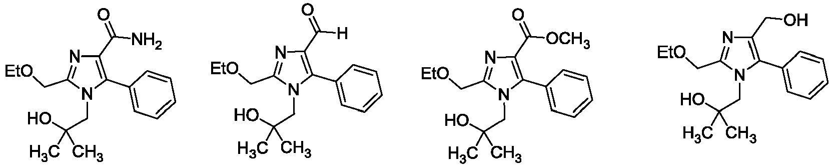

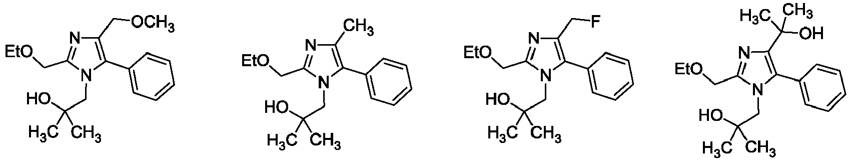

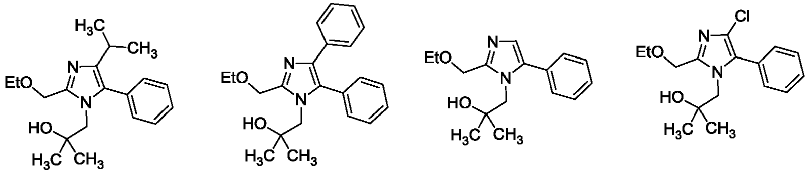

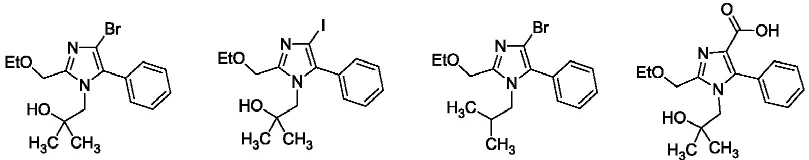

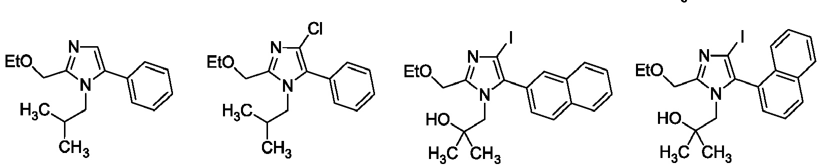

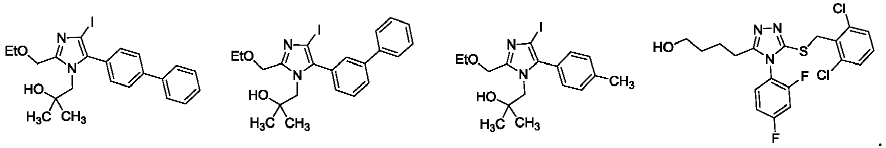

In another aspect, the present disclosure provides a pharmaceutical composition comprising the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X) of the present disclosure or a pharmaceutically acceptable salt thereof.

In some embodiments, it comprises a therapeutically effective amount of the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X) of the present disclosure or a pharmaceutically acceptable salt thereof.

In some other embodiments, the pharmaceutical composition of the present disclosure further comprises a pharmaceutically acceptable excipient.

The pharmaceutical composition of the present disclosure may be prepared by combining the compound of the present disclosure with a suitable pharmaceutically acceptable excipient, for example, may be formulated into a solid preparation, a semi-solid preparation, a liquid preparation or a gaseous preparation, such as a tablet, a pill, a capsule, a powder, a granule, an ointment, an emulsion, a suspension, a suppository, an injection, an inhalant, a gel, a microsphere and an aerosol.

Typical routes for administering the compound of the present disclosure or a pharmaceutically acceptable salt thereof or a pharmaceutical composition thereof include but are not limited to oral administration, rectal administration, topical administration, inhalation administration, parenteral administration, sublingual administration, intravaginal administration, intranasal administration, intraocular administration, intraperitoneal administration, intramuscular administration, subcutaneous administration, and intravenous administration.

The pharmaceutical composition of the present disclosure may be manufactured by methods well known in the art, such as a conventional mixing method, a dissolution method, a granulation method, a method of preparing dragee, a grinding method, an emulsification method, and a freeze-drying method.

In some embodiments, the pharmaceutical composition is in oral form. As for oral administration, the pharmaceutical composition may be formulated by mixing an active compound with a pharmaceutically acceptable excipient well known in the art. These excipients enable the compound of the present disclosure to be formulated into a tablet, a pill, a lozenge, a dragee, a capsule, a liquid, a gel, a syrup, a suspension and the like for oral administration to patients.

A solid oral composition may be prepared by a conventional mixing, filling or tableting method. For example, a solid oral composition may be obtained by the following method. The active compound is mixed with solid excipients, optionally, the resulting mixture is grinded, other suitable excipients are added if necessary, and then this mixture is processed into granules to yield the cores of tablets or dragees. Suitable excipients include but are not limited to a binder, a diluent, a disintegrant, a lubricant, a glidant, a sweetener, a flavoring agent, or the like.

The pharmaceutical composition may also be suitable for parenteral administration, for example, as a sterile solution, suspension or lyophilized product in a suitable unit dosage form.

In another aspect, the present disclosure relates to a method for treating diseases mediated by TLR8 receptor, comprising administering a therapeutically effective amount of the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X), or a pharmaceutically acceptable salt thereof, or a pharmaceutical composition thereof to a mammal in need of this treatment, preferably human.

In all administration methods of the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X) of the present disclosure, the daily dose is from 0.01 to 100mg/kg body weight, preferably from 0.05 to 50mg/kg body weight, and more preferably from 0.1 to 5mg/kg body weight, in the form of single dose or separate dose.

In another aspect, the present disclosure relates to use of the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X), or a pharmaceutically acceptable salt thereof or a pharmaceutical composition thereof in preparation of a medicament for preventing or treating a disease mediated by TLR8 receptor.

In another aspect, the present disclosure relates to the compound of formula (I), or the compound of formula (II), or the compound of formula (III), or the compound of formula (IV), or the compound of formula (V), or the compound of formula (VI), or the compound of formula (VII), or the compound of formula (VIII), or the compound of formula (IX), or the compound of formula (X) or a pharmaceutically acceptable salt thereof for preventing or treating a disease mediated by TLR8 receptor.

Said diseases mediated by TLR8 receptor include rheumatoid arthritis, inflammatory bowel disease, Alzheimer's syndrome, systemic lupus erythematosus, Adult Still's disease, or the like.

The compound of the present disclosure may be prepared by a variety of synthetic methods well known to those skilled in the art, including the specific embodiments exemplified below, the embodiments formed by combining these specific embodiments with other chemical synthetic methods, and equivalent alternatives well known to those skilled in the art. Preferred embodiments include but are not limited to the examples of the present disclosure.

The chemical reactions of the specific embodiments of the present disclosure are conducted in suitable solvents, said solvents should be suitable for the chemical changes of the present disclosure and the reagents and materials as required. In order to obtain the compound of the present disclosure, it is sometimes necessary for those skilled in the art to modify or select the synthetic steps or reaction schemes based on the existing embodiments.

### Definitions

Unless otherwise specified, the following terms used in the present disclosure have the following meanings. A specific term should not be considered as indefinite or ambiguous in a case where the term is not specifically defined, and should be understood as its ordinary meaning in the art. When a trade name appears herein, it is intended to refer to the corresponding commercial product or the active ingredient thereof.

The term "substituted" means that any one or more hydrogen atoms on a specific atom are replaced by a substituent, as long as the valence of the specific atom is normal and the substituted compound is stable. When the substituent is an oxo (i.e., =O), it means that two hydrogen atoms are substituted, and an oxo would not appear on an aromatic group.

The term "optional" or "optionally" means that the event or circumstance described subsequently may or may not occur, and such description includes both the case where said event or circumstance occurs and the case where said event or circumstance does not occur. For example, an ethyl group being "optionally" substituted by halogen means that the ethyl group may be unsubstituted (CH₂CH₃), monosubstituted (e.g., CH₂CH₂F), polysubstituted (e.g., CHFCH₂F, CH₂CHF₂, or the like), or fully substituted (CF₂CF₃). As could be understood by those skilled in the art, as for any group comprising one or more substituents, any substitution or substitution pattern that is sterically impossible to exist and/or cannot be synthesized would not be introduced therein.

Herein, "C₁-C₆" means that the group may comprise 1 carbon atom, 2 carbon atoms, 3 carbon atoms, 4 carbon atoms, 5 carbon atoms, or 6 carbon atoms, and "C₁-C₃" means that the group may comprise 1 carbon atom, 2 carbon atoms, or 3 carbon atoms.

When any variable (for example, R) occurs once or more in the composition or structure of a compound, the definition of the variable in each case is independent. Therefore, for example, if a group is substituted with two Rs, then each R has independent options; for another example, in a structural unit 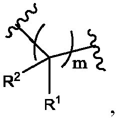 when m≥2, R¹ and R² in each repeating unit have independent options; for another example, in a structural unit 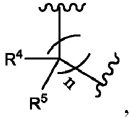 when n≥2, R⁴ and R⁵ in each repeating unit have independent options.

The term "halo" or "halogen" refers to fluorine, chlorine, bromine and iodine. The term "hydroxy" refers to -OH group. The term "cyano" refers to -CN group. The term "amino" refers to -NH₂ group. The term "nitro" refers to -NO₂ group.

The term "2-hydroxyprop-2-yl" refers to

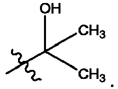

The term "hydroxymethyl" refers to -CH₂OH.

The term "2,6-dichlorobenzlthio" refers to

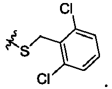

The term "formamidethio" refers to

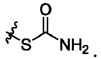

The term "acetamidethio" refers to

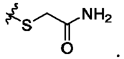

The term "alkyl" refers to a hydrocarbon group with a general formula of CₙH₂ₙ₊₁. This alkyl may be linear or branched. For example, the term "C₁-C₆ alkyl" refers to an alkyl group comprising 1 to 6 carbon atoms (such as methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). Similarly, the alkyl portions (i.e., alkyl) of alkoxy and alkylacyl have the same definition as above.

The term "alkenyl" refers to a linear or branched unsaturated aliphatic hydrocarbon group that is composed of carbon atoms and hydrogen atoms and has at least one double bond. Non-limiting examples of an alkenyl group include but are not limited to ethenyl, 1-propenyl, 2-propenyl, 1-butenyl, isobutenyl, 1,3-butadienyl, and the like.

The term "alkynyl" refers to a linear or branched unsaturated aliphatic hydrocarbon group that is composed of carbon atoms and hydrogen atoms and has at least one triple bond. Non-limiting examples of an alkynyl group include but are not limited to ethynyl (-C≡CH), 1-propynyl (-C≡C-CH₃), 2-propynyl (-CH₂-C≡CH), 1,3-butadiynyl (-C=C-C=CH), and the like.

The term "alkoxy" refers to -O- alkyl.

The term "cycloalkane" refers to a carbocyclic ring that is fully saturated and may exist as a monocyclic ring, a bridged ring or a spirocyclic ring. Unless otherwise indicated, the carbocyclic ring is generally a 3-10 membered ring, a 3-6 membered ring, a 3-5 membered ring, or a 3-4 membered ring. Non-limiting examples of an cycloalkyl group include but are not limited to cyclopropyl, cyclobutyl, cyclopentyl, cyclohexyl, bicyclo[2.2.2]octyl, adamantyl, and the like.

The term "treating" means administering the compound or preparation of the present disclosure to prevent, ameliorate or eliminate a disease or one or more symptoms associated with the disease, and comprises:
(i) preventing a disease or a disease state from occurring in a mammal, especially when such mammal is susceptible to the disease state but has not yet been diagnosed as having the disease state;
(ii) inhibiting a disease or a disease state, that is, restraining its development; and
(iii) alleviating a disease or a disease state, that is, causing the regression of the disease or the disease state.

The term "therapeutically effective amount" refers to the amount of the compound of the present disclosure used for (i) treating or preventing a specific disease, condition or disorder, (ii) alleviating, ameliorating or eliminating one or more symptoms of a specific disease, condition or disorder, or (iii) preventing or delaying the onset of one or more symptoms of a specific disease, condition or disorder as described herein. The amount of a compound of the present disclosure that constitutes the "therapeutically effective amount" varies depending on this compound, the disease state and its severity, the mode of administration and the age of the mammal to be treated, while it may be routinely determined by those skilled in the art based on their own knowledge and the content of the present disclosure.

The term "pharmaceutically acceptable" is intended to refer to those compounds, materials, compositions and/or dosage forms which, within the scope of reliable medical judgment, are suitable for use in contact with the tissues of human beings and animals without excessive toxicity, irritation, allergic response, or other problems or complications, and commensurate with a reasonable benefit/risk ratio.

As a pharmaceutically acceptable salt, for example, a metal salt, an ammonium salt, a salt formed with an organic base, a salt formed with an inorganic acid, a salt formed with an organic acid, a salt formed with an alkaline or acidic amino acid and the like may be mentioned.

The term "pharmaceutical composition" refers to a mixture of one or more compounds of the present disclosure or the salts thereof and pharmaceutically acceptable excipients. A pharmaceutical composition is intended to facilitate the administration of a compound of the present disclosure to an organism.

The term "pharmaceutically acceptable excipient" refers to those excipients which do not cause significant irritation to an organism and do not impair the biological activity and properties of this active compound. Suitable excipients are well known to those skilled in the art, such as carbohydrates, waxes, water-soluble and/or water-swellable polymers, hydrophilic or hydrophobic materials, gelatin, oils, solvents, water, and the like.

In the present disclosure, unless otherwise defined, the solvent ratio of the eluent used for column chromatography is a volume ratio.

TLR: Toll-like receptor; TNF-α: tumor necrosis factor; IL-6: interleukin-6; IL-1β: interleukin-1β; WST1: Cell Proliferation and Cytotoxicity Assay Kit; R848: Resiquimod; ssRNA40: single-stranded nucleic acid; ORN06: single-stranded nucleic acid; ELISA: enzyme-linked immunosorbent assay; THP-1: Human myeloid leukemia monocyte; OD: optical density, i.e., absorbance; RPMI 1640: cell culture medium; DMEM: high-glucose cell culture medium; FBS: fetal bovine serum; PBMC: peripheral blood mononuclear cell; Et: ethyl; CSCl₂: thiophosgene; DMSO: dimethyl sulfoxide; DMF: N,N-dimethylformamide; DMA: N,N-dimethylacetamide; TBDMSOTf: tert-butyldimethylsilyl triflate; n-BuLi: n-butyllithium; THF: tetrahydrofuran; K₂CO₃: potassium carbonate; Na₂CO₃: sodium carbonate; CHCl₃: chloroform; NaOH: sodium hydroxide; CH₃CN: acetonitrile; CH₃I: iodomethane; DAST: (diethylamino)sulfur trifluoride; NCS: N-chlorosuccinimide; NBS: N-bromosuccinimide; NIS: N-iodosuccinimide; TBAF: tetrabutylammonium fluoride

### BRIEF DESCRIPTION OF THE DRAWINGS

Figure 1 shows the inhibitory effect on R848-induced signaling and the inhibitory effect on ORN 06-induced signaling of Compound **24** in HEK-Blue hTLR8 cell line.
Figure 2 shows the results of the toxicity test of Compound **24** in HEK-Blue hTLR8 cell line.
Figure 3 shows the specific inhibitory effect of Compound **24** in HEK-Blue hTLR1/2, TLR2/6, TLR4, TLR5, TLR7, TLR8, and TLR9 cell lines.
Figure 4 shows the inhibitory effect of Compound **24** on TNF-α in TLR1/2, TLR2/6, TLR3, TLR4 or TLR5 signaling pathways in human peripheral blood mononuclear cells.
Figure 5 shows the inhibitory effect of Compound **24** on the inflammatory factor TNF-α in R848-induced signaling pathway in THP-1 cells.
Figure 6 shows the inhibitory effect of Compound **24** on the inflammatory factor IL-6 in R848-induced signaling pathway in THP-1 cells.
Figure 7 shows the inhibitory effect of Compound **24** on the inflammatory factor IL-1β in R848-induced signaling pathway in THP-1 cells.
Figure 8 shows the inhibitory effects of Compound **24** and Reference Compound 10 on the inflammatory factor TNF-α in R848-induced signaling pathway in human peripheral blood mononuclear cells.
Figure 9 shows the inhibitory effect of Compound **24** on the inflammatory factor TNF-α in R848-induced signaling pathway in peripheral blood mononuclear cells of patients suffering from rheumatoid arthritis.
Figure 10 shows the inhibitory effect of Compound **53** on R848-induced signaling in HEK-Blue hTLR8 cell line and the effect of Compound **53** on the viability of HEK-Blue hTLR8 cells.
Figure 11 shows the specific inhibitory effect of Compound **53** in HEK-Blue hTLR1/2, TLR2/6, TLR3, TLR4, TLR5, TLR7, TLR8 and TLR9 cell lines.
Figure 12 shows the inhibitory effect of Compound **53** on R848-induced signaling and the synergistic activating effect of Compound **53** on ssRNA40-induced signaling and ORN06-induced signaling in HEK-Blue hTLR8 cell line.
Figure 13 shows the activating effect of Reference Compound R848 on TLR8 as well as the synergistic activating effect of Reference Compound R848 with ssRNA40 and ORN06 on TLR8.
Figure 14 shows the synergistic activating effect of Compounds 60 to 64 on ssRNA40-activated TLR8 signaling in HEK-Blue hTLR8 cell line.
Figure 15 shows the results of the toxicity tests of Compounds 60 to 64 in HEK-Blue hTLR8 cell line.

### DETAILED DESCRIPTION

The present disclosure is described in detail below by Examples, but it is not intended to impose any disadvantageous limitation on the present disclosure. The present disclosure has been described in detail herein, and its specific embodiments are also disclosed therein. Various changes and improvements made to the specific embodiments of the present disclosure without departing from the spirit and scope of the present disclosure will be apparent to those skilled in the art.

### Example 1: Synthesis of 3-((2,6-dichlorobenzyl)thio)-4-(3-fluorophenyl)-4H-1,2,4-triazole (Compound 1)

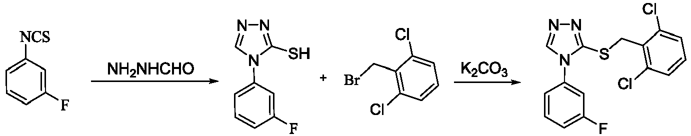

3-Fluoro-1-isothiocyanatobenzene compound (500 mg, 3.26 mmol) was weighed, formylhydrazine (196 mg, 3.26 mmol) was added, 8 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic with a pH of approximately 5, and a large amount of white solid appeared in the reaction system. The resultant was subjected to suction filtration, washed with water and then subjected to vacuum drying, so as to obtain 4-(3-fluorophenyl)-4H-1,2,4-triazole-3-thiol as a milky white solid (286 mg, yield: 45%). ¹H NMR (400MHz, DMSO-*d*₆) δ 14.01 (s, 1H), 8.75 (s, 1H), 7.68 (dt, *J* = 10.0, 2.3 Hz, 1H), 7.64-7.58 (m, 1H), 7.56 (ddd, *J* = 8.1, 1.9, 1.2 Hz, 1H), 7.41-7.35 (m, 1H).

4-(3-Fluorophenyl)-4H-1,2,4-triazole-3-thiol (100 mg, 0.51 mmol) was weighed, potassium carbonate solid (212 mg, 1.54 mmol) was added, 2,6-dichlorobenzyl bromide (129 mg, 0.54 mmol) was added, 8 mL of acetone was added, and the resulting mixture was reacted overnight at room temperature under stirring by a rotor. After rotary evaporation, the resultant was subjected to silica gel column chromatography (petroleum ether : ethyl acetate = 2 : 1), so as to obtain 3-((2,6-dichlorobenzyl)thio)-4-(3-fluorophenyl)-4H-1,2,4-triazole as a white solid (120 mg, yield: 66%). ¹H NMR (400MHz, DMSO-*d*₆) δ 8.99 (s, 1H), 7.74-7.66 (m, 1H), 7.57 (td, *J* = 8.2, 6.3 Hz, 2H), 7.46-7.35 (m, 3H), 7.35-7.26 (m, 1H), 4.41 (s, 2H). HRMS (ESI) calcd for C₁₅H₁₀Cl₂FN₃S[M+H]⁺: 354.0029; found: 354.0023.

### Example 2: Synthesis of 3-((2,6-dichlorobenzyl)thio)-4-(3-fluorophenyl)-5-methyl-4H-1,2,4-triazole (Compound 2)

3-Fluoro-1-isothiocyanatobenzene (500 mg, 3.26 mmol) was weighed, acetylhydrazine (242 mg, 3.26 mmol) and 8 mL of absolute ethanol were successively added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic with a pH of approximately 5, and a large amount of white solid appeared. The resultant was subjected to suction filtration, washed with water and then subjected to vacuum drying, so as to obtain 4-(3-fluorophenyl)-5-methyl-4H-1,2,4-triazole-3-thiol as a white solid (307 mg, yield: 45%). ¹H NMR (400 MHz, DMSO-*d*₆) δ 13.70 (s, 1H), 7.63 (td, *J* = 8.1, 6.4 Hz, 1H), 7.48-7.38 (m, 2H), 7.32 (ddd, *J* = 7.9, 1.9, 0.9 Hz, 1H), 2.13 (s, 3H).

4-(3-Fluorophenyl)-5-methyl-4H-1,2,4-triazole-3-thiol (100 mg, 0.48 mmol) was weighed, potassium carbonate solid (197 mg, 1.43 mmol) was added, 2,6-dichlorobenzyl bromide (120 mg, 0.50 mmol) was added, 8 mL of acetone was added, and the resulting mixture was reacted overnight at room temperature under stirring by a rotor. After rotary evaporation, the resultant was subjected to silica gel column chromatography(petroleum ether : ethyl acetate = 2 : 1), so as to obtain 3-((2,6-dichlorobenzyl)thio)-4-(3-fluorophenyl)-5-methyl-4H-1,2,4-triazole as a white solid (138 mg, yield: 78%). ¹H NMR (400MHz, chloroform-d) δ 7.42 (td, J = 8.2, 6.0 Hz, 1H), 7.19 (d, J = 8.4 Hz, 2H), 7.14 (tdd, J = 8.4, 2.5, 0.9 Hz, 1H), 7.08 (dd, J = 8.8, 7.3 Hz, 1H), 6.94 (ddd, J = 7.9, 1.9, 0.9 Hz, 1H), 6.82 (dt, J = 8.7, 2.3 Hz, 1H), 4.56 (s, 2H), 2.28 (s, 3H). HRMS (ESI) calcd for C₁₆H₁₂Cl₂FN₃S [M+H]⁺: 368.0186; found: 368.0183.

### Example 3: Synthesis of (4-(2,4-dichlorophenyl)-5-(methylthio)-4H-1,2,4-triazole-3-yl) methanol (Compound 3)

Methyl glycolate (200 mg, 2.22 mmol) was weighed and added to a 25-mL round-bottom flask, hydrazine monohydrate (166.7 mg, 2.66 mmol, purity: 80%) was added, 10 mL of absolute ethanol was added, and the resulting mixture was heated under reflux for 3h, so as to obtain 2-hydroxyacetylhydrazine as a white solid (160 mg, yield: 80%). ¹H NMR (400 MHz, DMSO-*d*₆) δ 8.83 (s, 1H), 4.55 (s, 2H), 4.34-4.00 (m, 1H), 3.90-3.78 (m, 2H).

The synthesized 2,4-dichloro-1-isothiocyanatobenzene compound (350 mg, 1.71 mmol) was weighed, 2-hydroxyacetylhydrazine (200 mg, 2.22 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain (4-(2,4-dichlorophenyl)-5-mercapto-4H-1,2,4-triazole-3-yl)methanol as a white solid (235 mg, yield: 50%).

(4-(2,4-Dichlorophenyl)-5-mercapto-4H-1,2,4-triazole-3-yl)methanol (235 mg, 0.855 mmol) was weighed, potassium carbonate solid (276 mg, 2 mmol) was added, 8 mL of acetone was added, iodomethane (80 µL, 1.28 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain (4-(2,4-dichlorophenyl)-5-(methylthio)-4H-1,2,4-triazole-3-yl)methanol as a white solid powder (198 mg, yield: 81%). ¹H NMR (400 MHz, DMSO-*d*₆) δ 7.97 (t, J = 1.3 Hz, 1H), 7.66 (d, J = 1.3 Hz, 2H), 5.36 (t, J = 5.7 Hz, 1H), 4.38 (m, 2H), 2.57 (s, 3H). ¹³C NMR (101 MHz, DMSO-*d*₆) δ 155.68, 152.41, 136.40, 133.21, 132.06, 130.48, 130.31, 129.27, 54.13, 15.23. HRMS (ESI) calcd for C₁₀H₉Cl₂N₃OS [M+H]⁺: 289.9922; found: 289.9909.

### Example 4: Synthesis of 2-(4-(2,4-dichlorophenyl)-5-(methylthio)-4H-1,2,4-triazole-3-yl)ethan-1-ol (Compound 4)

β-Propiolactone (300 mg, 4.16 mmol) was added to a 25-mL round-bottom flask, sodium methoxide (337.1 mg, 6.24 mmol) was added, and the resulting mixture was heated under reflux in 10 mL of diethyl ether solution for 1h. Afterwards, hydrazine monohydrate (395 mg, 6.24 mmol, purity: 80%) was added and the resultant was refluxed overnight. 10 µL of sample was taken and dried via rotary evaporation. 2,4-dichloro-1-isothiocyanatobenzene compound (204 mg, 1.00 mmol) was further added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 2-(4-(2,4-dichlorophenyl)-5-mercapto-4H-1,2,4-triazole-3-yl)ethan-1-ol as a white solid (147 mg, yield: 48%). ¹H NMR (400 MHz, DMSO-*d*₆) δ 13.84 (s, 1H), 7.98 (d, *J* = 2.3 Hz, 1H), 7.69 (dd, *J* = 8.5, 2.3 Hz, 1H), 7.62 (d, *J* = 8.6 Hz, 1H), 4.78 (t, *J* = 5.6 Hz, 1H), 3.53 (dq, *J* = 8.5, 2.9 Hz, 2H), 2.58 (dd, *J* = 15.3, 6.6 Hz, 1H), 2.45 (dt, *J* = 15.3, 6.6 Hz, 1H). ¹³C NMR (101 MHz, DMSO-*d*₆) δ 168.17, 150.54, 136.13, 133.68, 133.32, 130.82, 130.45, 129.23, 58.16, 29.60.

2-(4-(2,4-Dichlorophenyl)-5-mercapto-4H-1,2,4-triazole-3-yl)ethan-1-ol (116 mg, 0.38 mmol) was weighed, potassium carbonate solid (138 mg, 1.00 mmol) was added, 8 mL of acetone was added, iodomethane (40 µL, 0.72 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 2-(4-(2,4-dichlorophenyl)-5-(methylthio)-4H-1,2,4-triazole-3-yl)ethan-1-ol as a white solid powder (198 mg, yield: 81%). ¹H NMR (400 MHz, DMSO-*d*₆) δ 8.02 (s, 1H), 7.70 (s, 2H), 4.72 (t, *J* = 5.5 Hz, 1H), 3.64-3.50 (m, 2H), 2.66 (dt, *J* = 13.9, 6.8 Hz, 2H), 2.54 (s, 3H).¹³C NMR (101 MHz, DMSO-*d*₆) δ 154.08, 151.19, 136.59, 133.17, 132.34, 130.70, 130.11, 129.55, 59.04, 28.94, 15.34. HRMS (ESI) calcd for C₁₁H₁₁Cl₂N₃OS [M+H]⁺: 304.0078; found: 304.0070.

### Example 5: Synthesis of 4-(4-(2,4-dichlorophenyl)-5-(methylthio)-4H-1,2,4-triazole-3-yl)butan-1-ol (Compound 5)

2,4-Dichloro-1-isothiocyanatobenzene (200 mg, 0.977 mmol) was weighed and charged into a 25-mL round-bottom flask, 5-hydroxyvaleric acid hydrazine (181 mg, 1.37 mmol) was added, 8 mL of absolute ethanol was added, and the resulting mixture was refluxed overnight. The ethanol solution was evaporated to dryness via rotary evaporation, 8 mL of 2M aqueous sodium hydroxide solution was then added immediately, and the resulting mixture was refluxed overnight. After the reaction system was cooled, 2M aqueous HCl solution was added slowly until the solution became acidic, and a large amount of white solid appeared. The resultant was subjected to suction filtration, washed with water and then subjected to vacuum drying, so as to obtain 4-(4-(2,4-dichlorophenyl)-5-mercapto-4H-1,2,4-triazole-3-yl)butan-1-ol as a white solid (108 mg, yield: 33%). ¹H NMR (400 MHz, DMSO-*d*₆) δ 13.81 (s, 1H), 7.98 (d, *J* = 2.2 Hz, 1H), 7.73-7.66 (m, 2H), 3.31 (s, 2H), 2.35 (td, *J* = 7.4, 3.2 Hz, 2H), 1.61-1.48 (m, 2H), 1.40 (dt, *J* = 8.6, 6.3 Hz, 2H).

4-(4-(2,4-Dichlorophenyl)-5-mercapto-4H-1,2,4-triazole-3-yl)butan-1-ol (100 mg, 0.302 mmol) was weighed, potassium carbonate solid (138 mg, 1 mmol) was added, 8 mL of acetonitrile was added, iodomethane (28 µL, 0.453 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 4-(4-(2,4-dichlorophenyl)-5-(methylthio) -4H-1,2,4-triazole-3-yl)butan-1-ol as a white solid powder (85 mg, yield: 85%). ¹H NMR (400 MHz, DMSO-*d*₆) δ 8.01 (d, *J* = 1.9 Hz, 1H), 7.75-7.67 (m, 2H), 4.32 (t, *J* = 5.1 Hz, 1H), 3.33 (m, 2H), 2.54 (s, 3H), 2.44 (t, *J* = 7.5 Hz, 2H), 1.61-1.50 (m, 2H), 1.44-1.33 (m, 2H).¹³C NMR (101 MHz, DMSO-*d*₆) δ 155.36, 150.51, 135.98, 132.62, 131.45, 130.24, 129.59, 129.10, 60.06, 31.32, 23.95, 22.76, 14.80.

### Example 6: Synthesis of 5-(4-(2,4-dichlorophenyl)-5-(methylthio)-4H-1,2,4-triazole-3-yl)pentan-1-ol (Compound 6)

ε-Caprolactone (500 mg, 4.38 mmol) was added to a 25-mL round-bottom flask, hydrazine monohydrate (329 mg, 5.26 mmol, purity: 80%) was added, 8 mL of ethanol was added, and the resulting mixture was refluxed overnight and dried via rotary evaporation to obtain 6-hydroxyhexanohydrazide as a crude product, with a yield of approximately 100%. The crude product (320 mg, 1.23 mmol) was weighed and charged into a new 25-mL round-bottom flask, 2,4-dichloro-1-isothiocyanatobenzene compound (324 mg, 1.58 mmol) was added, 8 mL of absolute ethanol was added, and the resulting mixture was refluxed overnight. The ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 5-(4-(2,4-dichlorophenyl)-5-mercapto-4H-1,2,4-triazole-3-yl)pentan-1-ol (crude product) as a white solid (315mg).

5-(4-(2,4-Dichlorophenyl)-5-mercapto-4H-1,2,4-triazole-3-yl)pentan-1-ol (314 mg, 0.95 mmol) was weighed, potassium carbonate solid (276 mg, 2.00 mmol) was added, 8 mL of acetone was added, iodomethane (89 µL, 1.42 mmol) was added dropwise under stirring by a rotor, and the resulting mixture was reacted overnight at room temperature. After rotary evaporation, the resultant was subjected to silica gel column chromatography (dichloromethane : methanol = 20 : 1 to 10 : 1), so as to obtain 5-(4-(2,4-dichlorophenyl)-5-(methylthio)-4H-1,2,4-triazole-3-yl)pentan-1-ol as a yellow oily liquid (280 mg, yield: 85%). ¹H NMR (400 MHz, DMSO-*d*₆) δ 8.01 (d, *J* = 2.1 Hz, 1H), 7.76-7.67 (m, 2H), 4.29 (t, *J* = 5.2 Hz, 1H), 3.33 (d, *J* = 5.5 Hz, 2H), 2.54 (s, 3H), 2.43 (t, *J* = 7.5 Hz, 2H), 1.50 (p, *J* = 7.2 Hz, 2H), 1.37-1.21 (m, 4H). ¹³C NMR (101MHz, DMSO-*d*₆) δ 155.85, 151.12, 136.60, 133.16, 132.11, 130.78, 130.15, 129.64, 60.93, 32.43, 26.52, 25.32, 24.85, 15.34. HRMS (ESI) calcd for C₁₄H₁₇Cl₂N₃OS [M+H]⁺: 346.0548; found: 346.0532.

### Example 7: Synthesis of 4-(2,4-dichlorophenyl)-3-(methylthio)-5-propyl-4H-1,2,4-triazole (Compound 7)

2,4-Dichloro-1-isothiocyanatobenzene compound (204 mg, 1.00 mmol) was weighed and charged into a 25-mL round-bottom flask, daminozide (102 mg, 1.00 mmol) was added, 8 mL of absolute ethanol was added, and the resulting mixture was refluxed overnight. The ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 4-(2,4-dichlorophenyl)-5-propyl-4H-1,2,4-triazole-3-thiol (crude product) as a white solid (108 mg, yield: 37%).

4-(2,4-Dichlorophenyl)-5-propyl-4H-1,2,4-triazole-3-thiol (100 mg, 0.95 mmol) was weighed, potassium carbonate solid (276 mg, 2.00 mmol) was added, 8 mL of acetone was added, iodomethane (89 µL, 1.42 mmol) was added dropwise under stirring by a rotor, and the resulting mixture was reacted overnight at room temperature. After rotary evaporation, the resultant was subjected to silica gel column chromatography (petroleum ether : acetone = 1 : 1), so as to obtain 4-(2,4-dichlorophenyl)-3-(methylthio)-5-propyl-4H-1,2,4-triazole as a white solid (50 mg, yield: 48%). ¹H NMR (400 MHz, DMSO-*d*₆) δ 8.03 (d, *J*=2.2 Hz, 1H), 7.75 (d, *J*=8.5 Hz, 1H), 7.71 (dd, *J*=8.5, 2.2 Hz, 1H), 2.55 (s, 3H), 2.42 (t, *J*=7.5 Hz, 2H), 1.54 (h, *J*=7.2 Hz, 2H), 0.86 (t, *J*=7.4 Hz, 3H); ¹³C NMR (101MHz, DMSO-*d*₆) δ 155.18, 150.61, 136.08, 132.64, 131.61, 130.28, 129.61, 129.19, 26.26, 19.62, 14.80, 13.40. HRMS (ESI) calcd for C₁₂H₁₃Cl₂N₃S[M+H]⁺: 302.0286; found: 302.0271.

### Example 8: Synthesis of 4-(2,4-dichlorophenyl)-3-(3-methoxypropyl)-5-(methylthio)-4H-1,2,4-triazole (Compound 8)

3-(4-(2,4-Dichlorophenyl)-5-(methylthio)-4H-1,2,4-triazole-3-yl)propan-1-ol (60 mg, 0.19 mmol) was added into a 25-mL round-bottom flask, 5 mL of anhydrous tetrahydrofuran was added, followed by the addition of sodium hydride (60% dispersed in mineral oil, 15 mg, 0.38 mmol), and the resulting mixture was reacted overnight at room temperature. 10 mL of water and dichloromethane (10 mL × 3) were added to conduct extraction. The organic phases were combined, washed with saturated brine, and dried over anhydrous sodium sulfate. Afterwards, the resultant was subjected to silica gel column chromatography (petroleum ether : acetone = 1 : 1), so as to obtain 4-(2,4-dichlorophenyl)-3-(3-methoxypropyl)-5-(methylthio)-4H-1,2,4-triazole as a white solid (58 mg, yield: 88%). ¹H NMR (400 MHz, chloroform-*d*) δ 7.60 (d, *J* = 2.3 Hz, 1H), 7.42 (dd, *J* = 8.4, 2.3 Hz, 1H), 7.22 (d, *J* = 8.3 Hz, 1H), 3.38 (t, *J* = 6.1 Hz, 2H), 3.22 (s, 3H), 2.63 (s, 3H), 2.52 (tt, *J* = 15.6, 7.6 Hz, 2H), 1.94 (p, *J* = 6.7 Hz, 2H). ¹³C NMR (101 MHz, chloroform-*d*) δ 155.75, 152.73, 137.38, 133.80, 131.01, 130.60, 129.81, 128.78, 71.30, 58.51, 26.71, 21.97, 15.04. HRMS (ESI) calcd for C₁₃H₁₅Cl₂N₃OS[M+H]⁺: 332.0391; found: 332.0387.

### Example 9: Synthesis of propyl 3-(4-(2,4-dichlorophenyl)-5-(methylthio)-4H-1,2,4-triazole-3-yl)acetate (Compound 9)

3-(4-(2,4-Dichlorophenyl)-5-(methylthio)-4H-1,2,4-triazole-3-yl)propan-1-ol (60 mg, 0.19 mmol) was added into a 25-mL round-bottom flask, 5 mL of anhydrous dichloromethane was added, acetic anhydride (19 mg, 0.19 mmol) and 4-dimethylaminopyridine (4.61 mg, 0.038 mmol) were added, and the resulting mixture was reacted overnight at room temperature. 10 mL of water and dichloromethane (10 mL × 3) were added to conduct extraction. The organic phases were combined, washed with saturated brine, and dried over anhydrous sodium sulfate. Afterwards, the resultant was subjected to silica gel column chromatography (petroleum ether : acetone = 1 : 1), so as to obtain propyl 3-(4-(2,4-dichlorophenyl)-5-(methylthio)-4H-1,2,4-triazole-3-yl)acetate as a white solid (58 mg, yield: 88%). ¹H NMR (400 MHz, chloroform-*d*) δ 7.66 (d, *J* = 2.2 Hz, 1H), 7.48 (dd, *J* = 8.4, 2.2 Hz, 1H), 7.28 (d, *J* = 1.9 Hz, 1H), 4.10 (t, *J* = 6.1 Hz, 2H), 2.68 (s, 3H), 2.59 (m, 2H), 2.10-2.02 (m, 2H), 2.00 (s, 3H). ¹³C NMR (101 MHz, chloroform-*d*) δ 170.86, 154.99, 152.26, 137.44, 133.67, 131.00, 130.45, 129.53, 128.78, 63.14, 25.83, 21.81, 20.86, 14.90. HRMS (ESI) calcd for C₁₄H₁₅Cl₂N₃O₂S[M+H]⁺: 360.0340; found: 360.0327.

### Example 10: Synthesis of 3-(4-(2,4-dichlorophenyl)-5-mercapto-4H-1,2,4-triazole-3-yl)propan-1-ol (Compound 10)

2,4-Dichloroaniline (1.52g, 9.39 mmol) was weighed and charged into a 25-mL single-neck flask. A magnetic stirring bar was added, 2,4-dichloroaniline was dissolved in 10 mL of anhydrous chloroform, sodium carbonate (1.27g, 11.98 mmol) was added, and the resulting mixture was stirred in an ice bath. Thiophosgene (660 µL, 8.66 mmol) was slowly added dropwise, and the resultant was reacted in an ice bath for 2h. A sample was taken and the complete reaction of raw materials was detected. 2M aqueous sodium hydroxide solution was slowly added dropwise to quench the reaction, and then the resultant was extracted with dichloromethane and saturated aqueous sodium bicarbonate solution. The organic phase was washed with saturated sodium chloride solution, dried over anhydrous sodium sulfate, and concentrated via rotary evaporation. Afterwards, the resultant was subjected to silica gel column chromatography (petroleum ether : ethyl acetate = 20 : 1), so as to obtain 2,4-dichloro-1-isothiocyanatobenzene as a pale yellow oily liquid (1.91g, yield: 99%). ¹H NMR (400MHz, chloroform-d) δ 7.39 (d, J=2.2 Hz, 1H), 7.20 (dd, J = 8.6, 2.2 Hz, 1H), 7.14 (d, J = 8.6 Hz, 1H).

2,4-Dichloro-1-isothiocyanatobenzene (251 mg, 1.23 mmol) was weighed, 4-hydroxybutyric acid hydrazide (160 mg, 1.35 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 3-(4-(2,4-dichlorophenyl)-5-mercapto-4H-1,2,4-triazole-3-yl)propan-1-ol as a white solid (110 mg, yield: 76%). ¹H NMR (400 MHz, DMSO-*d*₆) δ 13.80 (s, 1H), 7.98 (d, *J*=2.2 Hz, 1H), 7.72 - 7.63 (m, 2H), 3.36 (s, 2H), 2.36 (dd, *J* = 8.6, 6.8 Hz, 2H), 1.66 (p, *J* = 6.7 Hz, 2H). HRMS (ESI) calcd for C₁₁H₁₁Cl₂N₃OS[M+H]⁺: 304.0078; found: 304.0068.

### Example 11: Synthesis of 3-(4-(2,4-dichlorophenyl)-5-(ethylthio)-4H-1,2,4-triazole-3-yl)propan-1-ol (Compound 11)

3-(4-(2,4-Dichlorophenyl)-5-mercapto-4H-1,2,4-triazole-3-yl)propan-1-ol (50 mg, 0.16 mmol) was weighed in a 25-mL round-bottom flask, 5 mL of acetone was added, iodoethane (54 mg, 0.32 mmol) and potassium carbonate (36 mg, 0.26 mmol) were added, and the resulting mixture was stirred and reacted overnight at room temperature. 10 mL of distilled water and dichloromethane (10 mL × 3) were added to conduct extraction. The organic phases were combined, washed with saturated sodium chloride solution, and dried over anhydrous sodium sulfate. Afterwards, the resultant was subjected to silica gel column chromatography and was eluted with dichloromethane and methanol (20 : 1). After the solvent was evaporated to dryness via rotary evaporation, 3-(4-(2,4-dichlorophenyl)-5-(ethylthio)-4H-1,2,4-triazole-3-yl)propan-1-ol was obtained as a white solid (37 mg, yield: 68%). HRMS (ESI) calcd for C₁₃H₁₅Cl₂N₃OS[M+H]⁺: 332.0391; found: 332.0389.

### Example 12: Synthesis of 3-(4-(2,4-dichlorophenyl)-5-(isopropylthio)-4H-1,2,4-triazole-3-yl)propan-1-ol (Compound 12)

3-(4-(2,4-Dichlorophenyl)-5-mercapto-4H-1,2,4-triazole-3-yl)propan-1-ol (80 mg, 0.26 mmol) was weighed in a 25-mL round-bottom flask, 5 mL of acetone was added, 2-iodopropane (54 mg, 0.32 mmol) and potassium carbonate (72 mg, 0.52 mmol) were added, and the resulting mixture was stirred and reacted overnight at room temperature. 10 mL of distilled water and dichloromethane (10 mL × 3) were added to conduct extraction. The organic phases were combined, washed with saturated sodium chloride solution, and dried over anhydrous sodium sulfate. Afterwards, the resultant was subjected to silica gel column chromatography and was eluted with petroleum ether and acetone (1 : 1). After the solvent was evaporated to dryness via rotary evaporation, 3-(4-(2,4-dichlorophenyl)-5-(isopropylthio)-4H-1,2,4-triazole-3-yl)propan-1-ol was obtained as a pale yellow oily liquid (45 mg, yield: 49%). ¹H NMR (400 MHz, DMSO-*d*₆) δ 8.00 (dd, *J* = 2.1, 1.1 Hz, 1H), 7.72-7.67 (m, 2H), 4.46 (td, *J* = 5.2, 1.1 Hz, 1H), 3.38 (t, *J* = 5.7 Hz, 2H), 2.49-2.44 (m, 2H), 1.75-1.64 (m, 2H), 1.26 (d, *J* = 6.7 Hz, 6H), 1.14 (d, *J* = 1.1 Hz, 1H). ¹³C NMR (101 MHz, DMSO-*d*₆) δ 155.42, 148.91, 135.92, 132.59, 131.63, 130.15, 130.08, 129.06, 59.67, 29.37, 23.10, 23.08, 21.44. HRMS (ESI) calcd for C₁₄H₁₇Cl₂N₃OS[M+H]⁺: 346.0548; found: 346.0525.

### Example 13: Synthesis of 3-(5-((cyclopropylmethyl)thio)-4-(2,4-dichlorophenyl)-4H-1,2,4-triazole-3-yl)propan-1-ol (Compound 13)

3-(4-(2,4-Dichlorophenyl)-5-mercapto-4H-1,2,4-triazole-3-yl)propan-1-ol (80 mg, 0.26 mmol) was weighed in a 25-mL round-bottom flask, 10 mL of acetonitrile was added, bromomethylcyclopropane (53 mg, 0.40 mmol) and sodium hydroxide (16 mg, 0.40 mmol) were added, and the resulting mixture was stirred and reacted overnight at room temperature. 10 mL of distilled water and dichloromethane (10 mL × 3) were added to conduct extraction. The organic phases were combined, washed with saturated sodium chloride solution, and dried over anhydrous sodium sulfate. Afterwards, the resultant was subjected to silica gel column chromatography and was eluted with dichloromethane and methanol (15 : 1). After the solvent was evaporated to dryness via rotary evaporation, 3-(5-((cyclopropylmethyl)thio)-4-(2,4-dichlorophenyl)-4H-1,2,4-triazole-3-yl)propan-1-ol was obtained as a white solid (43 mg, yield: 45%). ¹H NMR (400 MHz, chloroform-*d*) δ 7.63 (d, *J* = 2.3 Hz, 1H), 7.44 (dd, *J* = 8.4, 2.2 Hz, 1H), 7.24 (s, 1H), 3.76-3.68 (m, 2H), 3.15 (d, *J* = 7.3 Hz, 2H), 2.68-2.55 (m, 2H), 1.95 (qt, *J =* 7.7, 3.6 Hz, 2H), 1.14 (qq, *J* = 7.7, 4.9, 3.9 Hz, 1H), 0.63-0.54 (m, 2H), 0.28 (t, *J* = 5.1 Hz, 2H). ¹³C NMR (101 MHz, chloroform-*d*) δ 162.02, 155.65, 137.41, 137.36, 133.67, 130.94, 130.50, 128.71, 61.73, 39.02, 29.08, 22.38, 10.68, 5.98.

### Example 14: Synthesis of 2-((4-(2,4-dichlorophenyl)-5-(3-hydroxypropyl)-4H-1,2,4-triazole-3-yl)thio)acetamide (Compound 14)

3-(4-(2,4-Dichlorophenyl)-5-mercapto-4H-1,2,4-triazole-3-yl)propan-1-ol (80 mg, 0.26 mmol) was weighed in a 25-mL round-bottom flask, 10 mL of acetone was added, 2-iodoacetamide (59 mg, 0.32 mmol) and potassium carbonate (72 mg, 0.52 mmol) were added, and the resulting mixture was stirred and reacted overnight at room temperature. Distilled water (10 mL) and dichloromethane (10 mL × 3) were added to conduct extraction. The organic phases were combined, washed with saturated sodium chloride solution, and dried over anhydrous sodium sulfate. Afterwards, the resultant was subjected to silica gel column chromatography with gradient elution (dichloromethane : methanol = 20 : 1 to 10 : 1). After the solvent was evaporated to dryness via rotary evaporation, 2-((4-(2,4-dichlorophenyl)-5-(3-hydroxypropyl)-4H-1,2,4-triazole-3-yl)thio)acetamide was obtained as a white solid (111 mg, yield: 99%). ¹H NMR (400 MHz, DMSO-*d*₆) δ 8.02 (s, 1H), 7.71 (d, *J* = 1.8 Hz, 2H), 7.63 (s, 1H), 7.22 (s, 1H), 4.48 (td, *J* = 5.1, 1.7 Hz, 1H), 3.90-3.78 (m, 2H), 3.38 (s, 2H), 2.46 (d, *J* = 8.4 Hz, 2H), 1.75-1.62 (m, 2H). ¹³C NMR (101 MHz, DMSO-*d*₆) δ 168.49, 155.56, 149.62, 136.20, 132.68, 131.70, 130.40, 129.53, 129.20, 59.68, 36.49, 29.47, 21.31. HRMS (ESI) calcd for C₁₃H₁₄Cl₂N₄O₂S [M+H]⁺: 361.0293; found: 361.0275.

### Example 15: Synthesis of 3-(4-(2,4-difluorophenyl)-5-(methylthio)-4H-1,2,4-triazole-3-yl)propan-1-ol (Compound 15)

2,4-Difluorophenylisothiocyanate (300 mg, 1.75 mmol) was weighed, 4-hydroxybutyric acid hydrazide (207 mg, 1.75 mmol) was added, 8 mL of absolute ethanol was added, and the resulting mixture was refluxed overnight. The ethanol solution was evaporated to dryness via rotary evaporation, 8 mL of 2M aqueous sodium hydroxide solution was then added immediately, and the resulting mixture was refluxed overnight. 2M aqueous HCl solution was added slowly until the solution became acidic, and a large amount of white solid appeared. The resultant was subjected to suction filtration, washed with water and then subjected to vacuum drying, so as to obtain 3-(4-(2,4-difluorophenyl)-5-mercapto-4H-1,2,4-triazole-3-yl)propan-1-ol (crude product) as a white solid (258 mg, yield: 54%). ¹H NMR (400 MHz, DMSO-*d*₆) δ 13.82 (s, 1H), 7.75-7.55 (m, 2H), 7.39-7.30 (m, 1H), 4.50 (t, *J* = 5.1 Hz, 1H), 3.38-3.32 (m, 2H), 2.49-2.29 (m, 2H), 1.65 (dq, *J* = 8.0, 6.4 Hz, 2H).

3-(4-(2,4-Difluorophenyl)-5-mercapto-4H-1,2,4-triazole-3-yl)propan-1-ol (89 mg, 0.33 mmol) was weighed, potassium carbonate solid (138 mg, 1.00 mmol) was added, 10 mL of ethanol was added, iodomethane (30.8 µL, 0.50 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 3-(4-(2,4-difluorophenyl)-5-(methylthio)-4H-1,2,4-triazole-3-yl)propan-1-ol as a pale yellow oily liquid (39 mg, yield: 41%). ¹H NMR (400 MHz, DMSO-*d*₆) δ 7.73 (dd, *J* = 8.7, 6.0 Hz, 1H), 7.64 (td, *J* = 9.6, 2.6 Hz, 1H), 7.33 (td, *J* = 8.7, 2.6 Hz, 1H), 4.43 (t, *J* = 5.0 Hz, 1H), 3.41-3.31 (m, 2H), 2.50 (s, 3H), 2.46 (q, *J* = 4.4 Hz, 2H), 1.64 (m, 7.5 Hz, 2H). HRMS (ESI) calcd for C₁₂H₁₃F₂N₃OS [M+H]⁺: 286.0826; found: 286.0825.

### Example 16: Synthesis of 3-(4-(4-chlorophenyl)-5-(methylthio)-4H-1,2,4-triazole-3-yl)propan-1-ol (Compound 16)

4-Chloro-1-isothiocyanatobenzene (300 mg, 1.77 mmol) was weighed, 4-hydroxybutyric acid hydrazide (219 mg, 1.86 mmol) was added, 6 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(4-chlorophenyl)-5-mercapto-4H-1,2,4-triazole-3-yl)propan-1-ol (crude product) as a white solid (350 mg, yield: 73%).

3-(4-(4-Chlorophenyl)-5-mercapto-4H-1,2,4-triazole-3-yl)propan-1-ol (150 mg, 0.56 mmol) was weighed, potassium carbonate solid (138 mg, 1.00 mmol) was added, 8 mL of acetonitrile was added, iodomethane (38 µL, 0.60 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(4-chlorophenyl)-5-(methylthio)-4H-1,2,4-triazole-3-yl)propan-1-ol as a white solid powder (87 mg, yield: 55%). ¹H NMR (400 MHz, DMSO-*d*₆) δ 7.68 (d, *J* = 8.6 Hz, 2H), 7.52 (d, *J* = 8.6 Hz, 2H), 4.47 (t, *J* = 5.2 Hz, 1H), 3.37 (q, *J* = 6.1 Hz, 2H), 2.54 (m, 2H), 2.54 (s, 3H), 1.74-1.64 (m, 2H). ¹³C NMR (101 MHz, DMSO-*d*₆) δ 156.00, 151.01, 135.09, 132.51, 130.46, 129.75, 60.18, 29.94, 21.89, 15.09. HRMS (ESI) calcd for C₁₂H₁₄ClN₃OS[M+H]⁺: 284.0624; found: 284.0612.

### Example 17: Synthesis of 3-(4-(2-chlorophenyl)-5-(methylthio)-4H-1,2,4-triazole-3-yl)propan-1-ol (Compound 17)

2-Chloro-1-isothiocyanatobenzene (200 mg, 1.18 mmol) was weighed, 4-hydroxybutyric acid hydrazide (139 mg, 1.18 mmol) was added, 6 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(2-chlorophenyl)-5-mercapto-4H-1,2,4-triazole-3-yl)propan-1-ol as a pale yellow solid (140 mg, yield: 39%). ¹H NMR (400 MHz, DMSO-*d*₆) δ 13.79 (s, 1H), 7.74 (d, *J* = 7.7 Hz, 1H), 7.59 (dt, *J* = 15.2, 4.5 Hz, 3H), 3.35 (t, *J* = 6.2 Hz, 2H), 2.34 (t, *J* = 7.7 Hz, 2H), 1.65 (p, *J* = 6.7 Hz, 2H). ¹³C NMR (101MHz, DMSO-*d*₆) δ 156.15, 152.45, 132.80, 131.95, 131.13, 130.97, 129.81, 128.48, 61.75, 29.26, 22.40.

3-(4-(2-Chlorophenyl)-5-mercapto-4H-1,2,4-triazole-3-yl)propan-1-ol (80 mg, 0.30 mmol) was weighed, potassium carbonate solid (138 mg, 1.00 mmol) was added, 8 mL of acetone was added, iodomethane (28 µL, 0.45 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(2-chlorophenyl)-5-(methylthio)-4H-1,2,4-triazole-3-yl)propan-1-ol as brown oil (52 mg, yield: 61%). ¹H NMR (400 MHz, chloroform-*d*) δ 7.61 (dd, *J* = 8.0, 1.5 Hz, 1H), 7.52 (td, *J* = 7.8, 1.8 Hz, 1H), 7.45 (td, *J* = 7.6, 1.6 Hz, 1H), 7.30 (dd, *J* = 7.7, 1.7 Hz, 1H), 3.69 (dq, *J* = 8.2, 5.1 Hz, 2H), 2.81 (s, 1H), 2.64 (s, 3H), 2.62 - 2.52 (m, 2H), 1.92 (qd, *J* = 6.9, 5.0 Hz, 2H). ¹³C NMR (101 MHz, chloroform-*d*) δ 156.15, 152.45, 132.80, 131.95, 131.13, 130.97, 129.81, 128.48, 61.75, 29.26, 22.40, 14.97. HRMS (ESI) calcd for C₁₂H₁₄ClN₃OS[M+H]⁺: 284.0624; found: 284.0608.

### Example 18: Synthesis of 3-(4-(3-chlorophenyl)-5-(methylthio)-4H-1,2,4-triazole-3-yl)propan-1-ol (Compound 18)

3-Chloro-1-isothiocyanatobenzene (339 mg, 2.00 mmol) was weighed, 4-hydroxybutyric acid hydrazide (248 mg, 2.10 mmol) was added, 8 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(3-chlorophenyl)-5-mercapto-4H-1,2,4-triazole-3-yl)propan-1-ol as a white solid (166 mg, yield: 31%).

3-(4-(2-Chlorophenyl)-5-mercapto-4H-1,2,4-triazole-3-yl)propan-1-ol (100 mg, 0.35 mmol) was weighed, potassium carbonate solid (138 mg, 1.00 mmol) was added, 8 mL of acetone was added, iodomethane (33 µL, 0.53 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(3-chlorophenyl)-5-(methylthio)-4H-1,2,4-triazole-3-yl)propan-1-ol as a white solid (56 mg, yield: 56%). ¹H NMR (400 MHz, DMSO-*d*₆) δ 7.70 (d, *J* = 2.0 Hz, 1H), 7.69-7.66 (m, 1H), 7.63 (t, *J* = 7.8 Hz, 1H), 7.47 (dt, *J* = 7.6, 1.6 Hz, 1H), 4.50 (t, *J* = 5.2 Hz, 1H), 3.39 (d, *J* = 6.1 Hz, 2H), 2.57 (m, *J* = 4.8 Hz, 2H), 2.55 (s, 3H), 1.70 (m, *J* = 8.1, 6.4 Hz, 2H). ¹³C NMR (101 MHz, DMSO-*d*₆) δ 155.91, 150.99, 134.94, 134.43, 131.99, 130.57, 127.91, 126.81, 60.17, 29.85, 21.90, 15.11. HRMS (ESI) calcd for C₁₂H₁₄ClN₃OS [M+H]⁺: 284.0624; found: 284.0614.

### Example 19: Synthesis of 3-(4-(3,4-dichlorophenyl)-5-(methylthio)-4H-1,2,4-triazole-3-yl)propan-1-ol (Compound 19)

3,4-Dichloro-1-isothiocyanatobenzene (306 mg, 1.50 mmol) was weighed, 4-hydroxybutyric acid hydrazide (186 mg, 1.57 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(3,4-dichlorophenyl)-5-mercapto-4H-1,2,4-triazole-3-yl)propan-1-ol as a white solid (260 mg, yield: 57%).

3-(4-(3,4-Dichlorophenyl)-5-mercapto-4H-1,2,4-triazole-3-yl)propan-1-ol (100 mg, 0.33 mmol) was weighed, potassium carbonate solid (138 mg, 1.00 mmol) was added, 8 mL of acetone was added, iodomethane (33 µL, 0.53 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(3,4-dichlorophenyl)-5-(methylthio)-4H-1,2,4-triazole-3-yl)propan-1-ol as a white solid (55 mg, yield: 52%). ¹H NMR (400 MHz, chloroform-*d*) δ 7.63 (d, *J* = 8.5 Hz, 1H), 7.41 (d, *J* = 2.4 Hz, 1H), 7.14 (dd, *J* = 8.5, 2.4 Hz, 1H), 3.72 (t, *J* = 5.7 Hz, 2H), 2.92-2.74 (m, 1H), 2.70 (m, 2H), 2.67 (s, 3H), 2.00-1.92 (m, 2H). ¹³C NMR (101 MHz, chloroform-*d*) δ 155.72, 152.23, 134.94, 134.26, 132.37, 131.78, 129.09, 129.09, 126.47, 61.63, 29.24, 22.43, 14.67. HRMS (ESI) calcd for C₁₂H₁₃Cl₂N₃OS [M+H]⁺: 318.0235; found: 318.0222.

### Example 20: Synthesis of 3-(4-(2,3-dichlorophenyl)-5-(methylthio)-4H-1,2,4-triazole-3-yl)propan-1-ol (Compound 20)

2,3-Dichloro-1-isothiocyanatobenzene (314 mg, 1.54 mmol) was weighed, 4-hydroxybutyric acid hydrazide (190 mg, 1.61 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(2,3-dichlorophenyl)-5-mercapto-4H-1,2,4-triazole-3-yl)propan-1-ol as a white solid (400 mg, yield: 80%).

3-(4-(2,3-Dichlorophenyl)-5-mercapto-4H-1,2,4-triazole-3-yl)propan-1-ol (100 mg, 0.33 mmol) was weighed, potassium carbonate solid (138 mg, 1.00 mmol) was added, 8 mL of acetone was added, iodomethane (31 µL, 0.49 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(2,3-dichlorophenyl)-5-(methylthio) -4H-1,2,4-triazole-3-yl)propan-1-ol as a white solid (64 mg, yield: 61%). ¹H NMR (400 MHz, chloroform-*d*) δ 7.68 (dd, *J* = 8.0, 1.5 Hz, 1H), 7.41 (t, *J* = 8.0 Hz, 1H), 7.24 (d, *J* = 1.5 Hz, 1H), 3.71 (dq, *J* = 10.1, 5.3 Hz, 2H), 2.94 (s, 1H), 2.66 (s, 3H), 2.64-2.52 (m, 2H), 1.95 (dddd, *J* = 12.8, 7.4, 5.5, 1.6 Hz, 2H). ¹³C NMR (101 MHz, chloroform-*d*) δ 155.78, 152.13, 147.22, 135.08, 132.58, 131.89, 128.20, 127.86, 61.68, 29.10, 22.31, 14.90. HRMS (ESI) calcd for C₁₂H₁₃Cl₂N₃OS[M+H]⁺: 318.0235; found: 318.0222.

### Example 21: Synthesis of 3-(5-(methylthio)-4-(2,4,6-trichlorophenyl)-4H-1,2,4-triazole-3-yl)propan-1-ol (Compound 21)

2,4,6-Trichloro-1-isothiocyanatobenzene (314 mg, 1.54 mmol) was weighed, 4-hydroxybutyric acid hydrazide (248 mg, 2.10 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(2,4,6-dichlorophenyl)-5-mercapto-4H-1,2,4-triazole-3-yl)propan-1-ol as a white solid (330 mg, yield: 49%).

3-(4-(2,4,6-Dichlorophenyl)-5-mercapto-4H-1,2,4-triazole-3-yl)propan-1-ol (186 mg, 0.82 mmol) was weighed, potassium carbonate solid (276 mg, 2.00 mmol) was added, 8 mL of acetone was added, iodomethane (51 µL, 0.82 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(5-(methylthio)-4-(2,4,6-trichlorophenyl)-4H-1,2,4-triazole-3-yl)propan-1-ol as a white solid (87 mg, yield: 45%). ¹H NMR (400 MHz, DMSO-*d*₆) δ 8.12 (d, *J* = 11.4 Hz, 2H), 4.48 (p, *J* = 5.1 Hz, 1H), 3.41 (m, 2H), 2.59 (s, 3H), 2.45 (m, 2H), 1.72 (qd, *J* = 7.9, 3.4 Hz, 2H).¹³C NMR (101 MHz, DMSO-*d*₆) δ 155.64, 150.89, 137.34, 134.95, 130.14, 128.02, 60.11, 30.03, 21.53, 15.33. HRMS (ESI) calcd for C₁₂H₁₂Cl₃N₃OS [M+H]⁺: 351.9845; found: 351.9830.

### Example 22: Synthesis of 3-(5-(methylthio)-4-(2,3,4-trichlorophenyl)-4H-1,2,4-triazole-3-yl)propan-1-ol (Compound 22)

2,3,4-Trichloro-1-isothiocyanatobenzene (358 mg, 1.50 mmol) was weighed, 4-hydroxybutyric acid hydrazide (186 mg, 1.58 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-(2,3,4-dichlorophenyl)-5-mercapto-4H-1,2,4-triazole-3-yl)propan-1-ol as a white solid (170 mg, yield: 33%).

3-(4-(2,3,4-Dichlorophenyl)-5-mercapto-4H-1,2,4-triazole-3-yl)propan-1-ol (107 mg, 0.32 mmol) was weighed, potassium carbonate solid (276 mg, 2.00 mmol) was added, 8 mL of acetone was added, iodomethane (30 µL, 0.48 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(5-(methylthio)-4-(2,3,4-trichlorophenyl)-4H-1,2,4-triazole-3-yl)propan-1-ol as a white solid (35 mg, yield: 32%). ¹H NMR (400 MHz, DMSO-*d*₆) δ 7.96 (d, *J* = 8.6 Hz, 1H), 7.80 (d, *J* = 8.6 Hz, 1H), 4.48 (t, *J* = 5.2 Hz, 1H), 3.41-3.37 (m, 2H), 2.55 (s, 3H), 2.47 (t, *J* = 7.8 Hz, 2H), 1.77-1.65 (m, 2H). ¹³C NMR (101 MHz, DMSO-*d*₆) δ 155.87, 150.97, 135.78, 132.79, 132.35, 131.53, 130.56, 130.07, 60.11, 29.99, 21.66, 15.40. HRMS (ESI) calcd for C₁₂H₁₂Cl₃N₃OS [M+H]⁺: 351.9845; found: 351.9828.

### Example 23: Synthesis of 3-(5-(methylthio)-4-(3,4,5-trichlorophenyl)-4H-1,2,4-triazole-3-yl)propan-1-ol (Compound 23)

3,4,5-Trichloroaniline (786 mg, 4.00 mmol) was weighed and charged into a 25-mL single-neck flask. A magnetic stirring bar was added, 3,4,5-trichloroaniline was dissolved in 10 mL of anhydrous dichloromethane, sodium carbonate (1.27g, 11.98 mmol) was added, and the resulting mixture was stirred in an ice bath. Thiophosgene (335.5 µL, 5.37 mmol) was slowly added dropwise, and the resultant was reacted in an ice bath for 2h. A sample was taken and the complete reaction of raw materials was detected. 2M aqueous sodium hydroxide solution was slowly added dropwise to quench the reaction, and then the resultant was extracted with dichloromethane (3 × 10 mL) and saturated aqueous sodium bicarbonate solution (10 mL). The organic phases were washed with saturated sodium chloride solution, dried over anhydrous sodium sulfate, and concentrated via rotary evaporation. Afterwards, the resultant was subjected to silica gel column chromatography (petroleum ether : ethyl acetate = 20 : 1), so as to obtain 1,2,3-trichloro-5-isothiocyanic acid as a milky white solid (400 mg, yield: 42%). ¹H NMR (400 MHz, DMSO-*d*₆) δ 7.96 (s, 2H).

1,2,3-Trichloro-5-isothiocyanic acid (350 mg, 1.47 mmol) was weighed, 4-hydroxybutyric acid hydrazide (209 mg, 1.76 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(3,4,5-trichlorophenyl)-4H-1,2,4-triazole-3-yl)propan-1-ol as a white solid (198 mg, yield: 39%). ¹H NMR (400 MHz, DMSO-*d*₆) δ 13.81 (s, 1H), 7.94 (d, *J* = 1.7 Hz, 2H), 4.52 (dt, *J* = 5.3, 2.5 Hz, 1H), 3.41 (s, 2H), 2.50-2.45 (m, 2H), 1.68 (dq, *J* = 8.1, 6.3 Hz, 2H). ¹³C NMR (101 MHz, DMSO- *d*₆) δ 167.96, 152.51, 134.08, 133.89, 131.95, 130.18, 59.95, 28.73, 22.54.

3-(5-Mercapto-4-(3,4,5-trichlorophenyl)-4H-1,2,4-triazole-3-yl)propan-1-ol (190 mg, 0.56 mmol) was weighed, potassium carbonate solid (258 mg, 1.86 mmol) was added, 8 mL of acetonitrile was added, iodomethane (38 µL, 0.60 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 3-(5-(methylthio)-4-(3,4,5-trichlorophenyl)-4H-1,2,4-triazole-3-yl)propan-1-ol as a white solid (120 mg, yield: 61%). ¹H NMR (400 MHz, DMSO-*d*₆) δ 8.03 (s, 2H), 4.49 (t, *J* = 5.2 Hz, 1H), 3.42-3.37 (m, 2H), 2.63-2.56 (m, 2H), 2.55 (s, 3H), 1.73 (p, *J* = 6.6 Hz, 2H).¹³C NMR (101 MHz, DMSO-*d*₆) δ 155.94, 150.89, 134.38, 133.49, 132.44, 129.09, 60.17, 29.84, 21.82, 15.40. HRMS (ESI) calcd for C₁₂H₁₂Cl₃N₃OS[M+H]⁺: 351.9845; found: 351.9825.

### Example 24: Synthesis of 3-(5-(methylthio)-4-(3,4,5-trimethylphenyl)-4H-1,2,4-triazole-3-yl)propan-1-ol (Compound 24)

3,4,5-Trimethylaniline (541 mg, 4.00 mmol) was weighed and charged into a 25-mL single-neck flask. A magnetic stirring bar was added, 3,4,5-trimethylaniline was dissolved in 10 mL of anhydrous dichloromethane, sodium carbonate (1.27g, 11.98 mmol) was added, and the resulting mixture was stirred in an ice bath. Thiophosgene (335.5 µL, 4.40 mmol) was slowly added dropwise, and the resultant was reacted in an ice bath for 2h. A sample was taken and the complete reaction of raw materials was detected. 2M aqueous sodium hydroxide solution was slowly added dropwise to quench the reaction, and then the resultant was extracted with dichloromethane and saturated aqueous sodium bicarbonate solution. The organic phases were washed with saturated sodium chloride solution, dried over anhydrous sodium sulfate, and concentrated via rotary evaporation. Afterwards, the resultant was subjected to silica gel column chromatography (petroleum ether : ethyl acetate = 20 : 1), so as to obtain 3,4,5-trimethyl-1-isothiocyanatobenzene as a milky white solid (443 mg, yield: 63%). ¹H NMR (400 MHz, chloroform-*d*) δ 6.85 (s, 2H), 2.23 (s, 6H), 2.13 (s, 3H).

3,4,5-Trimethyl-1-isothiocyanatobenzene (430 mg, 2.43 mmol) was weighed, 4-hydroxybutyric acid hydrazide (295 mg, 2.50 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(3,4,5-trimethylphenyl)-4H-1,2,4-triazole-3-yl)propan-1-ol as a white solid (500 mg, yield: 45%). ¹H NMR (400 MHz, DMSO-*d*₆) δ 13.60 (s, 1H), 7.00 (s, 2H), 3.35 (t, *J* = 6.2 Hz, 2H), 2.44 (s, 2H), 2.43-2.35 (m, 2H), 2.29 (s, 6H), 2.18 (s, 3H), 1.66 (dq, *J* = 8.0, 6.3 Hz, 2H). ¹³C NMR (101 MHz, DMSO-*d*₆) δ 167.81, 152.54, 137.52, 136.57, 130.83, 126.86, 59.69, 28.59, 22.35, 20.33, 15.23.

3-(5-Mercapto-4-(3,4,5-trimethylphenyl)-4H-1,2,4-triazole-3-yl)propan-1-ol (300 mg, 1.32 mmol) was weighed, potassium carbonate (447 mg, 3.24 mmol) was added, 8 mL of acetonitrile was added, iodomethane (93 µL, 1.50 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 3-(5-(methylthio)-4-(3,4,5-trimethylphenyl)-4H-1,2,4-triazole-3-yl)propan-1-ol as a milky white solid powder (200 mg, yield: 64%). ¹H NMR (400 MHz, DMSO-*d*₆) δ 8.03 (s, 2H), 4.49 (t, *J* = 5.2 Hz, 1H), 3.42-3.37 (m, 2H), 2.63-2.56 (m, 2H), 2.55 (s, 3H), 1.73 (p, *J* = 6.6 Hz, 2H). ¹³C NMR (101 MHz, DMSO-*d*₆) δ 155.94, 150.89, 134.38, 133.49, 132.44, 129.09, 60.23, 29.99, 21.89, 20.53, 15.51, 14.80. HRMS (ESI) calcd for C₁₂H₁₂Cl₃N₃OS[M+H]⁺: 351.9845; found: 351.9825.

### Example 25: Synthesis of 3-(4-(4-methoxyphenyl)-5-(methylthio)-4H-1,2,4-triazole-3-yl)propan-1-ol (Compound 25)

4-Methoxy-1-isothiocyanatobenzene (227 mg, 1.37 mmol) was weighed, 4-hydroxybutyric acid hydrazide (186 mg, 1.58 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(4-methoxyphenyl)-4H-1,2,4-triazole-3-yl)propan-1-ol as a white solid (150 mg, yield: 38%). ¹H NMR (400 MHz, DMSO-*d*₆) δ 13.63 (s, 1H), 7.35-7.28 (m, 2H), 7.12-7.06 (m, 2H), 3.83 (s, 3H), 3.35 (t, *J* = 6.2 Hz, 2H), 2.42 (t, *J* = 7.6 Hz, 2H), 1.65 (dt, *J* = 8.3, 6.5 Hz, 2H).

3-(5-Mercapto-4-(4-methoxyphenyl)-4H-1,2,4-triazole-3-yl)propan-1-ol (100 mg, 0.39 mmol) was weighed, potassium carbonate solid (138 mg, 1.00 mmol) was added, 8 mL of acetone was added, iodomethane (64 µL, 0.59 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-(4-methoxyphenyl)-5-(methylthio)-4H-1,2,4-triazole-3-yl)propan-1-ol as a white solid (87 mg, yield: 45%). ¹H NMR (400 MHz, DMSO-*d*₆) δ 7.36 (d, *J* = 8.8 Hz, 2H), 7.12 (d, *J* = 8.8 Hz, 2H), 4.49 (t, *J* = 5.1 Hz, 1H), 3.84 (s, 3H), 2.54 (m, 4H), 2.53 (s, 3H), 1.68 (d, *J* = 7.1 Hz, 2H). ¹³C NMR (101 MHz, DMSO-*d*₆) δ 160.43, 156.30, 151.47, 129.07, 126.00, 115.46, 60.24, 55.99, 30.00, 21.90, 14.85. HRMS (ESI) calcd for C₁₃H₁₇N₃O₂S [M+H]⁺: 280.1120, [M+Na]⁺: 302.0930; found: 280.1107, [M+Na]⁺: 302.0924.

### Example 26: Synthesis of 3-(5-(methylthio)-4-(4-(trifluoromethyl)phenyl)-4H-1,2,4-triazole-3-yl)propan-1-ol (Compound 26)

4-Trifluoromethyl-1-isothiocyanatobenzene (305 mg, 1.00 mmol) was weighed, 4-hydroxybutyric acid hydrazide (186 mg, 1.00 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of orange-yellow solid appeared. The resultant was subjected to suction filtration, washed with water and then subjected to vacuum drying, so as to obtain 3-(5-mercapto-4-(4-trifluoromethylphenyl)-4H-1,2,4-triazole-3-yl)propan-1-ol as an orange-yellow solid (128 mg, yield: 40%). ¹H NMR (400 MHz, DMSO-*d*₆) δ 13.81 (s, 1H), 7.98 (d, *J* = 8.2 Hz, 2H), 7.73 (d, *J* = 8.2 Hz, 2H), 3.35 (d, *J* = 6.1 Hz, 2H), 2.47 (t, *J* = 7.6 Hz, 2H), 1.66 (p, *J* = 6.7 Hz, 2H). ¹³C NMR (101 MHz, DMSO- *d*₆*)* δ 167.90, 152.48, 137.86, 130.03, 127.03, 125.64, 122.93, 59.89, 28.75, 22.64.

3-(5-Mercapto-4-(4-trifluoromethylphenyl)-4H-1,2,4-triazole-3-yl)propan-1-ol (120 mg, 0.38 mmol) was weighed, potassium carbonate solid (138 mg, 1.00 mmol) was added, 8 mL of acetone was added, iodomethane (35 µL, 0.57 mmol) was added dropwise under stirring by a rotor, and the resulting mixture was reacted overnight at room temperature. After rotary evaporation, the resultant was subjected to silica gel column chromatography (dichloromethane : methanol = 15 : 1), so as to obtain 3-(5-(methylthio)-4-(4-(trifluoromethyl) phenyl)-4H-1,2,4-triazole-3-yl)propan-1-ol as a white solid (90 mg, yield: 75%). ¹H NMR (400 MHz, DMSO-*d*₆) δ 7.95 (d, *J* = 8.3 Hz, 2H), 7.70 (d, *J* = 8.3 Hz, 2H), 4.42 (t, *J* = 5.2 Hz, 1H), 3.32 (q, *J* = 6.0 Hz, 2H), 2.53 (m, 2H), 2.50 (s, 3H), 1.66 (p, *J* = 6.4 Hz, 2H). ¹³C NMR (101 MHz, DMSO- *d*₆) δ 155.87, 150.84, 137.25, 130.75, 130.43, 127.57, 125.52, 122.81, 60.13, 29.87, 21.92, 15.22. HRMS (ESI) calcd for C₁₃H₁₄F₃N₃OS [M+H]⁺: 318.0888; found: 318.0866.

### Example 27: Synthesis of 3-(5-(methylthio)-4-(3-(trifluoromethyl)phenyl)-4H-1,2,4-triazole-3-yl)propan-1-ol (Compound 27)

4-Trifluoromethyl-1-isothiocyanatobenzene (305 mg, 1.00 mmol) was weighed, 4-hydroxybutyric acid hydrazide (186 mg, 1.00 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(3-trifluoromethylphenyl)-4H-1,2,4-triazole-3-yl)propan-1-ol as a white solid (260 mg, yield: 82%). ¹H NMR (400 MHz, DMSO-*d*₆) δ 13.78 (s, 1H), 7.94 (d, *J* = 11.8 Hz, 2H), 7.87-7.76 (m, 2H), 3.36 (m, 2H), 2.46 (t, *J* = 7.6 Hz, 2H), 1.66 (p, *J* = 6.7 Hz, 2H). ¹³C NMR (101 MHz, DMSO-*d*₆) δ 168.02, 152.59, 135.05, 133.28, 131.20, 130.70, 130.37, 126.82, 126.10, 59.90, 28.71, 22.64.

3-(5-Mercapto-4-(3-trifluoromethylphenyl)-4H-1,2,4-triazole-3-yl)propan-1-ol (120 mg, 0.38 mmol) was weighed, potassium carbonate solid (138 mg, 1.00 mmol) was added, 8 mL of acetone was added, iodomethane (35 µL, 0.57 mmol) was added dropwise under stirring by a rotor, and the resulting mixture was reacted overnight at room temperature. After rotary evaporation, the resultant was subjected to silica gel column chromatography (dichloromethane : methanol = 15 : 1), so as to obtain 3-(5-(methylthio)-4-(3-(trifluoromethyl) phenyl)-4H-1,2,4-triazole-3-yl)propan-1-ol as a colorless oily liquid (92 mg, yield: 77%). ¹H NMR (400 MHz, DMSO-*d*₆) δ 8.02-7.96 (m, 2H), 7.89-7.80 (m, 2H), 4.49 (t, *J* = 5.2 Hz, 1H), 3.37 (m, 2H), 2.55 (d, *J* = 2.6 Hz, 5H), 1.75-1.66 (m, 2H). ¹³C NMR (101 MHz, DMSO-*d*₆) δ 155.96, 150.98, 134.45, 132.26, 131.78, 127.28, 125.07, 122.56, 60.13, 29.80, 21.89, 15.15. HRMS (ESI) calcd for C₁₃H₁₄F₃N₃OS [M+H]⁺: 318.0888, [M+Na]⁺: 340.0707; found: [M+H]⁺: 318.0869. [M+Na]⁺: 340.0687.

### Example 28: Synthesis of 3-(5-(methylthio)-4-(m-tolyl)-4H-1,2,4-triazole-3-yl)propan-1-ol (Compound 28)

3-Methyl-1-isothiocyanatobenzene (298 mg, 2.00 mmol) was weighed, 4-hydroxybutyric acid hydrazide (248 mg, 2.10 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(m-tolyl)-4H-1,2,4-triazole-3-yl)propan-1-ol as a white solid (260 mg, yield: 32%). ¹H NMR (400 MHz, DMSO-*d*₆) δ 13.65 (s, 1H), 7.46 (t, *J* = 7.7 Hz, 1H), 7.35 (d, *J* = 7.7 Hz, 1H), 7.25-7.12 (m, 2H), 4.47 (t, *J* = 5.2 Hz, 1H), 3.36 (t, *J* = 5.8 Hz, 2H), 2.43 (t, *J* = 7.6 Hz, 2H), 2.38 (s, 3H), 1.65 (p, *J* = 6.7 Hz, 2H). ¹³C NMR (101 MHz, DMSO-*d*₆) δ 168.06, 152.71, 139.51, 134.20, 130.55, 129.67, 129.05, 125.76, 59.95, 28.88, 22.63, 21.23.

3-(5-Mercapto-4-(m-tolyl)-4H-1,2,4-triazole-3-yl)propan-1-ol (150 mg, 0.60 mmol) was weighed, potassium carbonate solid (138 mg, 1.00 mmol) was added, 8 mL of acetone was added, iodomethane (56 µL, 0.90 mmol) was added dropwise under stirring by a rotor, and the resulting mixture was reacted overnight at room temperature. After rotary evaporation, the resultant was subjected to silica gel column chromatography (dichloromethane : methanol = 15 : 1), so as to obtain 3-(5-(methylthio)-4-(m-tolyl)-4H-1,2,4-triazole-3-yl)propan-1-ol as a white solid (231 mg, yield: 68%). ¹H NMR (400 MHz, chloroform-*d*) δ 7.41 (t, *J* = 8.1 Hz, 1H), 7.33 (d, *J* = 7.8 Hz, 1H), 7.11-6.97 (m, 2H), 3.70 (q, *J* = 5.4 Hz, 2H), 3.27 (t, *J* = 5.4 Hz, 1H), 2.69 (t, *J* = 7.0 Hz, 2H), 2.64 (s, 3H), 2.43 (s, 3H), 1.98-1.87 (m, 2H). ¹³C NMR (101 MHz, chloroform-*d*) δ 156.06, 152.39, 140.36, 133.09, 130.88, 129.78, 127.47, 124.03, 61.80, 29.35, 22.57, 21.30, 14.59. HRMS (ESI) calcd for C₁₃H₁₇N₃OS [M+H]⁺: 264.1171; found: 264.1173.

### Example 29: Synthesis of 3-(5-(methylthio)-4-(pyridin-3-yl)-4H-1,2,4-triazole-3-yl)propan-1-ol (Compound 29)

3-Pyridylisothiocyanate (300 mg, 2.20 mmol) was weighed, 4-hydroxybutyric acid hydrazide (260 mg, 2.20 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(pyridin-3-yl)-4H-1,2,4-triazole-3-yl)propan-1-ol (crude product) as a white solid (323 mg, yield: 62%).

3-(5-Mercapto-4-(pyridin-3-yl)-4H-1,2,4-triazole-3-yl)propan-1-ol (100 mg, 0.42 mmol) was weighed, potassium carbonate solid (138 mg, 1.00 mmol) was added, 8 mL of acetone was added, iodomethane (40 µL, 0.64 mmol) was added dropwise under stirring by a rotor, and the resulting mixture was reacted overnight at room temperature. After rotary evaporation, the resultant was subjected to silica gel column chromatography (dichloromethane : methanol = 15 : 1), so as to obtain 3-(5-(methylthio)-4-(pyridin-3-yl)-4H-1,2,4-triazole-3-yl)propan-1-ol as a white solid (231 mg, yield: 68%). ¹H NMR (400 MHz, DMSO-*d*₆) δ 8.74 (d, *J* = 4.8 Hz, 1H), 8.66 (d, *J* = 2.2 Hz, 1H), 7.97 (d, *J* = 8.0 Hz, 1H), 7.62 (dd, *J* = 8.0, 4.8 Hz, 1H), 4.42 (t, *J* = 5.1 Hz, 1H), 3.33 (q, *J* = 6.0 Hz, 2H), 2.53 (m, 2H), 2.50 (s, 3H), 1.66 (p, *J* = 6.5 Hz, 2H). ¹³C NMR (101 MHz, DMSO-*d*₆) δ 156.18, 151.40, 151.21, 148.51, 135.85, 130.66, 125.12, 60.13, 29.90, 21.86, 15.27. HRMS (ESI) calcd for C₁₁H₁₄N₄OS[M+H]⁺: 251.0967; found: 251.0968.

### Example 30: Synthesis of 3-(4-cyclohexyl-5-(methylthio)-4H-1,2,4-triazole-3-yl)propan-1-ol (Compound 30)

Cyclohexylisothiocyanate (283 mg, 2.00 mmol) was weighed, 4-hydroxybutyric acid hydrazide (248 mg, 2.10 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-cyclohexyl-5-mercapto-4H-1,2,4-triazole-3-yl)propan-1-ol (crude product) as a white solid (230 mg, yield: 48%).

3-(4-Cyclohexyl-5-mercapto-4H-1,2,4-triazole-3-yl)propan-1-ol (100 mg, 0.41 mmol) was weighed, potassium carbonate solid (138 mg, 1.00 mmol) was added, 8 mL of acetone was added, iodomethane (39 µL, 0.62 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-cyclohexyl-5-(methylthio)-4H-1,2,4-triazole-3-yl)propan-1-ol as a white solid (40 mg, yield: 38%). ¹H NMR (400 MHz, DMSO-*d*₆) δ 4.60 (t, *J* = 5.2 Hz, 1H), 4.02 (ddt, *J* = 12.4, 8.5, 3.8 Hz, 1H), 3.48 (q, *J* = 5.9 Hz, 2H), 2.78 (t, *J* = 7.6 Hz, 2H), 2.51 (p, *J* = 1.8 Hz, 2H), 2.01 (qd, *J* = 12.4, 3.4 Hz, 2H), 1.79 (m, *J* = 14.4, 9.3, 3.8 Hz, 6H), 1.67 (d, *J* = 13.2 Hz, 1H), 1.39 (qt, *J* = 12.9, 3.4 Hz, 2H), 1.18 (ttd, *J* = 12.9, 9.6, 4.9 Hz, 1H). ¹³C NMR (101 MHz, DMSO-*d*₆) δ 155.81, 149.14, 60.31, 55.51, 31.12, 30.77, 25.76, 25.09, 22.22, 15.88. HRMS (ESI) calcd for C₁₂H₂₁N₃OS [M+H]⁺: 256.1484, [M+Na]⁺: 278.1303; found: [M+H]⁺: 256.1471, [M+Na]⁺: 278.1289.

### Example 31: Synthesis of 3-(5-(methylthio)-4-(naphthalen-1-yl)-4H-1,2,4-triazole-3-yl)propan-1-ol (Compound 31)

1-Naphthyl isothiocyanate (370 mg, 2.00 mmol) was weighed, 4-hydroxybutyric acid hydrazide (248 mg, 2.10 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(5-mercapto-4-(naphthalen-1-yl)-4H-1,2,4-triazole-3-yl)propan-1-ol (crude product) as a white solid (352 mg, yield: 62%).

3-(5-Mercapto-4-(naphthalen-1-yl)-4H-1,2,4-triazole-3-yl)propan-1-ol (180 mg, 0.60 mmol) was weighed, potassium carbonate solid (276 mg, 2.00 mmol) was added, 8 mL of acetone was added, iodomethane (55 µL, 0.87 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(5-(methylthio)-4-(naphthalen-1-yl)-4H-1,2,4-triazole-3-yl)propan-1-ol as a white solid (97 mg, yield: 54%). ¹H NMR (400 MHz, DMSO-*d*₆) δ 8.17 (ddd, *J* = 28.4, 8.1, 3.6 Hz, 2H), 7.75-7.57 (m, 4H), 7.11 (dd, *J* = 8.6, 3.1 Hz, 1H), 4.41 (q, *J* = 4.7 Hz, 1H), 3.36 (d, *J* = 3.5 Hz, 2H), 2.52 (s, 3H), 2.48-2.29 (m, 2H), 1.65 (ddt, *J* = 15.0, 7.7, 3.6 Hz, 2H). ¹³C NMR (101 MHz, DMSO-*d*₆) δ 156.78, 152.07, 134.31, 131.15, 129.51, 129.16, 128.77, 127.68, 127.14, 126.34, 121.68, 60.13, 30.14, 21.87, 14.97. HRMS (ESI) calcd for C₁₆H₁₇N₃OS [M+H]⁺: 300.1171; found: 300.1155.

### Example 32: Synthesis of 3-(4-benzyl-5-(methylthio)-4H-1,2,4-triazole-3-yl)propan-1-ol (Compound 32)

Benzylisothiocyanate (300 mg, 2.01 mmol) was weighed, 4-hydroxybutyric acid hydrazide (237 mg, 2.01 mmol) was added, 6 mL of absolute ethanol was added, and the resulting mixture was refluxed overnight. After the complete conversion of the reaction was detected, ethanol was evaporated to dryness via rotary evaporation, 8 mL of 2M aqueous sodium hydroxide solution was then added immediately, and the resulting mixture was refluxed overnight. After cooling, 2M aqueous HCl solution was added slowly until the solution became acidic, and a large amount of white solid appeared. The resultant was subjected to suction filtration, washed with water and then subjected to vacuum drying, so as to obtain 3-(4-benzyl-5-mercapto-4H-1,2,4-triazole-3-yl)propan-1-ol (crude product) as a white solid (323 mg, yield: 64%).

3-(4-Benzyl-5-mercapto-4H-1,2,4-triazole-3-yl)propan-1-ol (100 mg, 0.40 mmol) was weighed, potassium carbonate solid (138 mg, 1.00 mmol) was added, 8 mL of acetone was added, iodomethane (37 µL, 0.60 mmol) was added dropwise under stirring by a rotor, and the resulting mixture was reacted overnight at room temperature. After rotary evaporation, the resultant was subjected to silica gel column chromatography (dichloromethane : methanol = 20 : 1), so as to obtain 3-(4-benzyl-5-(methylthio)-4H-1,2,4-triazole-3-yl)propan-1-ol as a white solid (97 mg, yield: 53%). ¹H NMR (400 MHz, DMSO-*d*₆) δ 7.37 (t, *J* = 7.3 Hz, 2H), 7.31 (t, *J* = 7.3 Hz, 1H), 7.08 (d, *J* = 7.1 Hz, 2H), 5.15 (s, 2H), 4.53 (t, *J* = 5.2 Hz, 1H), 3.43 (q, *J* = 6.1 Hz, 2H), 2.73-2.64 (m, 2H), 2.55 (s, 3H), 1.76 (dq, *J* = 7.5, 6.3 Hz, 2H). ¹³C NMR (101 MHz, DMSO-*d*₆) δ 156.28, 150.72, 136.10, 129.32, 128.27, 126.92, 60.26, 46.59, 30.24, 21.60, 15.68. HRMS (ESI) calcd for C₁₃H₁₇N₃OS[M+H]⁺: 264.1171; found: 264.1168.

### Example 33: Synthesis of 3-(5-((2,6-dichlorobenzyl)thio)-4-(2,4-difluorophenyl)-4H-1,2,4-triazole-3-yl)propan-1-ol (Compound 33)

The intermediate product 3-(4-(2,4-difluorophenyl)-5-mercapto-4H-1,2,4-triazole-3-yl)propan-1-ol (100 mg, 0.33 mmol) in Example 15 was weighed, potassium carbonate solid (136 mg, 0.99 mmol) was added, 2,6-dichlorobenzyl bromide (87 mg, 0.36 mmol) was added, 8 mL of acetonitrile was added, and the resulting mixture was reacted overnight at room temperature under stirring by a rotor. After rotary evaporation, the resultant was subjected to silica gel column chromatography with gradient elution (dichloromethane : methanol = 200 : 1 to 40 : 1), so as to obtain 3-(5-((2,6-dichlorobenzyl)thio)-4-(2,4-difluorophenyl)-4H-1,2,4-triazole-3-yl)propan-1-ol as a white solid (59 mg, yield: 42%). ¹H NMR (400 MHz, DMSO-*d*₆) δ 7.70-7.57 (m, 2H), 7.46 (d, J = 7.7 Hz, 2H), 7.38-7.29 (m, 2H), 4.48 (t, J = 5.1 Hz, 1H), 4.39 (s, 2H), 3.38 (q, J = 5.9 Hz, 2H), 2.56 (td, J = 7.6, 3.8 Hz, 2H), 1.77-1.66 (m, 2H). HRMS (ESI) calcd for C₁₈H₁₅Cl₂F₂N₃OS[M+H]⁺: 430.0359; found: 430.0361.

### Example 34: Synthesis of 4-(5-((2,6-dichlorobenzyl)thio)-4-(2,4-difluorophenyl)-4H-1,2,4-triazole-3-yl)butan-1-ol (Compound 34)

2,4-Difluoro-1-isothiocyanatobenzene (342 mg, 2.00 mmol) was weighed and charged into a 25-mL round-bottom flask, 5-hydroxyvaleric acid hydrazine (264 mg, 2.00 mmol) was added, 8 mL of absolute ethanol was added, and the resulting mixture was refluxed overnight. The ethanol solution was evaporated to dryness via rotary evaporation, 8 mL of 2M aqueous sodium hydroxide solution was then added immediately, and the resulting mixture was refluxed overnight. After the reaction system was cooled, 2M aqueous HCl solution was added slowly until the solution became acidic, and a large amount of white solid appeared. The resultant was subjected to suction filtration, washed with water and then subjected to vacuum drying, so as to obtain 4-(4-(2,4-difluorophenyl)-5-mercapto-4H-1,2,4-triazole-3-yl)butan-1-ol. 4-(4-(2,4-Difluorophenyl)-5-mercapto-4H-1,2,4-triazole-3-yl)butan-1-ol (100 mg, 0.31 mmol) was weighed, potassium carbonate solid (130 mg, 0.94 mmol) was added, 2,6-dichlorobenzyl bromide (83 mg, 0.35 mmol) was added, 8 mL of acetonitrile was added, and the resulting mixture was reacted overnight at room temperature under stirring by a rotor. After rotary evaporation, the resultant was subjected to silica gel column chromatography (dichloromethane : methanol = 20 : 1), so as to obtain a white solid (106 mg, yield: 76%). ¹H NMR (400 MHz, DMSO-*d*₆) δ 7.63 (dtt, J = 14.7, 8.8, 4.3 Hz, 2H), 7.47-7.42 (m, 2H), 7.33 (ddd, J = 12.5, 9.0, 6.9 Hz, 2H), 4.40-4.37 (s, 2H), 4.36 (m, 1H), 3.34-3.27 (m, 2H), 2.54 (td, J = 7.4, 3.4 Hz, 2H), 1.58 (q, J = 7.6 Hz, 2H), 1.44-1.34 (m, 2H). HRMS (ESI) calcd for C₁₉H₁₇Cl₂F₂N₃OS[M+H]⁺: 444.0516; found: 444.0516.

### Example 35: Synthesis of S-(4-(2,4-dichlorophenyl)-5-(3-hydroxypropyl)-4H-1,2,4-triazole-3-yl)carbamate (Compound 35)

3-(4-(2,4-Dichlorophenyl)-5-mercapto-4H-1,2,4-triazole-3-yl)propan-1-ol (100 mg, 0.33 mmol) synthesized in Example 10 was weighed, trichloroacetyl chloride (120 mg, 0.66 mmol) was added, and 5 mL of acetonitrile and 1 mL of DMF were added. After one hour, it was detected that the raw materials in the system had been completely converted. The reaction was stopped, the solvent was removed by rotary evaporation, and 7M ammonia methanol solution (10 mL) was added. The mixture was refluxed for 3h to enable the completion of the reaction, followed by rotary evaporation under reduced pressure, and the resultant was subjected to silica gel column chromatography (petroleum ether : acetone =3:1), so as to obtain a white solid (77 mg, yield: 67%). ¹H NMR (400 MHz, DMSO-*d*₆) δ 7.98 (d, J = 2.2 Hz, 1H), 7.71-7.62 (m, 2H), 4.51 (t, J = 5.1 Hz, 1H), 2.51 (m, 2H), 2.36 (dd, J = 8.5, 6.8 Hz, 2H), 1.65 (dq, J = 8.1, 6.3 Hz, 2H). ¹³C NMR (101 MHz, DMSO-*d*₆) 1 168.07, 152.63, 136.15, 133.70, 133.11, 132.45, 130.80, 130.52, 129.34, 59.88, 28.89, 22.39.

### Example 36: Synthesis of 3-(5-(methylthio)-4-(quinolin-3-yl)-4H-1,2,4-triazole-3-yl)propan-1-ol (Compound 36)

3-Aminoquinoline (577 mg, 4.00 mmol) was weighed and charged into a 25-mL single-neck flask. A magnetic stirring bar was added, 3-aminoquinoline was dissolved in 10 mL of tetrahydrofuran, triethylamine (1.22 g, 12.00 mmol) was added, and the resulting mixture was stirred in an ice bath. Thiophosgene (336 µL, 4.40 mmol) was slowly added dropwise, and the resultant was reacted in an ice bath for 2h. A sample was taken for detection, and after the complete reaction of the raw materials, 2M aqueous sodium hydroxide solution was slowly added dropwise to quench the reaction, and the resultant was then extracted with dichloromethane (3 × 10 mL) and saturated aqueous sodium bicarbonate solution (10 mL). The organic phases were washed with saturated sodium chloride solution, dried over anhydrous sodium sulfate, and concentrated via rotary evaporation. Afterwards, the resultant was subjected to silica gel column chromatography with gradient elution (petroleum ether : ethyl acetate = 20 : 1 to 10 : 1), so as to obtain 3-isothiocyanatoquinoline as a colorless liquid (300 mg, yield: 40%). ¹H NMR (400 MHz, chloroform-d) δ 8.80 (d, J = 2.4 Hz, 1H), 8.12 (d, J = 8.5 Hz, 1H), 7.96 (d, J = 2.5 Hz, 1H), 7.82-7.72 (m, 2H), 7.62 (ddd, J = 8.1, 7.1, 1.1 Hz, 1H). ¹³C NMR (101 MHz, chloroform-*d*) δ 147.71, 146.03, 138.99, 130.35, 130.06, 129.56, 128.01, 127.49, 127.40, 125.98.

3-Isothiocyanatoquinoline (253 mg, 1.36 mmol) was weighed, 4-hydroxybutyric acid hydrazide (169 mg, 1.43 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of pale yellow solid appeared. The resultant was subjected to suction filtration, washed with water and then subjected to vacuum drying, so as to obtain 3-(5-mercapto-4-(quinolin-3-yl)-4H-1,2,4-triazole-3-yl)propan-1-ol as a white solid (270 mg, yield: 70%). ¹H NMR (400 MHz, DMSO-*d*₆) δ 13.89 (s, 1H), 8.93 (d, J = 2.4 Hz, 1H), 8.61 (d, J = 2.4 Hz, 1H), 8.17-8.08 (m, 2H), 7.92 (ddd, J = 8.5, 6.9, 1.5 Hz, 1H), 7.75 (ddd, J = 8.1, 6.8, 1.1 Hz, 1H), 3.36 (t, J = 6.1 Hz, 2H), 2.59-2.52 (m, 2H), 1.73-1.65 (m, 2H). ¹³C NMR (101 MHz, DMSO-*d*₆) δ 168.46, 152.92, 150.22, 147.55, 135.86, 131.51, 129.31, 129.02, 128.12, 128.04, 127.66, 59.91, 28.86, 22.67.

3-(5-Mercapto-4-(quinolin-3-yl)-4H-1,2,4-triazole-3-yl)propan-1-ol (200 mg, 0.70 mmol) was weighed, potassium carbonate solid (138 mg, 1.00 mmol) was added, 8 mL of acetone was added, iodomethane (65 µL, 0.74 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 3-(5-(methylthio)-4-(quinolin-3-yl)-4H-1,2,4-triazole-3-yl)propan-1-ol as a pale yellow solid powder (174 mg, yield: 83%). ¹H NMR (400 MHz, DMSO-*d*₆) δ 8.97 (t, J = 2.2 Hz, 1H), 8.68 (s, 1H), 8.20-8.10 (m, 2H), 7.94 (ddt, J = 8.5, 7.0, 1.6 Hz, 1H), 7.82-7.72 (m, 1H), 4.47 (td, J = 5.2, 1.8 Hz, 1H), 3.39 (d, J = 4.6 Hz, 2H), 2.65-2.59 (m, 2H), 2.57 (d, J = 1.9 Hz, 3H), 1.78-1.68 (m, 2H).

### Example 37: Synthesis of 3-(5-(methylthio)-4-(quinolin-6-yl)-4H-1,2,4-triazole-3-yl)propan-1-ol (Compound 37)

6-Aminoquinoline (577 mg, 4.00 mmol) was weighed and charged into a 25-mL single-neck flask. A magnetic stirring bar was added, 6-aminoquinoline was dissolved in 10 mL of tetrahydrofuran, triethylamine (1.22 g, 12.00 mmol) was added, and the resulting mixture was stirred in an ice bath. Thiophosgene (336 µL, 4.40 mmol) was slowly added dropwise, and the resultant was reacted in an ice bath for 2h. A sample was taken for detection, and after the complete reaction of the raw materials, 2M aqueous sodium hydroxide solution was slowly added dropwise to quench the reaction, and then the resultant was extracted with dichloromethane and saturated aqueous sodium bicarbonate solution. The organic phase was washed with saturated sodium chloride solution, dried over anhydrous sodium sulfate, and concentrated via rotary evaporation. Afterwards, the resultant was subjected to silica gel column chromatography with gradient elution (petroleum ether : ethyl acetate = 20 : 1 to 10 : 1), so as to obtain 6-isothiocyanatoquinoline as a colorless liquid (520 mg, yield: 70%). ¹H NMR (400 MHz, chloroform-*d*) h 8.94 (dd, J = 4.2, 1.7 Hz, 1H), 8.15-8.07 (m, 2H), 7.67 (d, J = 2.3 Hz, 1H), 7.56 (dd, J = 9.0, 2.3 Hz, 1H), 7.46 (dd, J = 8.4, 4.2 Hz, 1H). ¹³C NMR (101 MHz, chloroform-*d*) δ 151.03, 146.66, 136.92, 135.46, 131.44, 129.45, 128.38, 127.36, 123.53, 122.23, 76.72.

6-Isothiocyanatoquinoline (300 mg, 1.61 mmol) was weighed, 4-hydroxybutyric acid hydrazide (200 mg, 1.69 mmol) was added, 8 mL of absolute ethanol was added, and the resulting mixture was refluxed overnight. After the complete conversion of the reaction was detected, the ethanol solution was evaporated to dryness via rotary evaporation, 8 mL of 2M aqueous sodium hydroxide solution was then added immediately, and the resulting mixture was refluxed overnight. After the complete conversion of the reaction was detected, 2M aqueous HCl solution was added slowly until the solution became acidic, and a large amount of yellow solid appeared. The resultant was subjected to suction filtration, washed with water and then subjected to vacuum drying, so as to obtain 3-(5-mercapto-4-(quinolin-6-yl)-4H-1,2,4-triazole-3-yl)propan-1-ol as a yellow solid (380 mg, yield: 82%). ¹H NMR (400 MHz, DMSO-*d*₆) δ 13.80 (s, 1H), 9.04 (dd, J = 4.2, 1.7 Hz, 1H), 8.48 (dd, J = 8.4, 1.7 Hz, 1H), 8.22-8.14 (m, 2H), 7.80 (dd, J = 8.9, 2.4 Hz, 1H), 7.66 (dd, J = 8.3, 4.2 Hz, 1H), 4.47 (s, 1H), 3.34 (t, J = 6.1 Hz, 2H), 2.53 (d, J = 8.5 Hz, 2H), 1.72-1.61 (m, 2H). ¹³C NMR (101 MHz, DMSO-*d*₆) δ 168.22, 152.79, 152.45, 147.74, 136.95, 132.09, 130.72, 129.97, 128.35, 128.28, 122.72, 59.92, 28.92, 22.71.

3-(5-Mercapto-4-(quinolin-6-yl)-4H-1,2,4-triazole-3-yl)propan-1-ol (200 mg, 0.70 mmol) was weighed, potassium carbonate solid (138 mg, 1.00 mmol) was added, 8 mL of acetone was added, iodomethane (65 µL, 0.74 mmol) was added dropwise under stirring by a rotor, and the resulting mixture was reacted overnight at room temperature. After rotary evaporation, the resultant was subjected to silica gel column chromatography (dichloromethane : methanol = 30 : 1), so as to obtain 3-(5-(methylthio)-4-(quinolin-6-yl)-4H-1,2,4-triazole-3-yl)propan-1-ol as a pale yellow solid powder (171 mg, yield: 83%). ¹H NMR (400 MHz, DMSO-*d*₆) δ 9.05 (dd, J = 4.2, 1.7 Hz, 1H), 8.49 (dd, J = 8.4, 1.7 Hz, 1H), 8.22 (d, J = 8.9 Hz, 1H), 8.19 (d, J = 2.4 Hz, 1H), 7.81 (dd, J = 8.9, 2.4 Hz, 1H), 7.68 (dd, J = 8.3, 4.2 Hz, 1H), 4.46 (t, J = 5.2 Hz, 1H), 3.38 (d, J = 5.9 Hz, 2H), 2.62 (dd, J = 8.3, 7.1 Hz, 2H), 2.56 (s, 3H), 1.75-1.67 (m, 2H). ¹³C NMR (101 MHz, DMSO-*d*₆) 0 156.17, 152.73, 151.26, 147.77, 136.98, 131.44, 131.36, 128.72, 128.38, 127.40, 123.08, 60.17, 30.03, 21.99, 15.08.

### Example 38: Synthesis of 2-(ethoxymethyl)-1-(2-hydroxy-2-methylpropyl)-5-phenyl-1H-imidazole-4-carbonitrile (Compound 38)

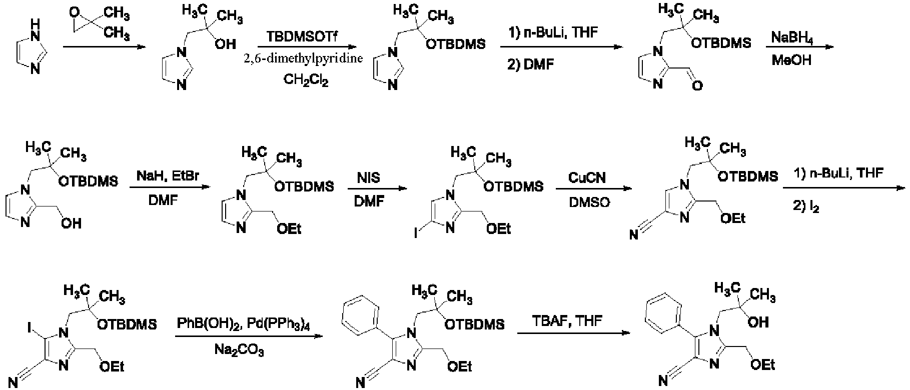

### (1) Synthesis of 1-{2-[(tert-butyldimethylsilyl)oxy]-2-methylpropyl}-1H-imidazole

At room temperature, 1-(1H-imidazol-1-yl)-2-methylpropan-2-ol (10.3 g, 73.5mmol, prepared according to the method reported in Tetrahedron, 63 (2), 2007, 469-473) and 2,6-dimethylpyridine (60.0 mL, 515.0 mmol) were dissolved in dichloromethane (900 mL), trifluoromethanesulfonic acid tert-butyldimethylsilyl ester (84.5 mL, 368.0 mmol) was added via an addition funnel over 10 min, and the reaction mixture was stirred at room temperature for 16 hours. Afterwards, the reaction mixture was concentrated to approximately 400 mL, washed with water (5 × 400 mL), washed with saturated aqueous sodium chloride solution (400 mL), dried over anhydrous sodium sulfate, filtered and evaporated to dryness via rotary evaporation. The resulting residue was subjected to azeotropic distillation with toluene (3 × 400 mL) and purified by flash SiO₂ chromatography (100 g of silica gel, gradient elution: dichloromethane to 4% methanol/96% dichloromethane), so as to obtain a red oil (18.1g, yield: 97%). ¹H NMR (400 MHz, DMSO-*d*₆) δ 7.51 (s, 1H), 7.06 (t, *J* = 1.1 Hz, 1H), 6.86 (s, 1H), 3.88 (s, 2H), 1.15 (s, 6H), 0.83 (s, 9H), 0.01 (s, 6H); ¹³C NMR (101 MHz DMSO-*d*₆) δ 138.30, 127.41, 120.76, 72.90, 57.87, 26.96, 25.74, 17.71, -2.31; MS (ESI⁺), calcd for C₁₃H₂₆N₂OSi (M+H): 255.1893; found: 255.1885.

### (2) Synthesis of 1-{2-[(tert-butyldimethylsilyl)oxy]-2-methylpropyl}-2-(ethoxymethyl)-1H-imidazole

A solution of 1-{2-[(tert-butyldimethylsilyl)oxy]-2-methylpropyl}-1H-imidazole (15.35g, 60.33 mmol) in tetrahydrofuran (735 mL) was cooled to -78°C in a dry ice/isopropanol bath. n-Butyllithium (36 mL, 91 mmol, 2.5M solution in hexane) was added slowly over 20 minutes, the internal temperature was kept at -55°C or lower, and the resulting solution was stirred at the same temperature for 40 minutes. Afterwards, the reaction flask was transferred to the dry ice/isopropanol bath (maintained between -40°C and -25°C) and the mixture therein was stirred for 1 hour. Afterwards, the reaction flask was put back into an environment at -78°C and the mixture therein was stirred until the internal temperature reached -65°C. At this time, N,N-dimethylformamide (9.40 mL, 121 mmol) was added dropwise, and the resulting mixture was stirred for 30 minutes. Afterwards, the reactant was stirred at room temperature for 18 hours. After the completion of the reaction, saturated aqueous ammonium chloride solution (100 mL) and water (100 mL) were added. The entire biphasic mixture was concentrated via rotary evaporation to remove approximately 500 mL of tetrahydrofuran. Saturated aqueous ammonium chloride solution (300 mL) was added for further dilution, and the resultant was extracted with ethyl acetate (3 × 200 mL). The combined organic layers were washed with saturated aqueous sodium chloride solution (300 mL), dried over magnesium sulfate, filtered and concentrated to dryness, so as to obtain crude 1-{2-[(tert-butyldimethylsilyl)oxy]-2-methylpropyl}-1H-imidazole-2-carbaldehyde as a yellow oil. This oil was dissolved in methanol (450 mL) and cooled in an ice bath, sodium borohydride (3.42g, 90.5 mmol) was added and the resulting mixture was kept in the ice bath for 5 minutes. Afterwards, the ice bath was removed, and the reactant was stirred at room temperature for 18.5 hours. Afterwards, saturated aqueous ammonium chloride solution (300 mL) was added, and the resulting mixture was extracted with dichloromethane (3 × 200 mL). The combined organic layers were washed with saturated aqueous sodium chloride solution (400 mL), dried over sodium sulfate, filtered and concentrated to dryness. The resulting off-white solid was subjected to azeotropic distillation with toluene (400 mL), and then subjected to rotary evaporation via a high vacuum pump for several hours, so as to obtain (1-{2-[(tert-butyldimethylsilyl)oxy]-2-methylpropyl}-1H-imidazol-2-yl) as a crude product. This solid was dissolved in N,N-dimethylformamide (450 mL) and cooled in an ice bath (≈0°C). Sodium hydride (3.62 g, 90.5 mmol, 60% dispersed in mineral oil) was added and the resulting mixture was stirred in an ice bath for 30 minutes. Afterwards, bromoethane (9.0 mL, 120 mmol) was added, the ice bath was then removed, and the reactant was allowed to react at room temperature for 18 hours. Afterwards, saturated aqueous ammonium chloride solution (100 mL) and water (150 mL) were added, and the resultant was extracted with ethyl acetate (3 × 200 mL). Then, the combined organic layers were washed with 5% aqueous lithium chloride solution (3 × 200 mL), washed with saturated aqueous sodium chloride solution (300 mL), dried over magnesium sulfate, filtered and concentrated to dryness. The resulting residue was purified by flash SiO₂ chromatography (340 g of silica gel, gradient elution: dichloromethane to 5% methanol/95% dichloromethane), so as to obtain an orange oil (13.45 g, yield for three steps: 71%). ¹H NMR (400 MHz, DMSO-*d*₆) δ 7.11 (d, *J* = 1.2 Hz, 1H), 6.82 (d, *J* = 1.2 Hz, 1H), 4.46 (s, 2H), 3.92 (s, 2H), 3.42 (q, *J* = 7.0 Hz, 2H), 1.19 (s, 6H), 1.08 (t, *J* = 7.0 Hz, 3H), 0.82 (s, 9H), 0.04 (s, 6H); ¹³C NMR (101 MHz DMSO-*d*₆) δ 144.76, 126.18, 121.86, 73.53, 64.74, 63.80, 56.56, 27.22, 25.77, 17.70, 14.90, -2.23; MS (ESI⁺), calcd for C₁₆H₃₃N₂O₂Si (M+H): 313.2311, found: 313.2316.

### (3) Synthesis of 1-{2-[(tert-butyldimethylsilyl)oxy]-2-methylpropyl}-2-(ethoxymethyl)-4-iodo-1H-imidazole

### 1-{2-[(Tert-butyldimethylsilyl)oxy]-2-methylpropyl}-2-(ethoxymethyl)-1H-imidazole

(3.00 g, 9.60 mmol) was dissolved in N,N-dimethylformamide (60 mL) and heated to 80°C. N-Iodosuccinimide (4.32g, 19.2 mmol) was dissolved in N,N-dimethylformamide (30 mL), the resultant was slowly added to the original reaction flask over 5 minutes, and the reaction mixture was stirred at 80°C for 16 hours. Afterwards, the reaction system was cooled to room temperature, 100 mL of water was added, sodium thiosulfate pentahydrate (solid) was added until no further change in color was observed, and a pale yellow suspension was obtained. The resulting mixture was extracted with ethyl acetate (3 × 70 mL). Afterwards, the combined organic layers were washed with 5% aqueous lithium chloride solution (3 × 150 mL), washed with 200 mL of saturated aqueous sodium chloride solution, dried over magnesium chloride, filtered and concentrated to dryness. The resultant was purified by flash SiO₂ chromatography (100 g of silica gel, gradient elution: hexane to 25% ethyl acetate/75% hexane), so as to obtain a pale yellow oil (2.44g, yield: 58%). ¹H NMR (400 MHz, DMSO-*d*₆) δ 7.25 (s, 1H), 4.44 (s, 2H), 3.91 (s, 2H), 3.42 (q, *J* = 7.0 Hz, 2H), 1.17 (s, 6H), 1.08 (t, *J* = 7.0 Hz, 3H), 0.83 (s, 9H), 0.04 (s, 6H); ¹³C NMR (101 MHz DMSO-*d*₆) δ 147.02, 127.62, 80.04, 73.43, 64.94, 63.11, 56.66, 27.05, 25.77, 17.75, 14.90, -2.26; MS (ESI⁺), calcd for C₁₆H₃₂IN₂O₂Si (M+H): 439.1278, found: 439.1274.

### (4) Synthesis of 1-{2-[(tert-butyldimethylsilyl)oxy]-2-methylpropyl}-2-(ethoxymethyl)-1H-imidazole-4-carbonitrile

1-{2-[(Tert-butyldimethylsilyl)oxy]-2-methylpropyl}-2-(ethoxymethyl)-4-iodo-1H-imidazole (2.00g, 4.56 mmol) was dissolved in dimethyl sulfoxide (60 mL), the air inside the system was repeatedly purged with nitrogen gas (× 3), copper (I) cyanide (0.817 g, 9.12 mmol) was added, the resulting mixture was degassed, and the system was backfilled again with nitrogen gas. The reaction system was heated at 150°C for 17 hours, and then cooled to room temperature. Ethyl acetate (300 mL) was added and the resulting mixture was filtered through a silica gel plug (about 70 g). The filtrate was washed with water (3 × 200 mL), washed with 200 mL of saturated aqueous sodium chloride solution, dried over magnesium sulfate, and filtered through a silica gel plug (about 70 g, eluted with ethyl acetate), so as to obtain a yellow oil (1.467 g, yield: 95%). ¹H NMR (400 MHz, DMSO-*d*₆) δ 7.98 (s, 1H), 4.52 (s, 2H), 4.02 (s, 2H), 3.45 (q, *J* = 7.0 Hz, 2H), 1.21 (s, 6H), 1.09 (t, *J* = 7.0 Hz, 3H), 0.79 (s, 9H), 0.03 (s, 6H); ¹³C NMR (101 MHz DMSO-*d*₆) δ 147.38, 131.98, 115.47, 109.95, 73.14, 65.25, 63.21, 57.05, 27.16, 25.74, 17.69, 14.85, -2.28; MS (ESI⁺), calcd for C₁₇H₃₂N₃O₂Si (M+H): 338.2264, found: 338.2257.

### (5) Synthesis of 1-{2-[(tert-butyldimethylsilyl)oxy]-2-methylpropyl}-2-(ethoxymethyl)-5-iodo-1H-imidazole-4-carbonitrile

1-{2-[(Tert-butyldimethylsilyl)oxy]-2-methylpropyl}-2-(ethoxymethyl)-1H-imidazole-4-carbonitrile (1.467g, 4.346 mmol) was charged into a dry flask, 60 mL of tetrahydrofuran was added for dissolution, and the mixture was cooled in a dry ice/isopropanol bath (approximately -78°C). n-Butyllithium (3.0 mL, 4.8 mmol, 1.6M solution in hexane) was slowly added over 5 minutes, and the reaction mixture was stirred in an ice bath for 30 minutes. Afterwards, a solution of iodine (2.21 g, 8.69 mmol) in tetrahydrofuran (9 mL) was added slowly over 10 minutes. The reaction mixture was further stirred in the dry ice bath for 30 minutes, and the reactant was stirred at room temperature for 17 hours. Afterwards, saturated aqueous ammonium chloride solution (60 mL) was added, and the resulting mixture was extracted with ethyl acetate (3 × 40 mL). The combined organic layers were washed with sodium thiosulfate pentahydrate (100 mL, 1M), washed with saturated aqueous sodium chloride solution (100 mL), dried over magnesium sulfate, filtered and concentrated to dryness. The resulting residue was purified by flash SiO₂ chromatography (100 g of silica gel, gradient elution: dichloromethane to 6% ethyl acetate/94% dichloromethane), so as to obtain an orange solid (1.928g, yield: 96%). ¹H NMR (400 MHz, DMSO-*d*₆) δ 4.63 (s, 2H), 4.10 (s, 2H), 3.43 (q, *J* = 7.0 Hz, 2H), 1.32 (s, 6H), 1.09 (t, *J* = 7.0 Hz, 3H), 0.72 (s, 9H), 0.06 (s, 6H); ¹³C NMR (101 MHz DMSO-*d*₆) δ 150.64, 119.51, 115.66, 91.76, 73.93, 65.38, 64.55, 57.23, 28.93, 25.87, 17.75, 14.87, -2.05; MS (ESI⁺), calcd for C₁₇H₃₁IN₃O₂Si (M+H): 464.1230, found: 464.1225.

### (6) Synthesis of 1-{2-[(tert-butyldimethylsilyl)oxy]-2-methylpropyl}-2-(ethoxymethyl)-5-phenyl-1H-imidazole-4-carbonitrile

1-{2-[(Tert-butyldimethylsilyl)oxy]-2-methylpropyl}-2-(ethoxymethyl)-5-iodo-1H-imidazole-4-carbonitrile (2.239 g, 4.831 mmol) and phenylboronic acid (0.647g, 5.31 mmol) were dissolved in 1,4-dioxane (63 mL), and aqueous sodium carbonate solution (32 mL, 1M) was added. The reaction solution was degassed and backfilled with nitrogen gas (4×). Tetrakis(triphenylphosphine)palladium(0) was added, and the resulting mixture was degassed and backfilled with nitrogen gas (4×) again. The mixture was heated at 80°C for 20 hours. Afterwards, the reaction mixture was cooled to room temperature, diluted with water (200 mL), and extracted with ethyl acetate (3 × 100 mL). The combined organic layers were washed with saturated aqueous sodium chloride solution (200 mL), dried over magnesium sulfate, filtered and concentrated to dryness. The resultant was purified by flash SiO₂ chromatography (100 g of silica gel, gradient elution: hexane to 15% ethyl acetate/85% hexane), so as to obtain a yellow oil (1.99g, yield: 99%). ¹H NMR (400 MHz, DMSO-*d*₆) δ 7.61-7.51 (m, 5H), 4.64 (s, 2H), 4.23 (s, 2H), 3.52 (q, *J* = 7.0 Hz, 2H), 1.15 (t, *J* =7.0 Hz, 3H), 0.86 (s, 6H), 0.70 (s, 9H), 0.03 (s, 6H); ¹³C NMR (101 MHz DMSO-*d*₆) δ 148.97, 142.61, 129.76, 129.36, 129.17, 127.49, 115.81, 110.52, 73.60, 65.51, 64.56, 54.77, 28.19, 25.72, 17.65, 14.91, -2.27; MS (ESI⁺), calcd for C₂₃H₃₆N₃O₂Si (M+H): 414.2577, found: 414.2569.

### (7) Synthesis of 2-(ethoxymethyl)-1-(2-hydroxy-2-methylpropyl)-5-phenyl-1H-imidazole-4-carbonitrile

To a solution of 1-{2-[(tert-butyldimethylsilyl)oxy]-2-methylpropyl}-2-(ethoxymethyl)-5-phenyl-1H-imidazole-4-carbonitrile (0.050g, 0.12 mmol) in tetrahydrofuran (2 mL), tetrabutylammonium fluoride (0.36 mL, 0.36 mmol, 1M solution in tetrahydrofuran) was added. The solution was stirred at room temperature for 18 hours. Afterwards, saturated aqueous ammonium chloride solution (5 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resultant was purified by flash SiO₂ chromatography (10 g of silica gel, gradient elution: hexane to 70% ethyl acetate/30% hexane), so as to obtain a white solid (0.028g, yield: 78%). ¹H NMR (400 MHz, DMSO-*d*₆) δ 7.59-7.50 (m, 5H), 4.80 (s, 1H), 4.72 (s, 2H), 4.13 (s, 2H), 3.52 (q, *J* = 7.0 Hz, 2H), 1.15 (t, *J* = 7.0 Hz, 3H), 0.75 (s, 6H); ¹³C NMR (101 MHz DMSO-*d*₆) δ 149.06, 142.88, 129.58, 129.43, 129.13, 127.50, 115.87, 110.36, 69.65, 65.38, 64.47, 53.94, 27.57, 14.95; MS (ESI⁺), calcd for C₁₇H₂₂N₃O₂ (M+H): 300.1712, found: 300.1717.

### Example 39: Synthesis of 2-(ethoxymethyl)-1-(2-hydroxy-2-methylpropyl)-5-phenyl-1H-imidazole-4-carboxamidine (Compound 39)

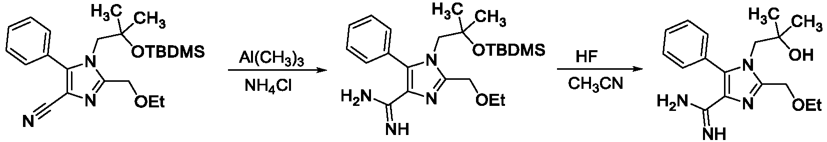

### (1) Synthesis of 1-[2-[(tert-butyldimethylsilyl)oxy]-2-methylpropyl]-2-(ethoxymethyl)-5-phenyl-1H-imidazole-4-carboxamidine

Ammonium chloride (0.026g, 0.48 mmol, solid) was dissolved in toluene (4 mL), and the mixture was cooled in an ice bath (≈0°C). Trimethylaluminum (0.73 mL, 0.73 mmol, 1M solution in heptane) was added over 2 minutes, and the resulting mixture was stirred in an ice bath for 30 minutes and then stirred at room temperature for 90 minutes. Afterwards, 1-[2-[(tert-butyldimethylsilyl)oxy]-2-methylpropyl}-2-(ethoxymethyl)-5-phenyl-1H-imidazole -4-carbonitrile (0.100g, 0.242 mmol) was dissolved in tetrahydrofuran (1.5 mL) and heated at 80°C for 17 hours. Afterwards, the solution was cooled to room temperature, saturated aqueous sodium bicarbonate solution (10 mL) was added, followed by the addition of saturated aqueous potassium sodium tartrate solution (30 mL), and the resultant was extracted with ethyl acetate (3 × 30 mL). The combined organic layers were washed with saturated aqueous sodium chloride solution (50 mL), dried over magnesium sulfate, filtered and concentrated to dryness. The resulting residue was purified by flash SiO₂ chromatography (10 g of silica gel, gradient elution: dichloromethane to 20% [1% triethylamine/99% methanol]/80% dichloromethane), so as to obtain a white solid (0.075g, yield: 72%). ¹H NMR (400 MHz, DMSO-*d*₆) δ 8.38 (br s, 3H), 7.60-7.55 (m, 3H), 7.54-7.50 (m, 2H), 4.70 (s, 2H), 4.12 (s, 2H), 3.55 (q, *J* = 7.0 Hz, 2H), 1.16 (t, *J* = 7.0 Hz, 3H), 0.83 (s, 6H), 0.75 (s, 9H), -0.01 (s, 6H); ¹³C NMR (101 MHz DMSO-*d*₆) δ 159.50, 147.67, 137.48, 130.84, 130.05, 129.33, 127.37, 125.33, 73.57, 65.60, 64.54, 54.50, 28.21, 25.84, 17.73, 14.96, -2.17; MS (ESI⁺), calcd for C₂₃H₃₉N₄O₂Si (M+H): 431.2842, found: 431.2841.

### (2) Synthesis of 2-(ethoxymethyl)-1-(2-hydroxy-2-methylpropyl)-5-phenyl-1H-imidazole-4-carboxamidine

To a solution of 1-{2-[(tert-butyldimethylsilyl)oxy]-2-methylpropyl}-2-(ethoxymethyl)-5-phenyl-1H-imidazole-4-carboxamidine (0.098g, 0.23 mmol) in acetonitrile (in an HDPE scintillation vial), 5 mL of hydrofluoric acid (5 mL, 48 wt%, in water) was added, and the solution was stirred at room temperature for 21 hours. Afterwards, the solution was added to a mixture of sodium carbonate (50.0 g, 472 mmol) in acetonitrile (100 mL, cooled to approximately 0°C in an ice bath) and the resulting mixture was stirred for 30 minutes. The solution was filtered, and the filtrate was concentrated to dryness. The resulting residue was purified by flash SiO₂ chromatography (10 g of silica gel, gradient elution: dichloromethane to 20% [1% triethylamine/99% methanol]/80% dichloromethane) three times, so as to obtain a white solid (0.015g, yield: 21%). ¹H NMR (400 MHz, DMSO-*d*₆) δ 8.44 (br s, 3H), 7.58-7.47 (m, 5H), 4.88 (s, 1H), 4.77 (s, 2H), 4.03 (s, 2H), 3.55 (q, *J* = 7.0 Hz, 2H), 1.16 (t, *J* = 7.0 Hz, 3H), 0.74 (s, 6H); ¹³C NMR (101 MHz DMSO-*d*₆) δ 159.95, 148.05, 137.53, 130.92, 129.86, 129.15, 127.46, 125.27, 69.50, 65.49, 64.58, 53.57, 27.76, 15.05; MS (ESI⁺), calcd for C₁₇H₂₅N₄O₂ (M+H): 317.1978, found: 317.1970.

### Example 40: Synthesis of 2-(ethoxymethyl)-1-(2-hydroxy-2-methylpropyl)-5-phenyl-1H-imidazole-4-carboxamide (Compound 40)

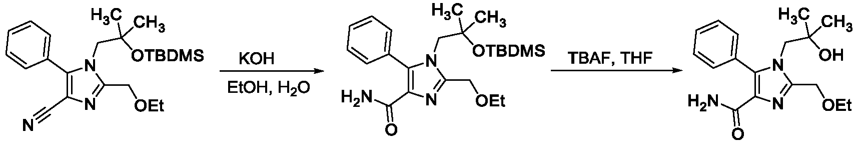

### (1) Synthesis of 1-{2-[(tert-butyldimethylsilyl)oxy]-2-methylpropyl}-2-(ethoxymethyl)-5-phenyl-1H-imidazole-4-carboxamide

1-{2-[(Tert-butyldimethylsilyl)oxy]-2-methylpropyl}-2-(ethoxymethyl)-5-phenyl-1H-imidazole-4-carbonitrile (0.100g, 0.242 mmol) was dissolved in ethanol (3 mL), a solution of potassium hydroxide (0.135g, 2.42 mmol) in water (3 mL) was added, and the resulting mixture was heated under reflux for 18 hours. Afterwards, the mixture was cooled to room temperature, hydrochloric acid (0.2 mL, 12.1M) was added, saturated aqueous sodium bicarbonate solution was then added until the mixture was neutral, and then the resultant was extracted with ethyl acetate (3 × 20 mL). The combined organic layers were washed with saturated aqueous sodium chloride solution, dried over magnesium sulfate, filtered and concentrated to dryness. The resulting solid was subjected to recrystallization with diethyl ether/hexane, so as to obtain an off-white solid (0.085g, yield: 82%). ¹H NMR (400 MHz, DMSO-*d*₆) δ 7.44-7.38 (m, 5H), 7.26 (s, 1H), 6.96 (s, 1H), 4.63 (s, 2H), 4.07 (s, 2H), 3.52 (q, *J* = 7.0 Hz, 2H), 1.15 (t, *J* = 7.0 Hz, 3H), 0.81 (s, 6H), 0.73 (s, 9H), -0.03 (s, 6H); ¹³C NMR (101 MHz DMSO-*d*₆) δ 164.09, 145.29, 135.40, 131.22, 130.95, 130.04, 128.15, 127.76, 73.65, 65.34, 64.86, 54.05, 28.26, 25.81, 17.71, 15.01, -2.20; MS (ESI⁺), calcd for C₂₃H₃₈N₃O₃Si (M+H): 432.2682, found: 432.2688.

### (2) Synthesis of 2-(ethoxymethyl)-1-(2-hydroxy-2-methylpropyl)-5-phenyl-1H-imidazole-4-carboxamide

To a solution of 1-{2-[(tert-butyldimethylsilyl)oxy]-2-methylpropyl}-2-(ethoxymethyl)-5-phenyl-1H-imidazole-4-carboxamide (0.041g, 0.095 mmol) in tetrahydrofuran (4 mL), tetrabutylammonium fluoride (95 µL, 0.095 mmol, 1M solution in tetrahydrofuran) was added, and the solution was stirred at room temperature for 18 hours. Afterwards, 10 mL of saturated aqueous ammonium chloride solution was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resulting solid was subjected to recrystallization with ethyl acetate and hexane, so as to obtain a white solid (0.019 g, yield: 63%). ¹H NMR (400 MHz, DMSO-*d*₆) δ 7.43-7.34 (m, 5H), 7.24 (d, *J* = 2.1 Hz, 1H), 6.94 (d, *J* = 2.1 Hz, 1H), 4.75 (s, 1H), 4.71 (s, 2H), 3.98 (s, 2H), 3.52 (q, *J* = 7.0 Hz, 2H), 1.15 (t, *J* = 7.0 Hz, 3H), 0.71 (s, 6H); ¹³C NMR (101 MHz DMSO-*d*₆) δ 164.15, 145.52, 135.59, 131.28, 130.77, 130.08, 128.03, 127.63, 69.61, 65.20, 64.76, 53.13, 27.77, 15.05; MS (ESI⁺), calcd for C₁₇H₂₄N₃O₃ (M+H): 318.1818, found: 318.1825.

### Example 41: Synthesis of 2-(ethoxymethyl)-1-(2-hydroxy-2-methylpropyl)-5-phenyl-1H-imidazole-4-carbaldehyde (Compound 41)

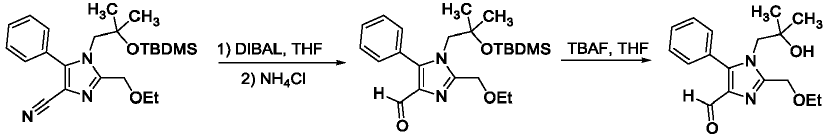

### (1) Synthesis of 1-{2-[(tert-butyldimethylsilyl)oxy]-2-methylpropyl}-2-(ethoxymethyl)-5-phenyl-1H-imidazole-4-carbaldehyde

A solution of 1-{2-[(tert-butyldimethylsilyl)oxy]-2-methylpropyl}-2-(ethoxymethyl)-5-phenyl-1H-imidazole-4-carbonitrile (1.70 g, 4.11 mmol) was cooled in a dry ice/isopropanol bath (≈ -78°C). Diisobutylaluminum hydride (8.2 mL, 8.2mmol, 1M solution in hexane) was added slowly over 10 minutes. The solution was stirred in a dry ice bath for 3.5 hours, saturated aqueous ammonium chloride solution (100 mL) was added, the mixture was allowed to stand until its temperature reached the room temperature and was extracted with ethyl acetate (3 × 75 mL). The combined organic layers were washed with saturated aqueous potassium sodium tartrate solution (150 mL), washed with saturated aqueous sodium chloride solution (150 mL), dried over magnesium sulfate, filtered and concentrated to dryness. The resultant was purified by flash SiO₂ chromatography (100 g of silica gel, gradient elution: hexane to 50% ethyl acetate/50% hexane), so as to obtain an off-white solid (1.21g, yield: 71%). ¹H NMR (400 MHz, DMSO-*d*₆) δ 9.56 (s, 1H), 7.55-7.51 (m, 5H), 4.68 (s, 2H), 4.18 (s, 2H), 3.53 (q, *J* = 7.0 Hz, 2H), 1.16 (t, *J* = 7.0 Hz, 3H), 0.85 (s, 6H), 0.72 (s, 9H), -0.02 (s, 6H); ¹³C NMR (101 MHz DMSO-*d*₆) δ 184.32, 148.29, 142.59, 136.24, 130.59, 129.36, 128.78, 127.95, 73.69, 65.43, 64.92, 54.18, 28.24, 25.76, 17.67, 14.94, -2.23; MS (ESI⁺), calcd for C₂₃H₃₇N₂O₃Si (M+H): 417.2574, found: 417.2570.

### (2) Synthesis of 2-(ethoxymethyl)-1-(2-hydroxy-2-methylpropyl)-5-phenyl-1H-imidazole-4-carbaldehyde

To a solution of 1-{2-[(tert-butyldimethylsilyl)oxy]-2-methylpropyl}-2-(ethoxymethyl)-5-phenyl-1H-imidazole-4-carbaldehyde (0.075g, 0.18 mmol) in tetrahydrofuran (6 mL), tetrabutylammonium fluoride (0.54 mL, 0.54 mmol, 1M solution in tetrahydrofuran) was added. The solution was stirred at room temperature for 16 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resulting residue was purified by flash SiO₂ chromatography (10 g of silica gel, gradient elution: dichloromethane to 7% methanol/93% dichloromethane), so as to obtain a white solid (0.083g, yield: 83%). ¹H NMR (400 MHz, DMSO-*d*₆) δ 9.53 (s, 1H), 7.57-7.47 (m, 5H), 4.80 (s, 1H), 4.76 (s, 2H), 4.09 (s, 2H), 3.53 (q, *J* = 7.0 Hz, 2H), 1.16 (t, *J* = 7.0 Hz, 3H), 0.75 (s, 6H); ¹³C NMR (101 MHz DMSO-*d*₆) δ 145.30, 138.16, 130.81, 130.61, 129.93, 128.61, 127.61, 74.00, 65.14, 65.08, 56.09, 54.24, 28.15, 25.82, 17.71, 15.01, -2.19; MS (ESI⁺), calcd for C₁₇H₂₃N₂O₃ (M+H): 303.1709, found: 303.1711.

### Example 42: Synthesis of methyl 2-(ethoxymethyl)-1-(2-hydroxy-2-methylpropyl)-5-phenyl-1H-imidazole-4-carboxylate (Compound 42)

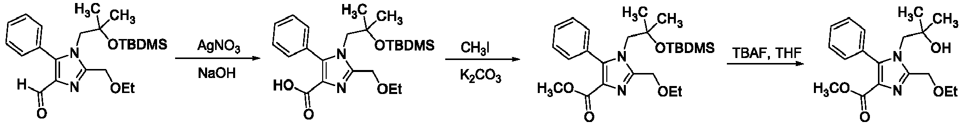

### (1) Synthesis of 1-{2-[(tert-butyldimethylsilyl)oxy]-2-methylpropyl}-2-(ethoxymethyl)-5-phenyl-1H-imidazole-4-carboxylic acid

1-{2-[(Tert-butyldimethylsilyl)oxy]-2-methylpropyl}-2-(ethoxymethyl)-5-phenyl-1H-imidazole-4-carbaldehyde (0.537g, 1.29 mmol) was dissolved in acetonitrile (5.4 mL), 10% aqueous sodium hydroxide solution (6.4 mL) was added, silver (I) nitrate (0.438 g, 2.58 mmol) was then added, and the resulting mixture was stirred at room temperature for 24 hours. Afterwards, the mixture was neutralized with sodium dihydrogen phosphate monohydrate (80 mL, 1M aqueous solution), diluted with saturated aqueous sodium chloride solution (50 mL), and the resulting mixture was extracted with ethyl acetate (4 × 50 mL). The combined organic layers were washed with saturated aqueous sodium chloride solution (100 mL), dried over magnesium sulfate, filtered and concentrated to dryness, so as to obtain a white solid (0.490g, yield: 88%). ¹H NMR (400 MHz, DMSO-*d*₆) δ 12.0 (br s, 1H), 7.47-7.38 (m, 5H), 4.64 (s, 2H), 4.06 (s, 2H), 3.50 (q, *J* = 7.0 Hz, 2H), 1.15 (t, *J* = 7.0 Hz, 3H), 0.82 (s, 6H), 0.73 (s, 9H), -0.03 (s, 6H); ¹³C NMR (101 MHz DMSO-*d*₆) δ 163.78, 146.21, 138.53, 131.04, 129.88, 128.70, 128.48, 127.95, 73.61, 65.26, 64.94, 54.17, 28.29, 25.79, 17.70, 14.98, -2.21; MS (ESI⁺), calcd for C₂₃H₃₇N₂O₄Si (M-H): 431.2366, found: 431.2375.

### (2) Synthesis of methyl 1-{2-[(tert-butyldimethylsilyl)oxy]-2-methylpropyl}-2-(ethoxymethyl)-5-phenyl-1H-imidazole-4-carboxylate

N,N-dimethylformamide (8.5 mL) was added to potassium carbonate (0.363g, 2.62 mmol), 1-{2-[(tert-butyldimethylsilyl)oxy] -2-methylpropyl}-2-(ethoxymethyl)-5-phenyl-1H-imidazole-4-carboxylic acid (0.283g, 0.654 mmol) was further added, and after the resulting mixture was stirred for 15 minutes, iodomethane (61 µL, 0.98 mmol) was added and the resultant was stirred for 17 hours. Afterwards, water (20 mL) was added, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resulting residue was purified by flash SiO₂ chromatography (10 g of silica gel, gradient elution: hexane to 35% ethyl acetate/65% hexane), so as to obtain a colorless oily liquid (0.254 g, yield: 87%). ¹H NMR (400 MHz, DMSO-*d*₆) δ 7.49-7.43 (m, 3H), 7.43-7.39 (m, 2H), 4.64 (s, 2H), 4.08 (s, 2H), 3.59 (s, 3H), 3.50 (q, *J* = 7.0 Hz, 2H), 1.14 (t, *J =* 7.0 Hz, 3H), 0.83 (s, 6H), 0.72 (s, 9H), -0.03 (s, 6H); ¹³C NMR (101 MHz DMSO-*d*₆) δ 162.84, 146.60, 139.09, 130.92, 129.49, 128.66, 128.02, 127.81, 73.60, 65.25, 64.91, 54.20, 50.74, 28.26, 25.77, 17.67, 14.94, -2.22; MS (ESI⁺), calcd for C₂₄H₃₉N₂O₄Si (M+H): 447.2679, found: 447.2677.

### (3) Synthesis of methyl 2-(ethoxymethyl)-1-(2-hydroxy-2-methylpropyl)-5-phenyl-1H - imidazole-4-carboxylate

To a solution of methyl 1-{2-[(tert-butyldimethylsilyl)oxy]-2-methylpropyl}-2-(ethoxymethyl)-5-phenyl-1H-imidazole-4-carboxylate (0.050g, 0.11 mmol) in tetrahydrofuran (3 mL), tetrabutylammonium fluoride (0.33 mL, 0.33 mmol, 1M solution in tetrahydrofuran) was added, and the solution was stirred at room temperature for 16 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution, dried over magnesium sulfate, filtered and concentrated to dryness. The resulting residue was purified by flash SiO₂ chromatography (10 g of silica gel, gradient elution: dichloromethane to 8% methanol/92% dichloromethane), so as to obtain a white solid (0.034 g, yield: 92%). ¹H NMR (400 MHz, DMSO-*d*₆) δ 7.49-7.45 (m, 2H), 7.41-7.34 (m, 3H), 4.67 (2 × s, 3H), 4.09 (s, 2H), 4.03 (s, 2H), 3.50 (q, *J* = 7.0 Hz, 2H), 3.16 (s, 3H), 1.14 (t, *J* = 7.0 Hz, 3H), 0.71 (s, 6H); ¹³C NMR (101 MHz DMSO-*d*₆) δ 145.78, 134.30, 132.45, 130.45, 130.03, 128.58, 127.79, 69.82, 66.77, 65.02, 64.87, 56.94, 53.39, 27.72, 15.06; MS (ESI⁺), calcd for C₁₈H₂₅N₂O₄ (M+H): 319.2022, found: 319.2020.

### Example 43: Synthesis of 1-[2-(ethoxymethyl)-4-(hydroxymethyl)-5-phenyl-1H-imidazole-1-yl]-2-methylpropan-2-ol (Compound 43)

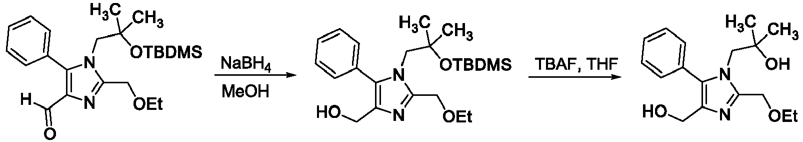

### (1) Synthesis of (1-{2-[(tert-butyldimethylsilyl)oxy]-2-methylpropyl}-2-(ethoxymethyl)-5-phenyl-1H-imidazole-4-yl)methanol

1-{2-[(Tert-butyldimethylsilyl)oxy]-2-methylpropyl}-2-(ethoxymethyl)-5-phenyl-1H-imidazole-4-carbaldehyde (0.458 g, 1.10 mmol) was dissolved in methanol (17 mL), and sodium borohydride (0.064 g, 1.7 mmol) was added slowly. The mixture was stirred at room temperature for 3 hours, saturated aqueous ammonium chloride solution (50 mL) was then added, and the mixture was extracted with dichloromethane (4 × 25 mL). The combined organic layers were washed with saturated aqueous sodium chloride solution (100 mL), dried over sodium sulfate, filtered and concentrated to dryness. The mixture was purified by flash SiO₂ chromatography (50 g of silica gel, gradient elution: dichloromethane to 7% methanol/93% dichloromethane), so as to obtain a white solid (0.413 g, yield: 90%). ¹H NMR (400 MHz, DMSO-*d*₆) δ 7.48-7.45 (m, 2H), 7.41-7.36 (m, 3H), 4.78 (t, *J* = 5.4, 1H), 4.60 (s, 2H), 4.17 (d, *J* = 5.2, 2H), 4.11 (s, 2H), 3.50 (q, *J* = 7.0 Hz, 2H), 1.14 (t, *J* = 7.0 Hz, 3H), 0.81 (s, 6H), 0.74 (s, 9H), -0.04 (s, 6H); ¹³C NMR (101 MHz DMSO-*d*₆) δ 145.30, 138.16, 130.81, 130.61, 129.93, 128.61, 127.61, 74.00, 65.14, 65.08, 56.09, 54.24, 28.15, 25.82, 17.71, 15.01, -2.19.

### (2) Synthesis of 1-[2-(ethoxymethyl)-4-(hydroxymethyl)-5-phenyl-1H-imidazole-1-yl]-2-methylpropan-2-ol

To a solution of (1-{2-[(tert-butyldimethylsilyl)oxy]-2-methylpropyl}-2-(ethoxymethyl)-5-phenyl-1H-imidazole-4-yl)methanol (0.030g, 0.072 mmol) in tetrahydrofuran (2 mL), tetrabutylammonium fluoride (0.22 mL, 0.22mmol, 1M solution in tetrahydrofuran) was added. The solution was stirred at room temperature for 16 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resultant was purified by flash SiO₂ chromatography (10 g of silica gel, gradient elution: hexane to 70% ethyl acetate/30% hexane), so as to obtain a white solid (0.018 g, yield: 82%). ¹H NMR (400 MHz, DMSO-*d*₆) δ 7.48-7.44 (m, 2H), 7.39-7.35 (m, 3H), 4.74 (t, *J* = 5.4 Hz, 1H), 4.66 (s, 2H), 4.64 (s, 1H), 4.16 (d, *J* = 5.4 Hz, 2H), 4.03 (s, 2H), 3.51 (q, *J* = 7.0 Hz, 2H), 1.14 (t, *J* = 7.0 Hz, 3H), 0.71 (s, 6H); ¹³C NMR (101 MHz DMSO-*d*₆) δ 145.53, 137.69, 130.86, 130.76, 130.09, 128.45, 127.52, 69.82, 65.01, 64.91, 56.15, 53.30, 27.72, 15.04; MS (ESI⁺), calcd for C₁₇H₂₅N₂O₃ (M+H): 305.1865, found: 305.1873.

### Example 44: Synthesis of 1-[2-(ethoxymethyl)-4-(methoxymethyl)-5-phenyl-1H-imidazole-1-yl]-2-methylpropan-2-ol (Compound 44)

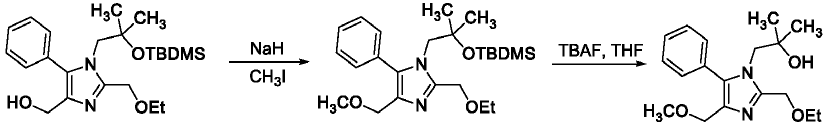

(1-{2-[(Tert-butyldimethylsilyl)oxy] -2-methylpropyl} -2-(ethoxymethyl)-5-phenyl-1H-imidazole-4-yl)methanol (0.050 g, 0.12 mmol) was dissolved in N,N-dimethylformamide (2 mL), and the resulting mixture was cooled in an ice bath (approximately 0°C). Sodium hydride (0.008 g, 0.2 mmol, 60% dispersed in mineral oil) was added, and the resulting mixture was stirred in the ice bath for 20 minutes. Afterwards, iodomethane (15 µL, 0.24 mmol) was added, the ice bath was removed, and the solution was stirred at room temperature for 19 hours. Afterwards, saturated aqueous ammonium chloride solution (10 mL) was added, and the resulting mixture was extracted with ethyl acetate (3 × 5 mL). The combined organic layers were washed with 5% aqueous lithium chloride solution (3×10 mL), washed with saturated aqueous sodium chloride solution (10 mL), dried over magnesium sulfate, filtered and concentrated to dryness. The resulting residue was filtered through a silica gel plug (≈10 g, eluted with ethyl acetate) and the filtrate was concentrated to dryness, so as to obtain crude 1-{2-[(tert-butyldimethylsilyl)oxy]-2-methylpropyl}2-(ethoxymethyl)-4-(methoxymethyl)-5-phenyl-1H-imidazole as a colorless oil (0.051 g, yield: 98%), which was used in the reaction of next step without further purification. The resulting colorless oil (0.045g, 0.10 mmol) was dissolved in tetrahydrofuran (3 mL), tetrabutylammonium fluoride (0.30 mL, 0.30 mmol, 1M solution in tetrahydrofuran) was added, and the solution was stirred at room temperature for 18 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resultant was purified by flash SiO₂ chromatography (10 g of silica gel, gradient elution: hexane to ethyl acetate to 10% methanol/90% ethyl acetate), so as to obtain a white solid (0.024 g, yield: 75%). ¹H NMR (400 MHz, DMSO-*d*₆) δ 7.48-7.41 (m, 3H), 7.39-7.35 (m, 2H), 4.78 (s, 1H), 4.72 (s, 2H), 3.98 (s, 2H), 3.57 (s, 3H), 3.50 (q, *J* = 7.0 Hz, 2H), 1.14 (t, *J* = 7.0 Hz, 3H), 0.72 (s, 6H); ¹³C NMR (101 MHz DMSO-*d*₆) δ 130.88, 129.89, 128.75, 127.60, 73.89, 65.20, 54.25, 28.24, 25.82, 17.71, 15.03, -2.19; MS (ESI⁺), calcd for C₁₈H₂₇N₂O₃ (M+H): 319.2022, found: 319.2020.

### Example 45: Synthesis of 1-[2-(ethoxymethyl)-4-methyl-5-phenyl-1H-imidazole-1-yl]-2-methylpropan-2-ol (Compound 45)

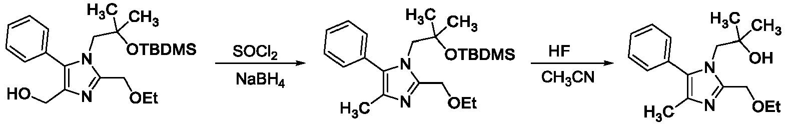

### (1) Synthesis of 1-{2-[(tert-butyldimethylsilyl)oxy]-2-methylpropyl}-2-(ethoxymethyl)-4-methyl-5-phenyl-1H-imidazole

(1-{2-[(Tert-butyldimethylsilyl)oxy]-2-methylpropyl}-2-(ethoxymethyl)-5-phenyl-1H-imidazole-4-yl)methanol (0.050g, 0.12 mmol) was dissolved in 1,2-dichloroethane (2 mL), and the mixture was cooled in an ice bath (≈0°C). Thionyl chloride (18 µL, 0.24 mmol) was added, and the solution was stirred in the ice bath for an additional 10 minutes. Afterwards, the solution was warmed to room temperature and then heated at 60°C for 2.5 hours. Thereafter, the solution was cooled to room temperature and diluted with dichloromethane (10 mL). The resulting organic solution was neutralized with saturated aqueous sodium bicarbonate solution (10 mL), washed with saturated aqueous sodium chloride solution (30 mL), dried over sodium sulfate, filtered and concentrated to dryness. The resulting residue was further dried via a high vacuum pump for 1 hour. The resulting residue was dissolved in dimethyl sulfoxide (2 mL), sodium borohydride (0.042 g, 1.1 mmol) was added, and the side wall of the reaction flask was immediately rinsed with additional dimethyl sulfoxide (1 mL). The mixture was stirred at room temperature for 24 hours. Afterwards, water (10 mL) was added, and the resulting mixture was extracted with diethyl ether (3 × 10 mL). The combined organic layers were washed with saturated aqueous sodium chloride solution (30 mL), dried over magnesium sulfate, filtered and concentrated to dryness. The resulting residue was purified by flash SiO₂ chromatography (10 g of silica gel, gradient elution: hexane to 14% ethyl acetate/86% hexane), so as to obtain a white solid (0.033 g, yield: 75%). ¹H NMR (400 MHz, DMSO-*d*₆) δ 7.55-7.51 (m, 2H), 7.49-7.42 (m, 3H), 4.93 (s, 2H), 4.18 (s, 2H), 3.55 (q, *J* = 7.0 Hz), 2.13 (s, 3H), 1.16 (t, *J*= 7.0 Hz, 3H), 0.86 (s, 6H), 0.71 (s, 9H), -0.04 (s, 6H); ¹³C NMR (101 MHz DMSO-*d*₆) δ 144.11, 131.26, 130.43, 129.21, 129.03, 128.77, 128.57, 73.36, 65.75, 60.67, 54.59, 28.26, 25.73, 17.64, 14.96, 10.92, -2.29; MS (ESI⁺), calcd for C₂₃H₃₉N₂O₂Si (M+H): 403.2781, found: 403.2779.

### (2) Synthesis of 1-[2-(ethoxymethyl)-4-methyl-5-phenyl-1H-imidazole-1-yl]-2-methylpropan-2-ol

To a solution of 1-{2-[(tert-butyldimethylsilyl)oxy]-2-methylpropyl}-2-(ethoxymethyl) -4-methyl-5-phenyl-1H-imidazole (0.025 g, 0.062 mmol) in acetonitrile (1.3 mL, in an HDPE scintillation vial), hydrofluoric acid (1.3 mL, 48wt% aqueous solution) was added. The solution was stirred at room temperature for 16 hours. Afterwards, saturated aqueous sodium carbonate solution was added until pH≈10, and the resulting mixture was extracted with ethyl acetate (3 × 20 mL). The combined organic layers were washed with saturated aqueous sodium chloride solution (40 mL), dried over magnesium sulfate, filtered and concentrated to dryness. The resulting residue was purified by flash SiO₂ chromatography (10 g of silica gel, gradient elution: dichloromethane to 7% methanol/93% dichloromethane), so as to obtain a white solid (0.016g, yield: 89%). ¹H NMR (400 MHz, DMSO-*d*₆) δ 7.47-7.44 (m, 2H), 7.37-7.33 (m, 1H), 7.30-7.28 (m, 2H), 4.62-4.62 (m, 3H), 3.98 (s, 2H), 3.49 (q, *J* = 7.0 Hz, 2H), 2.03 (s, 3H), 1.13 (t, *J* = 7.0 Hz, 3H), 0.70 (s, 6H); ¹³C NMR (101 MHz DMSO-*d*₆) δ 145.20, 132.93, 131.36, 130.05, 129.13, 128.63, 127.30, 69.83, 64.97, 64.81, 53.38, 27.76, 15.08, 13.13; MS (ESI⁺), calcd for C₁₇H₂₅N₂O₂ (M+H): 289.1916, found: 289.1905.

### Example 46: Synthesis of 1-[2-(ethoxymethyl)-4-(fluoromethyl)-5-phenyl-1H-imidazole-1-yl]-2-methylpropan-2-ol (Compound 46)

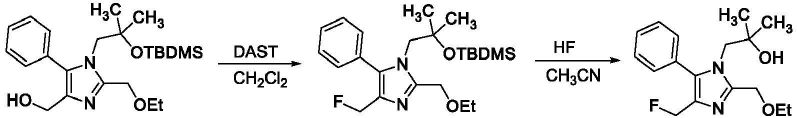

### (1) Synthesis of 1-{2-[(tert-butyldimethylsilyl)oxy]-2-methylpropyl}-2-(ethoxymethyl)-4-(fluoromethyl)-5-phenyl-1H-imidazole

(1-{2-[(Tert-butyldimethylsilyl)oxy] -2-methylpropyl} -2-(ethoxymethyl)-5 -phenyl-1H-imidazole-4-yl)methanol (0.113g, 0.270 mmol) was dissolved in dichloromethane (6 mL), the mixture was cooled in an ice bath (approximately 0°C), (diethylamino)sulfur trifluoride (0.35 mL, 0.35 mmol, 1M solution in dichloromethane) was added, and the solution was stirred in an ice bath for 1 hours. Afterwards, saturated aqueous sodium bicarbonate solution (10 mL) was added, and the resulting mixture was extracted with dichloromethane (3 × 10 mL). The combined organic layers were washed with saturated aqueous sodium chloride solution (30 mL), dried over sodium sulfate, filtered and concentrated to dryness. The resultant was purified by flash SiO₂ chromatography (10 g of silica gel, gradient elution: hexane to 30% ethyl acetate /70% hexane), so as to obtain a white solid (0.073 g, yield: 64%). ¹H NMR (400 MHz, DMSO-*d*₆) δ 7.54-7.50 (m, 2H), 7.47-7.40 (m, 3H), 5.08 (d, *J* = 50.0 Hz, 2H), 4.62 (s, 2H), 4.15 (s, 2H), 3.50 (q, *J* = 7.0 Hz, 2H), 1.15 (t, *J* = 7.0 Hz, 3H), 0.84 (s, 6H), 0.72 (s, 9H), -0.05 (s, 6H); ¹³C NMR (101 MHz DMSO-*d*₆) δ 146.40, 134.22, 134.16, 132.88, 132.70, 129.75, 129.73, 129.70, 128.93, 128.33, 78.38, 76.80, 73.76, 65.23, 64.97, 54.36, 28.21, 25.76, 17.67, 14.97, -2.25; MS (ESI⁺), calcd for C₂₃H₃₈FN₂O₂Si (M+H): 421.2686, found: 421.2696.

### (2) Synthesis of 1-[2-(ethoxymethyl)-4-(fluoromethyl)-5-phenyl-1H-imidazole-1-yl]-2-methylpropan-2-ol

1-{2-[(Tert-butyldimethylsilyl)oxy]-2-methylpropyl}-2-(ethoxymethyl)-4-(fluoromethyl) -5-phenyl-1H-imidazole (0.040 g, 0.095 mmol) was dissolved in acetonitrile (2 mL, in an HDPE scintillation vial), hydrofluoric acid (2 mL, 48wt% aqueous solution) was then added, and the solution was stirred at room temperature for 17 hours. Afterwards, saturated aqueous sodium carbonate solution was added until pH≈10, and the resulting mixture was extracted with ethyl acetate (3 × 30 mL). The combined organic layers were washed with saturated aqueous sodium chloride solution (50 mL), dried over magnesium sulfate, filtered and concentrated to dryness. The resulting residue was purified by flash SiO₂ chromatography (10 g of silica gel, gradient elution: dichloromethane to 6% methanol/94% dichloromethane), so as to obtain a colorless oil (0.019 g, yield: 66%). ¹H NMR (400 MHz, DMSO-*d*₆) δ 7.53-7.49 (m, 2H), 7.46-7.42 (m, 1H), 7.39-7.37 (m, 2H), 5.07 (d, *J* = 50.4 Hz, 2H), 4.73 (s, 1H), 4.69 (s, 2H), 4.06 (s, 2H), 3.51 (q, *J* = 7.0 Hz, 2H), 1.14 (t, *J* = 7.0 Hz, 3H), 0.72 (s, 6H); ¹³C NMR (101 MHz DMSO-*d*₆) δ 146.59, 134.55, 134.48, 132.52, 132.33, 130.06, 130.04, 129.67, 129.64, 128.82, 128.33, 78.49, 76.91, 69.79, 65.18, 64.84, 53.50, 27.73, 15.07; MS (ESI⁺), calcd for C₁₇H₂₄FN₂O₂ (M+H): 307.1822, found: 307.1819.

### Example 47: Synthesis of 1-[2-(ethoxymethyl)-4-(2-hydroxypropan-2-yl)-5-phenyl-1H-imidazole-1-yl]-2-methylpropan-2-ol (Compound 47)

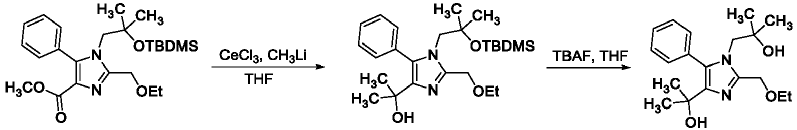

### (1) Synthesis of 2-(1-{2-[(tert-butyldimethylsilyl)oxy]-2-methylpropyl}-2-(ethoxymethyl)-5-phenyl-1H-imidazole-4-yl) propan-2-ol

In a flask that had been dried in an oven, cerium (III) chloride (0.054 g, 0.22 mmol) was dissolved in tetrahydrofuran (2 mL) and the resultant was stirred at room temperature for 2.5 hours, and the mixture was then cooled in a dry ice/isopropanol bath (approximately -78°C). Methyllithium (0.24 mL, 0.39 mmol, 1.6M solution in diethyl ether) was stirred in a dry ice bath for 30 minutes in advance. In a second flask that had been dried in an oven, 1-{2-[(tert-butyldimethylsilyl)oxy]-2-methylpropyl}-2-(ethoxymethyl)-5-phenyl-1H-imidazole -4-carboxylate (0.051 g, 0.11 mmol) was dissolved in tetrahydrofuran (1 mL), and the resultant was added to the first cooled flask and stirred in a dry ice bath for 30 minutes. The mixture was removed from the dry ice bath and stirred at room temperature for 17 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resultant was purified by flash SiO₂ chromatography (10 g of silica gel, gradient elution: dichloromethane to 80% ethyl acetate/20% dichloromethane), so as to obtain a white solid (0.037 g, yield: 76%). ¹H NMR (400 MHz, DMSO-*d*₆) δ 7.43-7.33 (m, 5H), 4.57 (s, 2H), 4.40 (s, 1H), 3.92 (s, 2H), 3.50 (q, *J* = 7.0 Hz, 2H), 1.27 (s, 6H), 1.14 (t, *J*= 7.0 Hz, 3H), 0.82 (s, 6H), 0.74 (s, 9H), -0.04 (s, 6H); ¹³C NMR (101 MHz DMSO-*d*₆) δ 144.20, 143.26, 132.16, 131.97, 127.73, 127.65, 127.39, 73.66, 69.17, 65.22, 65.16, 53.88, 31.32, 28.46, 25.83, 17.73, 15.05, -2.17; MS (ESI⁺), calcd for C₂₅H₄₃N₂O₃Si (M+H): 447.3043, found: 447.3035.

### (2) Synthesis of 1-[2-(ethoxymethyl)-4-(2-hydroxypropan-2-yl)-5-phenyl-1H-imidazole-1-yl]-2-methylpropan-2-ol

2-(1-{2-[(Tert-butyldimethylsilyl)oxy]-2-methylpropyl}-2-(ethoxymethyl)-5-phenyl-1H-imidazole-4-yl) propan-2-ol (0.030g, 0.067 mmol) was dissolved in tetrahydrofuran (2 mL), tetrabutylammonium fluoride (0.20 mL, 0.20 mmol, 1M solution in tetrahydrofuran) was added, and the solution was stirred at room temperature for 22 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resulting residue was purified by flash SiO₂ chromatography (10 g of silica gel, gradient elution: dichloromethane to 5% methanol/95% dichloromethane), so as to obtain a colorless oil (0.019 g, yield: 86%). ¹H NMR (400 MHz, DMSO-*d*₆) δ 7.47-7.30 (m, 5H), 4.69 (s, 1H), 4.66 (s, 2H), 4.50 (s, 2H), 3.83 (s, 2H), 3.51 (q, *J* = 7.0 Hz, 2H), 1.25 (s, 6H), 1.14 (t, *J* = 7.0 Hz, 3H), 0.74 (s, 6H); ¹³C NMR (101 MHz DMSO-*d*₆) δ 143.40, 143.27, 132.18, 131.87, 127.86, 127.82, 127.68, 69.59, 68.90, 65.17, 64.86, 53.05, 31.16, 27.94, 15.09; MS (ESI⁺), calcd for C₁₉H₂₉N₂O₃ (M+H): 333.2178, found: 333.2191.

### Example 48: Synthesis of 1-[2-(ethoxymethyl)-5-phenyl-4-(prop-2-yl)-1H-imidazole-1-yl]-2-methylpropan-2-ol (Compound 48)

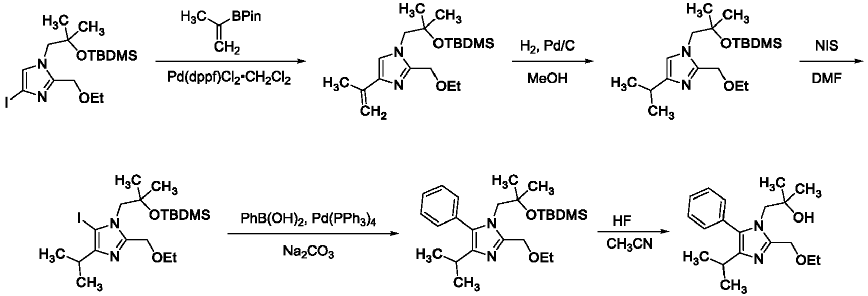

### (1) Synthesis of 1-{2-[(tert-butyldimethylsilyl)oxy]-2-methylpropyl}-2-(ethoxymethyl)-4-(prop-1-ene-2-yl)-1H-imidazole

A mixture of 1-{2-[(tert-butyldimethylsilyl)oxy]-2-methylpropyl}-2-(ethoxymethyl)-4-iodo-1H-imidazole (0.300g, 0.684 mmol) and aqueous sodium carbonate solution (2M, 2.5 mL) was dissolved in N,N-dimethylformamide (5 mL), and the resultant was degassed and backfilled with nitrogen gas (4×).(1,1'-Bis(diphenylphosphino)ferrocene)palladium (II) dichloride · dichloromethane (0.028g, 0.034 mmol) was added to the above system, and the resulting mixture was degassed and backfilled with nitrogen gas (4×) again. Isopropenyl pinacol boronate (0.14 mL, 0.75 mmol) was added, and the resulting mixture was heated at 65°C for 17 hours. Afterwards, the mixture was cooled to room temperature, diluted with water (30 mL), and extracted with ethyl acetate (3 × 20 mL). The combined organic layers were washed with 5% aqueous lithium chloride solution (2 × 40 mL), washed with saturated aqueous sodium chloride solution (50 mL), dried over magnesium sulfate, filtered and concentrated to dryness. The resultant was purified by flash SiO₂ chromatography (25 g of silica gel, gradient elution: hexane to 30%~70% ethyl acetate), so as to obtain a yellow oil (0.193 g, yield: 80%). ¹H NMR (400 MHz, DMSO-*d*₆) δ 7.14 (s, 1H), 5.48 (dd, *J* = 2.8, 0.57 Hz, 1H), 4.77 (dd, *J* = 2.8, 1.5 Hz, 1H), 4.45 (s, 2H), 3.89 (s, 2H), 3.44 (q, *J* = 7.0 Hz, 2H), 1.93 (s, 3H), 1.19 (s, 6H), 1.09 (t, *J* = 7.0 Hz, 3H), 0.86 (s, 9H), 0.06 (s, 6H); ¹³C NMR (101 MHz DMSO-*d*₆) δ 144.92, 139.48, 136.06, 118.69, 108.09, 73.63, 64.89, 63.78, 56.65, 27.01, 25.77, 19.95, 17.74, 14.95, -2.22.

### (2) Synthesis of 1-{2-[(tert-butyldimethylsilyl)oxy]-2-methylpropyl}-2-(ethoxymethyl)-4-(prop-2-yl)-1H-imidazole

1-{2-[(Tert-butyldimethylsilyl)oxy]-2-methylpropyl}-2-(ethoxymethyl)-4-(prop-1-ene-2-yl)-1H-imidazole (0.190g, 0.539 mmol) was dissolved in methanol (9 mL), and palladium on carbon (0.010g, content: 5%) was added. The mixture was degassed and backfilled with nitrogen gas (4×), and then degassed and backfilled with hydrogen gas (4×). Thereafter, the reaction mixture was stirred under a hydrogen atmosphere (hydrogen balloon) for 17 hours. Afterwards, the mixture was degassed and backfilled with nitrogen gas (4×), diluted with dichloromethane (20 mL), filtered through celite, and rinsed with dichloromethane. The filtrate was concentrated to dryness. The resulting residue was purified by flash SiO₂ chromatography (10 g of silica gel, gradient elution: hexane to 40%-50% ethyl acetate/hexane), so as to obtain a colorless oil (0.180 g, yield: 94%). ¹H NMR (400 MHz, DMSO-*d*₆) δ 6.81 (d, *J* = 0.7 Hz, 1H), 4.40 (s, 2H), 3.83 (s, 2H), 3.42 (q, *J* = 7.0 Hz, 2H), 2.70 (dsep, *J* = 6.9, 0.7 Hz, 1H), 1.17 (s, 6H), 1.13 (d, *J* = 6.9 Hz, 6H), 1.08 (t, *J* = 7.0 Hz, 3H), 0.85 (s, 9H), 0.05 (s, 6H); ¹³C NMR (101 MHz DMSO-*d*₆) δ 145.53, 143.67, 115.95, 73.63, 64.79, 63.87, 56.52, 27.22, 27.09, 25.77, 22.38, 17.72, 14.95, -2.24.

### (3) Synthesis of 1-{2-[(tert-butyldimethylsilyl)oxy]-2-methylpropyl}-2-(ethoxymethyl)-5-iodo-4-(prop-2-yl)-1H-imidazole

1-{2-[(Tert-butyldimethylsilyl)oxy]-2-methylpropyl}-2-(ethoxymethyl)-4-(prop-2-yl)-1H -imidazole (0.175 g, 0.493 mmol) was dissolved in N,N-dimethylformamide (5 mL), N-Iodosuccinimide (0.166 g, 0.740 mmol) was added, and the resulting solution was heated at 80°C for 17 hours. Afterwards, the solution was cooled to room temperature, diluted with water (30 mL), and sodium thiosulfate pentahydrate (solid) was added until no change in color was observed. The resulting mixture was extracted with ethyl acetate (3 × 20 mL). The combined organic layers were washed with 5% aqueous lithium chloride solution (3 × 40 mL), washed with saturated aqueous sodium chloride solution (50 mL), dried over magnesium sulfate, filtered and concentrated to dryness. The resultant was purified by flash SiO₂ chromatography (10 g of silica gel, gradient elution: hexane to 20% ethyl acetate/80% hexane), so as to obtain a colorless oil (0.130 g, yield: 55%). ¹H NMR (400 MHz, DMSO-*d*₆) δ 4.57 (s, 2H), 3.97 (s, 2H), 3.43 (q, *J* = 7.0 Hz, 2H), 2.81 (sep, *J* = 6.9 Hz, 1H), 1.30 (s, 6H), 1.12 (d, *J* = 6.9 Hz, 6H), 1.10 (t, *J* = 7.0 Hz, 3H), 0.74 (s, 9H), 0.05 (s, 6H); ¹³C NMR (101 MHz DMSO-*d*₆) δ 149.15, 147.72, 74.05, 73.65, 65.31, 65.07, 56.16, 29.05, 27.45, 25.92, 22.27, 17.77, 14.96, -2.05; MS (ESI⁺), calcd for C₁₉H₃₈IN₂O₂Si (M+H): 481.1747, found: 481.1745.

### (4) Synthesis of 1-{2-[(tert-butyldimethylsilyl)oxy]-2-methylpropyl}-2-(ethoxymethyl)-5-phenyl-4-(prop-2-yl)-1H-imidazole

1-{2-[(Tert-butyldimethylsilyl)oxy]-2-methylpropyl}-2-(ethoxymethyl)-5-iodo-4-(prop-2 -yl)-1H-imidazole (0.120 g, 0.250 mmol) and phenylboronic acid (0.034 g, 0.28 mmol) were dissolved in 1,4-dioxane (3.4 mL), aqueous sodium carbonate solution (1.7 mL, 1M) was added, and the reaction solution was degassed and backfilled with nitrogen gas (4×). Tetrakis(triphenylphosphine)palladium(0) (0.015g, 0.013 mmol) was added, the mixture was degassed and backfilled with nitrogen gas (4×) again, and the mixture was heated at 80°C for 18 hours. Afterwards, the mixture was cooled to room temperature, diluted with water (10 mL), and the resulting mixture was extracted with ethyl acetate (3 × 5 mL). The combined organic layers were washed with saturated aqueous sodium chloride solution (10 mL), dried over magnesium sulfate, filtered and concentrated to dryness. The resultant was purified by flash SiO₂ chromatography (10 g of silica gel, gradient elution: hexane to 25% ethyl acetate/75% hexane), so as to obtain a colorless oil (0.080 g, yield: 74%). ¹H NMR (400 MHz, DMSO-*d*₆) δ 7.49-7.45 (m, 2H), 7.38-7.34 (m, 1H), 4.56 (s, 2H), 4.03 (s, 2H), 3.50 (q, *J* = 7.0 Hz, 2H), 2.76 (sep, *J* = 6.8 Hz, 1H), 1.15 (t, *J* = 7.0 Hz, 3H), 1.11 (d, *J* = 6.2 Hz, 6H), 0.82 (s, 6H), 0.70 (s, 9H), -0.06 (s, 6H); ¹³C NMR (101 MHz DMSO-*d*₆) δ 145.31, 143.19, 131.37, 129.92, 128.74, 127.35, 127.03, 73.62, 65.28, 65.20, 54.05, 28.43, 25.78, 25.43, 23.17, 17.67, 15.01, -2.26; MS (ESI⁺), calcd for C₂₅H₄₃N₂O₂Si (M+H): 431.3094, found: 431.3090.

### (5) Synthesis of 1-[2-(ethoxymethyl)-5-phenyl-4-(prop-2-yl)-1H-imidazole-1-yl]-2-methylpropan-2-ol

1-{2-[(Tert-butyldimethylsilyl)oxy]-2-methylpropyl}-2-(ethoxymethyl)-5-phenyl-4-(prop -2-yl)-1H-imidazole (0.075g, 0.17 mmol) was dissolved in acetonitrile (3.5 mL, in an HDPE scintillation vial), hydrofluoric acid (3.5 mL, 48wt% aqueous solution) was added, and the solution was stirred at room temperature for 17 hours. Afterwards, saturated aqueous sodium carbonate solution was added until pH≈10, and the resulting mixture was extracted with ethyl acetate (3 × 30 mL). The combined organic layers were washed with saturated aqueous sodium chloride solution (30 mL), dried over magnesium sulfate, filtered and concentrated to dryness. The resultant was purified by flash SiO₂ chromatography (10 g of silica gel, gradient elution: dichloromethane to 7% methanol/93% dichloromethane), so as to obtain a white solid (0.052 g, yield: 96%). ¹H NMR (400 MHz, DMSO-*d*₆) δ 7.47-7.44 (m, 2H), 7.38-7.34 (m, 1H), 7.28-7.25 (m, 2H), 4.64 (s, 3H), 3.95 (s, 2H), 3.51 (q, *J* = 7.0 Hz, 2H), 2.72 (sep, *J* = 6.8 Hz, 1H), 1.14 (t, *J* = 7.0 Hz, 3H), 1.10 (d, *J* = 6.8 Hz, 6H), 0.70 (s, 6H); ¹³C NMR (101 MHz DMSO-*d*₆) δ 145.08, 142.72, 131.29, 130.39, 128.59, 127.64, 127.45, 69.82, 65.11, 65.04, 53.19, 27.81, 25.54, 23.22, 15.10; MS (ESI⁺), calcd for C₁₉H₂₉N₂O₂ (M+H): 317.2229, found: 317.2223.

### Example 49: Synthesis of 1-[2-(ethoxymethyl)-4,5-diphenyl-1H-imidazole-1-yl]-2-methylpropan-2-ol (Compound 49)

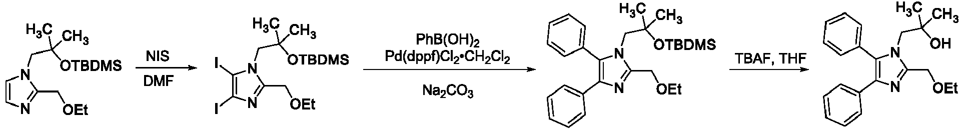

### (1) Synthesis of 1-{2-[(tert-butyldimethylsilyl)oxy]-2-methylpropyl}-2-(ethoxymethyl)-4,5-diiodo-1H-imidazole

1-{2-[(Tert-butyldimethylsilyl)oxy]-2-methylpropyl}-2-(ethoxymethyl)-1H-imidazole (3.00 g, 9.60 mmol) was dissolved in N,N-dimethylformamide (60 mL) and heated to 80°C. N-Iodosuccinimide (4.32 g, 19.20 mmol) was dissolved in N,N-dimethylformamide (30 mL), the resultant was slowly added to the original reaction flask over 5 minutes, and the reaction mixture was stirred at 80°C for 16 hours. Afterwards, the reaction system was cooled to room temperature and 100 mL of water was added, sodium thiosulfate pentahydrate (solid) was added until no further change in color was observed, and a pale yellow suspension was obtained. The resulting mixture was extracted with ethyl acetate (3 × 70 mL). Thereafter, the combined organic layers were washed with 5% aqueous lithium chloride solution (3 × 150 mL), washed with 200 mL of saturated aqueous sodium chloride solution, dried over magnesium chloride, filtered and concentrated to dryness. The resultant was purified by flash SiO₂ chromatography to obtain 1-{2-[(tert-butyldimethylsilyl)oxy]-2-methylpropyl}-2-(ethoxymethyl)-4,5-diiodo-1H-imidazole as a white solid (0.434 g, yield: 8%), ¹H NMR (400 MHz, DMSO-*d*₆) δ 4.58 (s, 2H), 4.09 (s, 2H), 3.40 (q, *J* = 7.0 Hz, 2H), 1.29 (s, 6H), 1.08 (t, *J* = 7.0 Hz, 3H), 0.75 (s, 9H), 0.05 (s, 6H); ¹³C NMR (101 MHz DMSO-*d*₆) δ 150.27, 96.31, 89.26, 74.14, 65.14, 64.68, 58.01, 28.83, 25.92, 17.80, 14.92, -2.02; MS (ESI⁺), calcd for C₁₆H₃₁I₂N₂O₂Si (M+H): 565.0244, found: 565.0242.

### (2) Synthesis of 1-{2-[(tert-butyldimethylsilyl)oxy]-2-methylpropyl}-2-(ethoxymethyl)-4,5-diphenyl-1H-imidazole

1-{2-[(Tert-butyldimethylsilyl)oxy]-2-methylpropyl}-2-(ethoxymethyl)-4,5-diiodo-1H-imidazole (0.100g, 0.18 mmol) and phenylboronic acid (0.065g, 0.53 mmol) were dissolved in 1,4-dioxane (4 mL), aqueous sodium carbonate solution (0.55 mL, 1.1 mmol, 2M) was added, and the resulting mixture was degassed and backfilled with nitrogen gas (4×). (1,1'-Bis(diphenylphosphino)ferrocene)palladium (II) dichloride · dichloromethane (0.015 g, 0.018 mmol) was added, and the resulting mixture was degassed and backfilled with nitrogen gas (4×) again. Thereafter, the reaction mixture was heated at 100°C for 17 hours. Afterwards, the reaction mixture was diluted with water (10 mL) and extracted with ethyl acetate (3 × 10 mL). The combined organic layers were washed with saturated aqueous sodium chloride solution (20 mL), dried over magnesium sulfate, filtered and concentrated to dryness. The resulting residue was purified by flash SiO₂ chromatography (10 g of silica gel, gradient elution: hexane to 30%-70% ethyl acetate/hexane), so as to obtain a yellow oil (0.045 g, yield: 55%). ¹H NMR (400 MHz, DMSO-*d*₆) δ 7.50-7.41 (m, 3H), 7.36-7.33 (m, 2H), 7.31-7.29 (m, 2H), 7.20-7.16 (m, 2H), 7.13-7.09 (m, 1H), 4.67 (s, 2H), 4.06 (s, 2H), 3.52 (q, *J* = 7.0 Hz, 2H), 1.15 (t, *J* = 7.0 Hz, 3H), 0.86 (s, 6H), 0.75 (s, 9H), -0.02 (s, 6H); ¹³C NMR (101 MHz DMSO-*d*₆) δ 146.07, 136.00, 134.88, 131.19, 131.06, 129.40, 129.06, 128.45, 127.99, 126.53, 126.07, 73.64, 65.20, 65.09, 54.11, 28.48, 25.83, 17.76, 15.05, -2.16.

### (3) Synthesis of 1-[2-(ethoxymethyl)-4,5-diphenyl-1H-imidazole-1-yl]-2-methylpropan-2-ol

To a solution of 1-{2-[(tert-butyldimethylsilyl)oxy]-2-methylpropyl}-2-(ethoxymethyl)-4,5-diphenyl-1H-imidazole (0.035 g, 0.075 mmol) in tetrahydrofuran (2 mL), tetrabutylammonium fluoride (0.23 mL, 0.23 mmol, 1M solution in tetrahydrofuran) was added. The solution was stirred at room temperature for 17 hours. Afterwards, saturated aqueous ammonium chloride solution (5 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resulting residue was purified by flash SiO₂ chromatography (10 g of silica gel, gradient elution: hexane to ethyl acetate), so as to obtain an off-white solid (0.022 g, yield: 85%). ¹H NMR (400 MHz, DMSO-*d*₆) δ 7.50-7.42 (m, 3H), 7.34-7.29 (m, 4H), 7.19-7.15 (m, 2H), 7.12-7.08 (m, 1H), 4.76 (s, 1H), 4.75 (s, 2H), 3.07 (s, 2H), 3.53 (q, *J* = 7.0 Hz, 2H), 1.15 (t, *J* = 7.0 Hz, 3H), 0.76 (s, 3H); MS (ESI⁺), calcd for C₂₂H₂₇N₂O₂ (M+H): 351.2072, found: 315.2076.

### Example 50: Synthesis of 1-[2-(ethoxymethyl)-5-phenyl-1H-imidazole-1-yl]-2-methylpropan-2-ol (Compound 50)

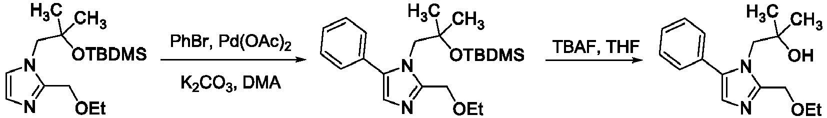

### (1) Synthesis of 1-{2-[(tert-butyldimethylsilyl)oxy]-2-methylpropyl}-2-(ethoxymethyl)-5-phenyl-1H-imidazole

A mixture of 1-{2-[(tert-butyldimethylsilyl)oxy]-2-methylpropyl}-2-(ethoxymethyl)-1H-imidazole (7.00 g, 22.4 mmol), bromobenzene (3.9 mL, 37 mmol) and potassium carbonate (10.2 g, 74.0 mmol) was dissolved in N,N-dimethylacetamide (180 mL), and the resultant was degassed and backfilled with nitrogen gas (4×).Palladium (II) acetate (0.831 g, 3.70 mmol) was added, and the mixture was degassed and backfilled with nitrogen gas (4×) again. The mixture was heated at 150°C for 21 hours, and then the reaction system was cooled to room temperature. Water (200 mL) was added, and the resulting mixture was extracted with ethyl acetate (3 × 200 mL). The combined organic layers were washed with 5% aqueous lithium chloride solution (3 × 400 mL), washed with saturated aqueous sodium chloride solution (400 mL), dried over magnesium sulfate, filtered and concentrated to dryness. The resulting residue was purified by flash SiO₂ chromatography (340 g of silica gel, gradient elution: dichloromethane to 4% methanol/96% dichloromethane), so as to obtain a red oil (2.88 g, yield: 20%). ¹H NMR (400 MHz, DMSO-*d*₆) δ 7.47-7.40 (m, 4H), 7.38-7.33 (m, 1H), 6.91 (s, 1H), 4.60 (s, 2H), 4.20 (s, 2H), 3.49 (q, *J* = 7.0 Hz, 2H), 1.14 (t, *J* = 7.0 Hz, 3H), 0.86 (s, 6H), 0.72 (s, 9H), -0.05 (s, 6H); ¹³C NMR (101 MHz DMSO-*d*₆) δ 147.32, 133.86, 131.35, 128.88, 128.30, 127.49, 126.83, 73.90, 65.16, 65.04, 54.23, 28.21, 25.75, 17.66, 14.97, -2.26; MS (ESI⁺), calcd for C₂₂H₃₇N₂O₂Si (M+H): 389.2624, found: 389.2622.

(2) Synthesis of 1-[2-(ethoxymethyl)-5-phenyl-1H-imidazole-1-yl]-2-methylpropan-2-ol 1-{2-[(Tert-butyldimethylsilyl)oxy]-2-methylpropyl}tetrahydrofuran-2-(ethoxymethyl)-5 -phenyl-1H-imidazole (0.050 g, 0.13 mmol) was dissolved in tetrahydrofuran (3 mL), and tetrabutylammonium fluoride (0.39 mL, 0.39mmol, 1M solution in tetrahydrofuran) was added. The solution was stirred at room temperature for 18 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution, dried over magnesium sulfate, filtered and concentrated to dryness. The resulting residue was purified by flash SiO₂ chromatography (10 g of silica gel, gradient elution: dichloromethane to 10% methanol/90% dichloromethane), so as to obtain a yellow oil (0.032 g, yield: 89%). ¹H NMR (400 MHz, DMSO-*d*₆) δ 7.46-7.33 (m, 5H), 6.88 (s, 1H), 4.67 (s, 2H), 4.65 (s, 1H), 4.12 (s, 2H), 3.50 (q, *J* = 7.0 Hz, 2 H), 1.14 (t, *J* = 7.0 Hz, 3H), 0.73 (s, 6H); ¹³C NMR (101 MHz DMSO-*d*₆) δ 147.31, 134.13, 131.29, 128.75, 128.68, 127.47, 126.49, 69.92, 64.96, 53.31, 27.72, 15.04; MS (ESI⁺), calcd for C₁₆H₂₂N₂O₂ (M+H): 275.2, found: 275.2.

### Example 51: Synthesis of 1-[4-chloro-2-(ethoxymethyl)-5-phenyl-1H-imidazole-1-yl]-2-methylpropan-2-ol (Compound 51)

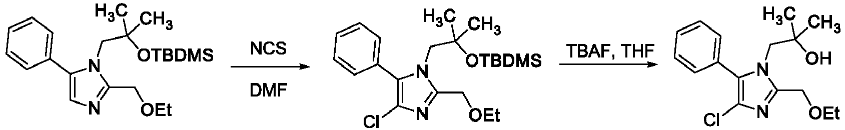

1-{2-[(Tert-butyldimethylsilyl)oxy]-2-methylpropyl}-2-(ethoxymethyl)-5-phenyl-1H-imidazole (0.050g, 0.13 mmol) was dissolved in N,N-dimethylformamide (2 mL), and the resultant was heated at 60°C. A solution of N-chlorosuccinimide (0.027 g, 0.20 mmol) in N,N-dimethylformamide (1 mL) was further added, and the resulting solution was stirred at 60°C for 15 hours. Afterwards, the solution was cooled to room temperature and water (20 mL) was added. Sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 15 mL), washed with saturated aqueous sodium chloride solution (30 mL), dried over magnesium sulfate, filtered and concentrated to dryness. The resulting residue was filtered through a silica gel plug (≈10 g, eluted with ethyl acetate), and the filtrate was concentrated to dryness, so as to obtain crude 1-{2-[(tert-butyldimethylsilyl)oxy] -2-methylpropyl}-4-chloro-2-(ethoxymethyl)-5-phenyl-1H-imidazole (0.055 g, >99%). The residue was dissolved in tetrahydrofuran (5 mL), and tetrabutylammonium fluoride (0.39 mL, 0.39 mmol, 1M solution in tetrahydrofuran) was added. The solution was stirred at room temperature for 16 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution, dried over magnesium sulfate, filtered and concentrated to dryness. The resulting residue was purified by flash SiO₂ chromatography (10 g of silica gel, gradient elution: hexane to 60% ethyl acetate/40% hexane) to obtain a white solid (0.037 g, yield: 93%). ¹H NMR (400 MHz, DMSO-*d*₆) δ 7.51-7.47 (m, 2H), 7.44-7.39 (m, 3H), 4.73 (s, 1H), 4.64 (s, 2H), 4.04 (s, 2H), 3.51 (q, *J* = 7.0 Hz, 2H), 1.14 (t, *J* = 7.0 Hz, 3H), 0.73 (s, 6H); ¹³C NMR (101 MHz DMSO-*d*₆) δ 145.27, 130.11, 128.78, 128.68, 128.27, 127.91, 125.17, 69.63, 65.13, 64.45, 54.00, 27.60, 14.98; MS (ESI⁺), calcd for C₁₆H₂₂ClN₂O₂ (M+H): 309.1370, found: 309.1376.

### Example 52: Synthesis of 1-[4-bromo-2-(ethoxymethyl)-5-phenyl-1H-imidazole-1-yl]-2-methylpropan-2-ol (Compound 52)

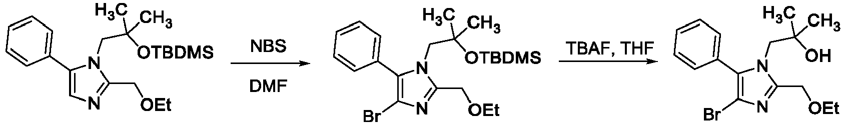

### (1) Synthesis of 4-bromo-1-{2-[(tert-butyldimethylsilyl)oxy]-2-methylpropyl}-2-(ethoxymethyl)-5-phenyl-1H-imidazole

To a solution of 1-{2-[(tert-butyldimethylsilyl)oxy]-2-methylpropyl}-2-(ethoxymethyl)-5-phenyl-1H-imidazole (0.300 g, 0.77 mmol) in N,N-dimethylformamide (11 mL), a solution of N-bromosuccinimide (0.144 g, 0.81 mmol) in N,N-dimethylformamide (4 mL) was added, and the solution was stirred for 18 hours. Afterwards, the solution was diluted with water (15 mL), sodium thiosulfate pentahydrate (solid) was added until no further change in color was observed, and a pale yellow suspension was obtained. The resulting mixture was extracted with ethyl acetate (3 × 20 mL). The combined organic layers were washed with 5% aqueous lithium chloride solution (3 × 40 mL), washed with saturated aqueous sodium chloride solution (40 mL), dried over magnesium sulfate, filtered and concentrated to dryness. The resulting residue was purified by flash SiO₂ chromatography (25 g of silica gel, gradient elution: dichloromethane to 5% methanol/95% dichloromethane), so as to obtain a white solid (0.320 g, yield: 89%). ¹H NMR (400 MHz, DMSO-*d*₆) δ 7.52-7.48 (m, 2H), 7.45-7.40 (m, 3H), 4.56 (s, 2H), 4.13 (s, 2H), 3.50 (q, *J* = 7.0 Hz, 2H), 1.15 (t, *J* = 7.0 Hz, 3H), 0.84 (s, 6H), 0.72 (s, 9H), -0.04 (s, 6H); ¹³C NMR (101 MHz DMSO-*d*₆) δ 146.45, 130.39, 130.09, 129.26, 128.79, 128.40, 113.50, 73.62, 65.25, 64.61, 54.90, 28.18, 25.75, 17.66, 14.94, -2.25; MS (ESI⁺), calcd for C₂₂H₃₆BrN₂O₂Si (M+H): 353.0866, found: 353.0868.

### (2) Synthesis of 1-[4-bromo-2-(ethoxymethyl)-5-phenyl-1H-imidazole-1-yl]-2-methylpropan-2-ol

4-Bromo-1-{2-[(tert-butyldimethylsilyl)oxy]-2-methylpropyl}-2-(ethoxymethyl)-5-pheny l-1H-imidazole (0.050 g, 0.11 mmol) was dissolved in tetrahydrofuran (5 mL), tetrabutylammonium fluoride (0.33 mL, 0.33 mmol, 1M solution in tetrahydrofuran) was added, and the solution was stirred for 17 hours. Afterwards, saturated aqueous ammonium chloride solution (10 mL) was added, and the resulting mixture was extracted with ethyl acetate (3 × 5 mL). The combined organic layers were dried over magnesium sulfate, filtered and concentrated to dryness. The resulting residue was purified by flash SiO₂ chromatography (10 g of silica gel, gradient elution: dichloromethane to 8% methanol/92% dichloromethane), so as to obtain a white solid (0.034 g, yield: 87%). ¹H NMR (400 MHz, DMSO-*d*₆) δ 7.51-7.47 (m, 2H), 7.44-7.38 (m, 3H), 7.43 (s, 1H), 4.64 (s, 2H), 4.04 (s, 2H), 3.50 (q, *J* = 7.0 Hz, 2H), 1.14 (t, *J* = 7.0 Hz, 3H), 0.73 (s, 6H); ¹³C NMR (101 MHz DMSO-*d*₆) δ 146.63, 130.67, 130.38, 129.25, 128.67, 128.37, 113.24, 69.67, 65.19, 64.52, 54.10, 27.65, 15.03; MS (ESI⁺), calcd for C₁₆H₂₂BrN₂O₂ (M+H): 353.0865 & 355.0846, found: 353.0868 & 355.0847.

### Example 53: Synthesis of 1-[2-(ethoxymethyl)-4-iodo-5-phenyl-1H-imidazole-1-yl]-2-methylpropan-2-ol (Compound 53)

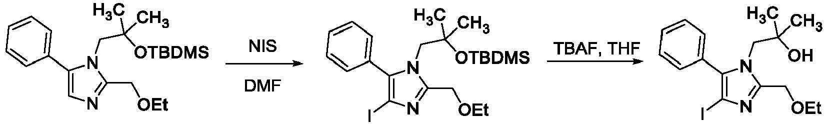

### (1) Synthesis of 1-{2-[(tert-butyldimethylsilyl)oxy]-2-methylpropyl}-2-(ethoxymethyl)-4-iodo-5-phenyl-1H-imidazole

1-{2-[(Tert-butyldimethylsilyl)oxy]-2-methylpropyl}-2-(ethoxymethyl)-5-phenyl-1H-imidazole (0.178g, 0.46 mmol) was dissolved in N,N-dimethylformamide (5 mL), a solution of N-iodosuccinimide (0.113g, 0.50 mmol) in N,N-dimethylformamide (1 mL) was added at 60°C, and the resulting solution was stirred at 60°C for 17 hours. Afterwards, water (10 mL) was added, sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resultant was purified by flash SiO₂ chromatography (10 g of silica gel, gradient elution: hexane to 20% ethyl acetate /80% hexane), so as to obtain a colorless oil (0.094 g, yield: 40%). ¹H NMR (400 MHz, DMSO-*d*₆) δ 7.52-7.48 (m, 2H), 7.44-7.40 (m, 3H), 4.57 (s, 2H), 4.13 (s, 2H), 3.50 (q, *J* = 7.0 Hz, 2H), 1.15 (t, *J* = 7.0 Hz, 3H), 0.84 (s, 6H), 0.73 (s, 9H), -0.03 (s, 6H); ¹³C NMR (101 MHz DMSO-*d*₆) δ 146.46, 130.40, 130.10, 129.27, 128.80, 128.41, 113.50, 73.62, 65.26, 64.61, 54.90, 28.18, 25.76, 17.67, 14.95, -2.24; MS (ESI⁺), calcd for C₂₂H₃₆IN₂O₂Si (M+H): 515.1591, found: 515.1609.

### (2) Synthesis of 1-[2-(ethoxymethyl)-4-iodo-5-phenyl-1H-imidazole-1-yl]-2-methylpropan-2-ol

To a solution of 1-{2-[(tert-butyldimethylsilyl)oxy]-2-methylpropyl}-2-(ethoxymethyl)-4-iodo-5-phenyl-1H-imidazole (0.040g, 0.078 mmol) in tetrahydrofuran (5 mL), tetrabutylammonium fluoride (0.23 mL, 0.23 mmol, 1M solution in tetrahydrofuran) was added, and the solution was stirred at room temperature for 17 hours. Afterwards, saturated aqueous ammonium chloride solution (10 mL) was added, and the mixture was extracted with ethyl acetate (3 × 5 mL). The combined organic layers were washed with saturated aqueous sodium chloride solution (10 mL), dried over magnesium sulfate, filtered and concentrated to dryness. The resulting residue was purified by flash SiO₂ chromatography (10 g of silica gel, gradient elution: hexane to 70% ethyl acetate/30% hexane), so as to obtain a white solid (0.027 g, yield: 87%). ¹H NMR (400 MHz, DMSO-*d*₆) δ 7.51-7.47 (m, 2H), 7.44-7.38 (m, 3H), 4.74 (s, 2H), 4.65 (s, 2H), 4.04 (s, 2H), 3.50 (q, *J* = 7.0 Hz, 2H), 1.14 (t, *J* = 7.0 Hz, 3H), 0.73 (s, 6H); ¹³C NMR (101 MHz DMSO-*d*₆) δ 146.62, 130.65, 130.37, 129.25, 128.66, 128.35, 113.24, 69.66, 65.17, 64.52, 54.09, 27.64, 15.03; MS (ESI⁺), calcd for C₁₆H₂₂IN₂O₂ (M+H): 401.0726, found: 401.0731.

### Example 54: Synthesis of 2-(ethoxymethyl)-1-(2-hydroxy-2-methylpropyl)-5-phenyl-1H-imidazole-4-carboxylic acid (Compound 54)

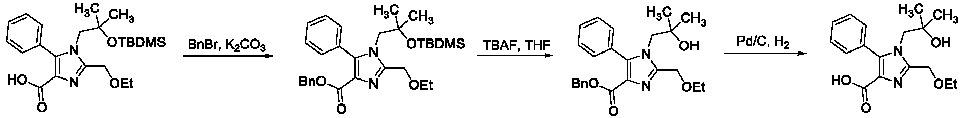

### (1) Synthesis of benzyl 1-{2-[(tert-butyldimethylsilyl)oxy]-2-methylpropyl}-2-(ethoxymethyl)-5-phenyl-1H-imidazole-4-carboxylate

1-{2-[(Tert-butyldimethylsilyl)oxy]-2-methylpropyl}-2-(ethoxymethyl)-5-phenyl-1H-imidazole-4-carboxylic acid (0.100 g, 0.23 mmol) and potassium carbonate (0.128 g, 0.92 mmol) were added to N,N-dimethylformamide (3 mL), benzyl bromide (42 µL, 0.35 mmol) was added, and the resulting mixture was stirred at room temperature for 17 hours. Afterwards, the mixture was diluted with water (20 mL) and extracted with ethyl acetate (4 × 10 mL). The combined organic layers were washed with 5% aqueous lithium chloride solution (3 × 30 mL), washed with saturated aqueous sodium chloride solution (40 mL), dried over magnesium sulfate, filtered and concentrated to dryness. The resulting residue was purified by flash SiO₂ chromatography (10 g of silica gel, gradient elution: hexane to 35% ethyl acetate/65% hexane), so as to obtain a white solid (0.118 g, yield: 98%). ¹H NMR (400 MHz, DMSO-*d*₆) δ 7.44-7.38 (m, 5H), 7.32-7.26 (m, 3H), 7.17-7.12 (m, 2H), 5.10 (s, 2H), 4.65 (s, 2H), 4.07 (s, 2H), 3.49 (q, *J* = 7.0 Hz, 2H), 1.14 (t, *J* = 7.0 Hz, 3H), 0.83 (s, 6H), 0.72 (s, 9H), -0.03 (s, 6H); ¹³C NMR (101 MHz DMSO-*d*₆) δ 162.21, 146.69, 139.36, 136.20, 131.01, 129.54, 128.69, 128.25, 128.09, 127.86, 127.81, 127.78, 73.60, 65.32, 64.97, 64.91, 54.24, 28.32, 25.80, 17.71, 14.98, -2.20; MS (ESI⁺), calcd for C₃₀H₄₃N₂O₄Si (M+H): 523.2992, found: 523.3004.

### (2) Synthesis of benzyl 2-(ethoxymethyl)-1-(2-hydroxy-2-methylpropyl)-5-phenyl-1H-imidazole-4-carboxylate

Benzyl 1-{2-[(tert-butyldimethylsilyl)oxy]-2-methylpropyl}-2-(ethoxymethyl)-5-phenyl-1H-imidazole-4-carboxylate (0.108 g, 0.207 mmol) was dissolved in tetrahydrofuran (3 mL), tetrabutylammonium fluoride (0.62 mL, 0.62 mmol, 1M solution in tetrahydrofuran) was added, and the solution was stirred for 15 hours. Afterwards, saturated aqueous ammonium chloride solution (10 mL) was added, and the resulting mixture was extracted with ethyl acetate (3 × 5 mL). The combined organic layers were washed with saturated aqueous sodium chloride solution (10 mL), dried over magnesium sulfate, filtered and concentrated to dryness. The resulting residue was purified by flash SiO₂ chromatography (10 g of silica gel, gradient elution: hexane to 95% ethyl acetate/5% hexane), so as to obtain a white solid (0.081 g, yield: 95%). ¹H NMR (400 MHz, DMSO-*d*₆) δ 7.43-7.39 (m, 3H), 7.39-7.35 (m, 2H), 7.16-7.12 (m, 2H), 5.08 (s, 2H), 4.79 (s, 1H), 4.72 (s, 2H), 3.97 (s, 2H), 3.49 (q, *J* = 7.0 Hz, 2H), 1.13 (t, *J* = 7.0 Hz, 3H), 0.73 (s, 6H); ¹³C NMR (101 MHz DMSO-*d*₆) δ 162.28, 146.95, 139.65, 136.26, 131.11, 129.63, 128.60, 128.27, 127.98, 127.79, 127.67, 69.59, 65.23, 64.90, 64.88, 53.40, 27.84, 15.06; MS (ESI⁺), calcd for C₂₄H₂₉N₂O₄ (M+H): 409.2127, found: 409.2134.

### (3) Synthesis of 2-(ethoxymethyl)-1-(2-hydroxy-2-methylpropyl)-5-phenyl-1H-imidazole-4-carboxylic acid

To a solution of benzyl 2-(ethoxymethyl)-1-(2-hydroxy-2-methylpropyl)-5-phenyl-1H-imidazole-4-carboxylate (0.076g, 0.19 mmol) in methanol (5 mL), palladium on carbon (0.008 g, content: 5%) was added. The mixture was degassed and backfilled with nitrogen gas (4×), and then degassed and backfilled with hydrogen gas (4×). Thereafter, the reaction mixture was stirred under a hydrogen atmosphere (balloon) for 17 hours. Afterwards, the mixture was degassed and backfilled with nitrogen gas (4×), diluted with dichloromethane (15 mL), filtered through celite, and rinsed with dichloromethane. The filtrate was concentrated to dryness, and the resulting solid was subjected to recrystallization with cold diethyl ether, so as to obtain a white solid (0.056 g, yield: 93%). ¹H NMR (400 MHz, DMSO-*d*₆) δ 11.9 (br s, 1H), 7.46-7.35 (m, 5H), 4.77-4.71 (2×s, 3H), 3.97 (s, 2H), 3.50 (q, *J* = 7.0 Hz, 2H), 1.14 (t, *J* = 7.0 Hz, 3H), 0.72 (s, 6H); ¹³C NMR (101 MHz DMSO-*d*₆) δ 163.88, 146.48, 138.83, 131.15, 129.97, 128.52, 128.39, 127.85, 69.61, 65.18, 64.90, 53.31, 27.82, 15.07; MS (ESI⁺), calcd for C₁₇H₂₃N₂O₄ (M-H): 317.1501, found: 317.1517.

### Example 55: Synthesis of 2-(ethoxymethyl)-1-(2-methylpropyl)-5-phenyl-1H-imidazole (Compound 55)

### (1) Synthesis of 2-(ethoxymethyl)-1-(2-methylpropyl)-1H-imidazole

2-Imidazolecarbaldehyde (5.00 g, 52.0 mmol) and cesium carbonate (33.9 g, 104 mmol) were dissolved in N,N-dimethylformamide (250 mL), 1-bromo-2-methylpropane (6.2 mL, yielding 57 mmol) was added, and the resulting mixture was stirred at room temperature for 18 hours. Afterwards, the mixture was diluted with ethyl acetate (500 mL), washed with water (2 × 300 mL), washed with 5% aqueous lithium chloride solution (2 × 200 mL), washed with saturated aqueous sodium chloride solution (200 mL), dried over magnesium sulfate, filtered and concentrated to dryness, so as to obtain 1-(2-methylpropyl)-1H-imidazole-2-carbaldehyde (crude product) as a yellow oil. The resulting oil was dissolved in methanol (250 mL). Sodium borohydride (2.95 g, 78.0 mmol) was added and the resultant was stirred at room temperature for 17 hours. Afterwards, saturated aqueous ammonium chloride solution (200 mL) was added, and the resulting mixture was extracted with dichloromethane (3 × 200 mL). The combined organic layers were washed with saturated aqueous sodium chloride solution (400 mL), dried over sodium sulfate, filtered and concentrated to dryness. The resulting residue was subjected to azeotropic distillation with dichloromethane and placed in a high vacuum pump for several hours, so as to obtain crude [1-(2-methylpropyl)-1H-imidazole-2-yl]methanol as a clear yellow oil. The yellow oil was dissolved in N,N-dimethylformamide (250 mL) and cooled in an ice bath (≈0°C). Sodium hydride (4.17 g, 104 mmol, 60% dispersed in mineral oil) was added, and the mixture was stirred in an ice bath for 45 minutes. Afterwards, bromoethane (9.7 mL, 130 mmol) was added, and the mixture was stirred in an ice bath for 1 hour. Afterwards, the ice bath was removed, and the mixture was stirred at room temperature for 15 hours. Afterwards, saturated aqueous ammonium chloride solution (200 mL) and water (50 mL) were added, and the resulting mixture was extracted with ethyl acetate (500 mL). The organic layer was washed with water (2 × 400 mL), washed with 5% aqueous lithium chloride solution (400 mL), washed with saturated aqueous sodium chloride solution (400 mL), dried over magnesium sulfate, filtered and concentrated to dryness. The resulting residue was filtered through a silica gel plug (about 70 g), and the filtrate was concentrated to obtain a colorless oil (4.966 g, yield for three steps: 52%). ¹H NMR (400 MHz, DMSO-*d*₆) δ 7.15 (d, *J* = 1.2 Hz, 1H), 6.81 (d, *J* = 1.2 Hz, 1H), 4.44 (s, 2H), 3.75 (d, *J* = 7.5 Hz, 2H), 3.42 (q, *J* = 7.0 Hz, 2H), 2.05 (sep, *J* = 6.7 Hz, 1H), 1.09 (t, *J* = 7.0 Hz, 3H), 0.84 (d, *J* = 6.7 Hz, 6H); ¹³C NMR (101 MHz DMSO-*d*₆) δ 144.17, 126.60, 121.37, 64.71, 63.62, 52.46, 29.24, 19.66, 14.96; MS (ESI⁺), calcd for C₁₀H₁₉N₂O (M+H): 183.1497, found: 183.1498.

### (2) Synthesis of 2-(ethoxymethyl)-1-(2-methylpropyl)-5-phenyl-1H-imidazole

2-(Ethoxymethyl)-1-(2-methylpropyl)-1H-imidazole (1.00g, 1.3 mmol), bromobenzene (0.44 mL, 4.2 mmol) and potassium acetate (1.16g, 8.40 mmol) were dissolved in N,N-dimethylacetamide (30 mL), and the mixture was degassed and backfilled with nitrogen gas (4×). Palladium (II) acetate (0.094g, 0.42 mmol) was added, and the mixture was degassed, backfilled with nitrogen gas (4×) again and heated at 150°C for 16 hours. Afterwards, the mixture was cooled to room temperature, diluted with ethyl acetate (120 mL), washed with water (100 mL), washed with 5% aqueous lithium chloride solution (3 × 100 mL), washed with saturated aqueous sodium chloride solution (100 mL), dried over magnesium sulfate, filtered and concentrated to dryness. The resulting residue was purified by flash SiO₂ chromatography (100 g of silica gel, gradient elution: hexane to 50% ethyl acetate/50% hexane), so as to obtain a yellow oil (0.540 g, yield: 38%). ¹H NMR (400 MHz, DMSO-*d*₆) δ 7.48-7.43 (m, 4H), 7.41-7.36 (m, 1H), 6.92 (s, 1H), 4.52 (s, 2H), 3.94 (d, *J* = 7.7 Hz, 2H), 3.51 (q, *J* = 7.0 Hz, 2H), 1.64 (sep, *J* = 6.7 Hz, 1H), 1.13 (t, *J* = 7.0 Hz, 3H), 0.60 (d, *J* = 6.7 Hz, 6H); ¹³C NMR (101 MHz DMSO-*d*₆) δ 145.76, 133.60, 130.77, 128.79, 128.23, 127.75, 126.62, 64.90, 64.23, 50.62, 28.48, 19.36, 14.96.

### Example 56: Synthesis of 4-chloro-2-(ethoxymethyl)-1-(2-methylpropyl)-5-phenyl-1H-imidazole (Compound 56)

A solution of 2-(ethoxymethyl)-1-(2-methylpropyl)-5-phenyl-1H-imidazole (0.050 g, 0.19 mmol) in N,N-dimethylformamide (2 mL) was heated to 60°C, a solution of N-chlorosuccinimide (0.039 g, 0.29 mmol) in N,N-dimethylformamide (1 mL) was added, and the resultant was stirred at 60°C for 18 hours. Afterwards, the solution was cooled to room temperature and diluted with water (10 mL). Sodium thiosulfate pentahydrate (solid) was added until the mixture was white, and the mixture was extracted with ethyl acetate (10 mL). The organic layer was washed with 5% aqueous lithium chloride solution (2 × 10 mL), washed with saturated aqueous sodium chloride solution (10 mL), dried over magnesium sulfate, filtered and concentrated to dryness. The resulting residue was purified by flash SiO₂ chromatography (10 g of silica gel, gradient elution: hexane to 40% ethyl acetate/60% hexane), so as to obtain a white solid (0.029 g, yield: 52%). ¹H NMR (400 MHz, DMSO-*d*₆) δ 7.53-7.41 (m, 5H), 4.50 (s, 2H), 3.86 (d, *J* = 7.7 Hz, 2H), 3.51 (q, *J* = q, 7.0 Hz, 2H), 1.57 (sep, *J* = 6.7 Hz, 1H), 1.14 (t, *J* = 7.0 Hz, 3H), 0.59 (d, *J* = 6.7 Hz, 6H); ¹³C NMR (101 MHz DMSO-*d*₆) δ 143.54, 129.73, 128.84, 128.67, 128.37, 127.64, 125.19, 65.08, 63.62, 51.31, 28.20, 19.28, 14.92.

### Example 57: Synthesis of 4-bromo-2-(ethoxymethyl)-1-(2-methylpropyl)-5-phenyl-1H-imidazole (Compound 57)

2-(Ethoxymethyl)-1-(2-methylpropyl)-5-phenyl-1H-imidazole (0.107 g, 0.414 mmol) was dissolved in N,N-dimethylformamide (4 mL), a solution of N-bromosuccinimide (0.077 g, 0.43 mmol) in N,N-dimethylformamide (2 mL) was added, and the mixture was stirred at room temperature for 48 hours. Afterwards, the solution was diluted with water (15 mL), sodium thiosulfate pentahydrate (solid) was added until the mixture was white, and the mixture was extracted with ethyl acetate (20 mL). The organic layer was washed with 5% aqueous lithium chloride solution (2 × 20 mL), washed with saturated aqueous sodium chloride solution (20 mL), dried over magnesium sulfate, filtered and concentrated to dryness. The resultant was purified by flash SiO₂ chromatography (25 g of silica gel, gradient elution: hexane to 40% ethyl acetate/60% hexane), so as to obtain a white solid (0.123 g, yield: 88%). ¹H NMR (400 MHz, DMSO-*d*₆) δ 7.54-7.40 (m, 5H), 4.50 (s, 2H), 3.85 (d, *J* = 7.7 Hz, 2H), 3.51 (q, *J* = 7.0 Hz, 2H), 1.57 (sep, *J* = 6.7 Hz, 1H), 1.14 (t, *J* = 7.0 Hz, 3H), 0.59 (d, *J* = 6.7 Hz, 6H).

### Example 58: Synthesis of 2-(ethoxymethyl)-4-iodo-1-(2-methylpropyl)-5-phenyl-1H-imidazole (Compound 58)

2-(Ethoxymethyl)-1-(2-methylpropyl)-5-phenyl-1H-imidazole (0.050 g, 0.19 mmol) was dissolved in N,N-dimethylformamide (2 mL), a solution of N-iodosuccinimide (0.065 g, 0.29 mmol) in N,N-dimethylformamide (1 mL) was added, and the mixture was stirred at 60°C for 18 hours. Afterwards, the solution was cooled to room temperature and diluted with water (10 mL). Sodium thiosulfate pentahydrate (solid) was added until the mixture was white, and the mixture was extracted with ethyl acetate (20 mL). The organic layer was washed with 5% aqueous lithium chloride solution (2 × 20 mL), washed with saturated aqueous sodium chloride solution (20 mL), dried over magnesium sulfate, filtered and concentrated to dryness. The resulting residue was purified by flash SiO₂ chromatography (10 g of silica gel, gradient elution: hexane to 40% ethyl acetate/60% hexane), so as to obtain a white solid (0.067 g, yield: 92%). ¹H NMR (400 MHz, DMSO-*d*₆) δ 7.52-7.43 (m, 3H), 7.39-7.36 (m, 2H), 4.49 (s, 2H), 3.83 (d, *J* = 7.7 Hz, 2H), 3.51 (q, *J* = 7.0 Hz, 2H), 1.55 (sep, *J* = 6.7 Hz, 1H), 1.13 (t, *J* = 7.0 Hz, 3H), 0.57 (d, *J* = 6.7 Hz, 6H); ¹³C NMR (101 MHz DMSO-*d*₆) δ 147.18, 135.52, 130.22, 129.89, 128.72, 128.68, 84.64, 65.07, 63.70, 51.40, 28.25, 19.31, 14.94.

### Example 59: Synthesis of 4-iodo-1-(2-methylpropyl)-5-phenyl-1H-imidazole (Compound 59)

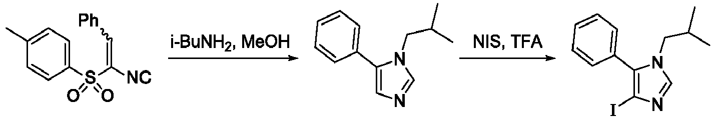

### (1) Synthesis of 1-(2-methylpropyl)-5-phenyl-1H-imidazole

1-(1-Isocyano-2-phenylsulfonyl)-4-methylbenzene (0.094 g, 0.33 mmol, prepared according to the method reported in WO 2011056652 A1) was dissolved in methanol (4 mL), isobutylamine (66 µL, 0.66 mmol) was added, and the mixture was stirred at room temperature for 17 hours. Afterwards, the solution was concentrated to dryness and purified by flash SiO₂ chromatography (25 g of silica gel, gradient elution: dichloromethane to 7% methanol/93% dichloromethane), so as to obtain a white solid (0.045 g, yield: 68%). ¹H NMR (400 MHz, DMSO-*d*₆) δ 7.73 (s, 1H), 7.48-7.43 (m, 4H), 7.40-7.35 (m, 1H), 6.99 (s, 1H), 3.89 (d, *J* = 7.5 Hz, 2H), 1.66 (sep, *J* = 6.7 Hz, 1H), 0.66 (d, *J* = 6.7 Hz, 6H).

### (2) Synthesis of 4-iodo-1-(2-methylpropyl)-5-phenyl-1H-imidazole

1-(2-Methylpropyl)-5-phenyl-1H-imidazole (0.046g, 0.23 mmol) was dissolved in acetonitrile (3 mL), trifluoroacetic acid (5 µL, 0.07 mmol) and N-Iodosuccinimide (0.056g, 0.25 mmol) were added, and the mixture was stirred at 80°C for 16 hours. Afterwards, the solution was cooled to room temperature and diluted with water (9 mL). Sodium thiosulfate pentahydrate (solid) was added until no change in color was observed, saturated sodium bicarbonate solution (1 mL) was added, and the resultant was extracted with ethyl acetate (3 × 5 mL). The combined organic layers were washed with saturated aqueous sodium chloride solution (30 mL), dried over magnesium sulfate, filtered and concentrated to dryness. The resulting residue was purified by flash SiO₂ chromatography (10 g of silica gel, gradient elution: dichloromethane to 5% methanol/95% dichloromethane), so as to obtain a yellow oil (0.060 g, yield: 80%). ¹H NMR (400 MHz, DMSO-*d*₆) δ 7.82 (s, 1H), 7.52-7.42 (m, 3H), 7.39-7.36 (m, 2H), 3.77 (d, *J* = 7.5 Hz, 2H), 1.56 (sep, *J* = 6.7 Hz, 1H), 0.62 (d, *J* = 6.7 Hz, 6H); ¹³C NMR (101 MHz DMSO-*d*₆) δ 140.66, 134.14, 130.15, 129.46, 128.74, 128.63, 85.28, 52.71, 28.57, 19.31; MS (ESI⁺), calcd for C₁₃H₁₇N₂ (M+H): 327.0358, found: 327.0349.

### Example 60: Synthesis of 1-(5-([1,1'-biphenyl]-4-yl)-2-(ethoxymethyl)-4-iodo-1H-imidazole-1-yl)-2-methylpropan-2-ol (Compound 60)

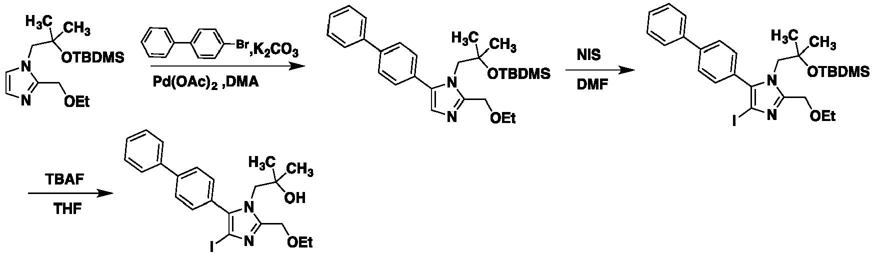

(1) The product 1-{2-[(tert-butyldimethylsilyl)oxy]-2-methylpropyl}-2-(ethoxymethyl)-1H-imidazole (312 mg, 1.00 mmol) synthesized in step (2) of Example 38 was weighed and added to a 25-mL round-bottom flask. 4-Bromobiphenyl (466 mg, 2.00 mmol), potassium carbonate (552 mg, 4.00 mmol) and palladium acetate (38 mg, 0.17 mmol) were added, and 10 mL of DMA was added as a solvent. The reaction system was repeatedly purged with nitrogen gas for 3 times and then protected with a nitrogen balloon, heated to 80°C, and stirred and reacted for 12h. Afterwards, a large amount of water was added to the reaction mixture, which was then extracted with ethyl acetate (3 × 100 mL), washed with saturated aqueous sodium chloride solution (200 mL), dried over anhydrous sodium sulfate, filtered and evaporated to dryness via rotary evaporation. The resultant was purified by flash SiO₂ chromatography (petroleum ether : ethyl acetate = 3 : 1), so as to obtain 5-([1,1'-biphenyl] -4-yl)-1-(2-((tert-butyldimethylsilyl)oxy)-2-methylpropyl)-2-(ethoxymethyl)-1H-imidazole as brown oil (60 mg, yield: 13%). ¹H NMR (400 MHz, chloroform-*d*) δ 7.66-7.62 (m, 4H), 7.49-7.40 (m, 5H), 7.03 (s, 1H), 4.25 (s, 2H), 3.58 (q, *J* = 7.0 Hz, 2H), 1.25 (d, *J* = 7.0 Hz, 3H), 0.95 (s, 6H), 0.78 (s, 9H), -0.02 (s, 6H). ¹³C NMR (101 MHz, chloroform-*d*) δ 147.87, 140.43, 134.25, 130.76, 129.26, 128.98, 127.74, 127.66, 127.53, 127.09, 77.16, 74.54, 66.05, 65.89, 55.16, 28.72, 26.07, 18.14, 15.27, -1.92.
(2) 5-([1,1'-Biphenyl]-4-yl)-1-(2-((tert-butyldimethylsilyl)oxy)-2-methylpropyl)-2-(ethoxymethyl)-1H-imidazole (56 mg, 0.12 mmol) was weighed and added to a 25-mL round-bottom flask, N-iodosuccinimide (30 mg, 0.13 mmol) and DMF (as a solvent) were added successively, and the mixture was heated to 60°C and reacted for 12 hours. Afterwards, water (10 mL) was added, sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resultant was purified by flash SiO₂ chromatography (10 g of silica gel, gradient elution: hexane to 20% ethyl acetate/80% hexane), so as to obtain 5-([1,1'-biphenyl]-4-yl)-1-(2-((tert-butyldimethylsilyl)oxy)-2-methylpropyl)-2-(ethoxymethyl)-4-iodo-1H-imidazole as a pale yellow liquid (32 mg, yield: 45%). ¹H NMR (400 MHz, chloroform-*d*) δ 7.67 (dd, *J* = 15.5, 7.8 Hz, 4H), 7.51-7.34 (m, 5H), 4.75 (s, 2H), 4.19 (s, 2H), 3.58 (q, *J* = 7.0 Hz, 2H), 0.93 (s, 6H), 0.79 (s, 9H), -0.01 (s, 6H). ¹³C NMR (101 MHz, chloroform-*d*) δ 149.23, 141.09, 140.17, 135.72, 133.22, 130.99, 129.51, 128.88, 127.71, 127.26, 127.06, 74.25, 65.96, 65.63, 55.77, 28.59, 25.97, 17.91, 15.13, -2.01.
(3) 5-([1,1'-Biphenyl]-4-yl)-1-(2-((tert-butyldimethylsilyl)oxy)-2-methylpropyl)-2-(ethoxymethyl)-4-iodo-1H-imidazole (27 mg, 0.05 mmol) was weighed and added to a 25-mL round-bottom flask, and TBAF (140 µL, with a concentration of 1M in tetrahydrofuran, 0.14 mmol) and tetrahydrofuran (5 mL) were added successively. The mixture was stirred at room temperature overnight, tetrahydrofuran was removed via rotary evaporation under reduced pressure, and purified water (50 mL) and ethyl acetate (3×50 mL) were added to conduct extraction. The resulting mixture was washed with saturated aqueous sodium chloride solution (100 mL), dried over anhydrous sodium sulfate, filtered and evaporated to dryness via rotary evaporation. The resultant was purified by flash SiO₂ chromatography (dichloromethane : methanol = 30 : 1), so as to obtain a pale yellow oily liquid (21 mg, yield: 99%). ¹H NMR (400 MHz, chloroform-*d*) δ 7.71 (d, *J* = 8.3 Hz, 2H), 7.66 (d, *J* = 7.3 Hz, 2H), 7.47 (d, *J* = 15.1 Hz, 2H), 7.39 (d, *J* = 7.8 Hz, 3H), 4.68 (s, 2H), 4.17 (s, 2H), 3.68 (q, *J* = 7.0 Hz, 2H), 3.61 (s, 1H), 1.26 (d, *J* = 7.0 Hz, 3H), 0.93 (s, 6H). ¹³C NMR (101 MHz, chloroform-*d*) δ 147.61, 141.54, 140.02, 136.64, 131.26, 128.91, 128.82, 127.82, 127.36, 127.08, 84.98, 70.45, 66.31, 64.61, 55.59, 27.74, 14.90.

### Example 61: Synthesis of 1-(5-([1,1'-biphenyl]-3-yl)-2-(ethoxymethyl)-4-iodo-1H-imidazole-1-yl)-2-methylpropan-2-ol (Compound 61)

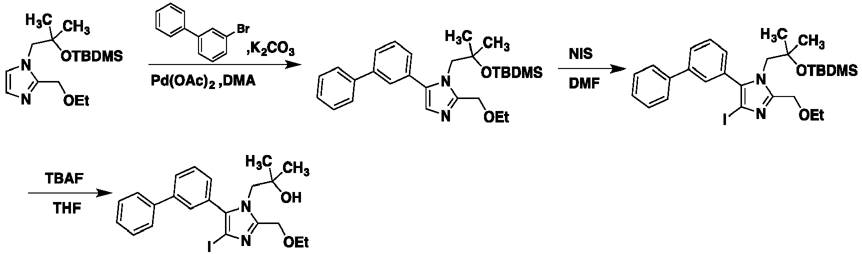

(1) The product 1-{2-[(tert-butyldimethylsilyl)oxy]-2-methylpropyl}-2-(ethoxymethyl)-1H-imidazole (312mg,1.00 mmol) synthesized in step (2) of Example 38 was weighed and added to a 25-mL round-bottom flask, 3-bromobiphenyl (466 mg, 2.00 mmol), potassium carbonate (552 mg, 4.00 mmol) and palladium acetate (38 mg, 0.17 mmol) were added, and 10 mL of DMA was added as a solvent. The reaction system was repeatedly purged with nitrogen gas for 3 times and then protected with a nitrogen balloon, heated to 80°C, and stirred and reacted for 12h. Afterwards, a large amount of water was added to the reaction mixture, which was then extracted with ethyl acetate (3 × 100 mL), washed with saturated aqueous sodium chloride solution (200 mL), dried over anhydrous sodium sulfate, filtered and evaporated to dryness via rotary evaporation. The resultant was purified by flash SiO₂ chromatography (petroleum ether : ethyl acetate = 3 : 1), so as to obtain 5-([1,1'-biphenyl]-3-yl)-1-(2-((tert-butyldimethylsilyl)oxy)-2-methylpropyl)-2-(ethoxymethyl)-1H-imidazole as a pale yellow oil (48 mg, yield: 10%). ¹H NMR (400 MHz, chloroform-*d*) δ 7.61-7.54 (m, 4H), 7.50-7.43 (m, 3H), 7.39-7.31 (m, 2H), 7.04 (s, 1H), 4.78 (s, 2H), 4.24 (s, 2H), 3.58 (q, *J* = 7.0 Hz, 2H), 1.24 (t, *J* = 7.0 Hz, 3H), 0.94 (s, 6H), 0.78 (s, 9H). ¹³C NMR (101 MHz, chloroform-*d*) δ 147.71, 141.85, 140.60, 134.38, 132.21, 129.22, 128.87, 127.68, 127.65, 127.56, 127.15, 126.43, 74.42, 65.93, 65.81, 55.08, 28.62, 25.96, 18.02, 15.15, -2.04.
(2) 5-([1,1'-Biphenyl]-3-yl)-1-(2-((tert-butyldimethylsilyl)oxy)-2-methylpropyl)-2-(ethoxymethyl)-1H-imidazole (48 mg, 0.10 mmol) was weighed and added to a 25-mL round-bottom flask, N-iodosuccinimide (28 mg, 0.12 mmol) and DMF (as a solvent) were added successively, and the mixture was heated to 60°C and reacted for 12 hours. Afterwards, water (10 mL) was added, sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resultant was purified by flash SiO₂ chromatography (10 g of silica gel, gradient elution: hexane to 20% ethyl acetate/80% hexane), so as to obtain 5-([1,1'-biphenyl]-3-yl)-1-(2-((tert-butyldimethylsilyl)oxy)-2-methylpropyl)-2-(ethoxymethyl)-4-iodo-1H-imidazole as a pale yellow liquid (13 mg, yield: 21%). ¹H NMR (400 MHz, chloroform-*d*) δ 7.67-7.58 (m, 4H), 7.50 (dt, *J* = 19.6, 7.7 Hz, 3H), 7.41-7.30 (m, 2H), 4.75 (s, 2H), 4.19 (s, 2H), 3.58 (q, *J* = 7.0 Hz, 2H), 1.25 (d, *J* = 7.0 Hz, 3H), 0.92 (s, 6H), 0.78 (s, 9H), -0.02 (s, 6H). ¹³C NMR (101 MHz, chloroform-*d*) δ 149.20, 141.63, 140.42, 135.96, 131.09, 129.47, 129.27, 129.11, 127.69, 127.17, 84.76, 74.26, 66.00, 65.63, 55.83, 28.59, 25.96, 18.03, 15.13, -2.02.
(3) 5-([1,1'-Biphenyl]-3-yl)-1-(2-((tert-butyldimethylsilyl)oxy)-2-methylpropyl)-2-(ethoxymethyl)-4-iodo-1H-imidazole (13 mg, 0.02 mmol) was weighed and added to a 25-mL round-bottom flask, and TBAF (66 µL, with a concentration of 1M in tetrahydrofuran, 0.06 mmol) and tetrahydrofuran (5 mL) were added successively. The mixture was stirred at room temperature overnight, tetrahydrofuran was removed via rotary evaporation under reduced pressure, and purified water (50 mL) and ethyl acetate (3×50 mL) were added to conduct extraction. The resulting mixture was washed with saturated aqueous sodium chloride solution (100 mL), dried over anhydrous sodium sulfate, filtered and evaporated to dryness via rotary evaporation. The resultant was purified by flash SiO₂ chromatography (dichloromethane : methanol = 30 : 1), so as to obtain a pale yellow oily liquid (10 mg, yield: 95%). ¹H NMR (400 MHz, chloroform-*d*) δ 7.71-7.54 (m, 5H), 7.50 (t, *J* = 7.5 Hz, 2H), 7.41 (t, *J* = 7.2 Hz, 1H), 7.31 (d, *J* = 7.6 Hz, 1H), 4.71 (s, 2H), 4.20 (s, 2H), 3.71 (q, *J* = 7.0 Hz, 2H), 3.61 (s, 1H), 1.29 (s, 3H), 0.95 (s, 6H). ¹³C NMR (101 MHz, chloroform-*d*) δ 147.58, 141.72, 140.23, 136.90, 130.44, 129.71, 129.51, 129.22, 128.95, 127.79, 127.63, 127.15, 85.30, 70.45, 66.33, 64.56, 55.63, 27.74, 14.89.

### Example 62: Synthesis of 1-(2-(ethoxymethyl)-4-iodo-5-(naphthalen-1-yl)-1H-imidazole-1-yl)-2-methylpropan-2-ol (Compound 62)

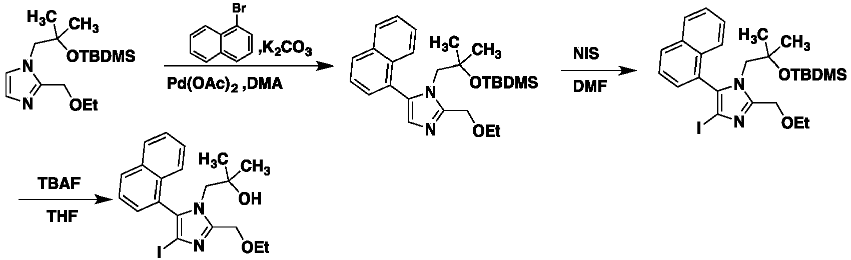

(1) The product 1-{2-[(tert-butyldimethylsilyl)oxy]-2-methylpropyl}-2-(ethoxymethyl)-1H-imidazole (312 mg, 1.00 mmol) synthesized in step (2) of Example 38 was weighed and added to a 25-mL round-bottom flask, 1-bromonaphthalene (416 mg, 2.00 mmol), potassium carbonate (552 mg, 4.00 mmol) and palladium acetate (38 mg, 0.17 mmol) were added, and 10 mL of DMA was added as a solvent. The reaction system was repeatedly purged with nitrogen gas for 3 times and then protected with a nitrogen balloon, heated to 80°C, and stirred and reacted for 12h. Afterwards, a large amount of water was added to the reaction mixture, which was then extracted with ethyl acetate (3 × 100 mL), washed with saturated aqueous sodium chloride solution (200 mL), dried over anhydrous sodium sulfate, filtered and evaporated to dryness via rotary evaporation. The resultant was purified by flash SiO₂ chromatography (petroleum ether : ethyl acetate = 3 : 1) , so as to obtain 1-(2-((tert-butyldimethylsilyl)oxy)-2-methylpropyl)-2-(ethoxymethyl)-5-(naphthalen-1-yl)-1H-imidazole as a brown oily liquid (173 mg, yield: 31%). ¹H NMR (400 MHz, chloroform-*d*) δ 7.90-7.85 (m, 2H), 7.49 (ddtd, *J* = 12.7, 8.5, 7.0, 1.7 Hz, 5H), 7.12 (s, 1H), 4.86 (s, 2H), 4.29-3.87 (m, 2H), 3.63 (q, *J* = 7.0 Hz, 2H), 1.28 (t, *J* = 7.0 Hz, 3H), 0.84 (s, 3H), 0.78 (s, 9H), 0.74 (s, 3H), -0.05 (s, 6H). ¹³C NMR (101 MHz, chloroform-*d*) δ 148.05, 134.43, 132.86, 132.25, 129.53, 129.28, 129.21, 129.01, 128.92, 127.20, 126.59, 125.93, 125.64, 74.45, 66.40, 66.15, 55.85, 26.43, 18.49, 15.69, -1.61.
(2) 1-(2-((Tert-butyldimethylsilyl)oxy)-2-methylpropyl)-2-(ethoxymethyl)-5-(naphthalen-1-yl)-1H-imidazole (173 mg, 0.37 mmol) was weighed and added to a 25-mL round-bottom flask, N-iodosuccinimide (101 mg, 0.45 mmol) and DMF (as a solvent) were added successively, and the mixture was heated to 60°C and reacted for 12 hours. Afterwards, water (10 mL) was added, sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resultant was purified by flash SiO₂ chromatography (10 g of silica gel, gradient elution: hexane to 20% ethyl acetate/80% hexane), so as to obtain 1-(2-((tert-butyldimethylsilyl)oxy)-2-methylpropyl)-2-(ethoxymethyl)-4-iodo-5-(naphthalen-1-yl)-1H-imidazole as a pale yellow solid (135 mg, yield: 64%). ¹H NMR (400 MHz, chloroform-*d*) δ 7.92 (dd, *J* = 11.8, 8.4 Hz, 2H), 7.58-7.46 (m, 5H), 4.90-4.71 (m, 2H), 4.17 (d, *J* = 13.8 Hz, 1H), 3.79 (d, *J* = 13.8 Hz, 1H), 3.67-3.58 (m, 2H), 1.27 (t, *J* = 6.6 Hz, 3H), 0.85 (s, 3H), 0.77 (s, 9H), 0.75 (s, 3H), -0.05 (d, *J* = 4.5 Hz, 6H). ¹³C NMR (101 MHz, chloroform-*d*) δ 149.36, 134.67, 133.76, 131.97, 130.67, 129.72, 128.63, 127.57, 126.92, 126.26, 125.67, 125.12, 86.33, 73.80, 65.87, 65.61, 56.22, 28.64, 25.95, 18.03, 15.18, -2.07.
(3) 1-(2-((Tert-butyldimethylsilyl)oxy)-2-methylpropyl)-2-(ethoxymethyl)-4-iodo-5-(naphthalen-1-yl)-1H-imidazole (130 mg, 0.23 mmol) was weighed and added to a 25-mL round-bottom flask, and TBAF (690 µL, with a concentration of 1M in tetrahydrofuran, 0.69 mmol) and tetrahydrofuran (5 mL) were added successively. The mixture was stirred at room temperature overnight, tetrahydrofuran was removed via rotary evaporation under reduced pressure, and purified water (50 mL) and ethyl acetate (3×50 mL) were added to conduct extraction. The resulting mixture was washed with saturated aqueous sodium chloride solution (100 mL), dried over anhydrous sodium sulfate, filtered and evaporated to dryness via rotary evaporation. The resultant was purified by flash SiO₂ chromatography (dichloromethane : methanol = 30 : 1), so as to obtain 1-(2-(ethoxymethyl)-4-iodo-5-(naphthalen-1-yl)-1H-imidazole-1-yl)-2-methylpropan-2-ol as a pale yellow solid (89 mg, yield: 86%). ¹H NMR (400 MHz, chloroform-d) δ 8.01 (d, *J* = 8.3 Hz, 1H), 7.96 (dd, *J* = 7.8, 1.4 Hz, 1H), 7.64-7.55 (m, 2H), 7.55-7.48 (m, 2H), 7.44 (d, *J* = 8.3 Hz, 1H), 4.83-4.70 (m, 2H), 4.14 (d, *J* = 15.0 Hz, 1H), 3.83-3.66 (m, 4H), 1.30 (t, *J* = 7.0 Hz, 3H), 0.90 (s, 3H), 0.81 (s, 3H).

### Example 63: Synthesis of 1-(2-(ethoxymethyl)-4-iodo-5-(naphthalen-2-yl)-1H-imidazole-1-yl)-2-methylpropan-2-ol (Compound 63)

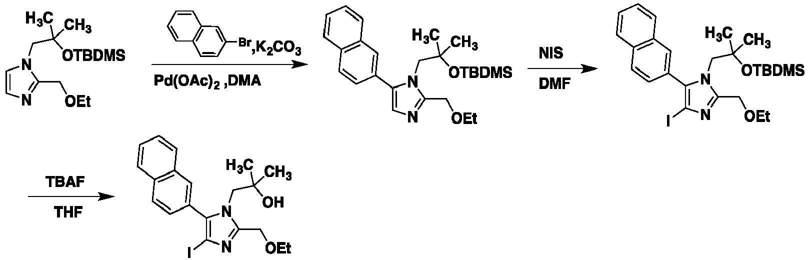

(1) The product 1-{2-[(tert-butyldimethylsilyl)oxy]-2-methylpropyl}-2-(ethoxymethyl) 1H-imidazole (312 mg, 1.00 mmol) synthesized in step (2) of Example 38 was weighed and added to a 25-mL round-bottom flask, 2-bromonaphthalene (416 mg, 2.00 mmol), potassium carbonate (552 mg, 4.00 mmol) and palladium acetate (38 mg, 0.17 mmol) were added, and 10 mL of DMA was added as a solvent. The reaction system was repeatedly purged with nitrogen gas for 3 times and then protected with a nitrogen balloon, heated to 80°C, and stirred and reacted for 12h. Afterwards, a large amount of water was added to the reaction mixture, which was then extracted with ethyl acetate (3 × 100 mL), washed with saturated aqueous sodium chloride solution (200 mL), dried over anhydrous sodium sulfate, filtered and evaporated to dryness via rotary evaporation. The resultant was purified by flash SiO₂ chromatography (petroleum ether : ethyl acetate = 3 : 1), so as to obtain 1-(2-((tert-butyldimethylsilyl)oxy)-2-methylpropyl)-2-(ethoxymethyl)-5-(naphthalen-2-yl)-1H-imidazole as a brown oily liquid (111 mg, yield: 20%). ¹H NMR (400 MHz, chloroform-*d*) δ 7.89-7.79 (m, 4H), 7.54-7.44 (m, 3H), 7.08 (s, 1H), 4.81 (s, 2H), 4.29 (s, 2H), 3.65-3.51 (m, 2H), 1.25 (t, *J* = 7.0 Hz, 3H), 0.89 (s, 6H), 0.77 (s, 9H), -0.04 (s, 6H). ¹³C NMR (101 MHz, chloroform-*d*) δ 147.86, 134.46, 133.44, 132.52, 129.12, 128.53, 127.98, 127.93, 127.75, 127.57, 126.66, 126.52, 126.30, 74.43, 65.96, 65.78, 55.17, 28.60, 25.96, 18.02, 15.18, -2.04.
(2) 1-(2-((Tert-butyldimethylsilyl)oxy)-2-methylpropyl)-2-(ethoxymethyl)-5-(naphthalen-2-yl)-1H-imidazole (100 mg, 0.23 mmol) was weighed and added to a 25-mL round-bottom flask, N-iodosuccinimide (62 mg, 0.27 mmol) and DMF (as a solvent) were added successively, and the mixture was heated to 60°C and reacted for 12 hours. Afterwards, water (10 mL) was added, sodium thiosulfate pentahydrate (solid) was added until no further change in color was observed, and the resulting mixture was extracted with ethyl acetate (3 × 10 mL). The combined organic layers were washed with 5% aqueous lithium chloride solution (3 × 30 mL), washed with saturated aqueous sodium chloride solution (30 mL), dried over magnesium sulfate, filtered and concentrated to dryness. The resultant was purified by flash SiO₂ chromatography (10 g of silica gel, gradient elution: hexane to 20% ethyl acetate/80% hexane), so as to obtain 1-(2-((tert-butyldimethylsilyl)oxy)-2-methylpropyl)-2-(ethoxymethyl)-4-iodo-5-(naphthalen-2-yl)-1H-imidazole as a pale yellow liquid (13 mg, yield: 21%). ¹H NMR (400 MHz, chloroform-d) δ 7.94-7.82 (m, 4H), 7.54 (d, *J* = 5.0 Hz, 2H), 7.47 (s, 1H), 4.77 (s, 2H), 4.22 (s, 2H), 3.59 (q, *J* = 7.0 Hz, 2H), 1.26 (d, *J* = 6.9 Hz, 3H), 0.87 (s, 6H), 0.78 (s, 9H), -0.03 (s, 6H). ¹³C NMR (101 MHz, chloroform-*d*) δ 149.28, 135.98, 133.15, 132.86, 130.09, 128.41, 128.17, 128.03, 127.93, 127.83, 126.81, 126.57, 84.91, 74.24, 65.66, 55.87, 42.19, 28.34, 25.97, 18.03, 15.15, -2.02.
(3) 1-(2-((Tert-butyldimethylsilyl)oxy)-2-methylpropyl)-2-(ethoxymethyl)-4-iodo-5-(naphthalen-2-yl)-1H-imidazole (10 mg, 0.02 mmol) was weighed and added to a 25-mL round-bottom flask, and TBAF (66 µL, with a concentration of 1M in tetrahydrofuran, 0.06 mmol) and tetrahydrofuran (5 mL) were added successively. The mixture was stirred at room temperature overnight, tetrahydrofuran was removed via rotary evaporation under reduced pressure, and purified water (50 mL) and ethyl acetate (3×50 mL) were added to conduct extraction. The resulting mixture was washed with saturated aqueous sodium chloride solution (100 mL), dried over anhydrous sodium sulfate, filtered and evaporated to dryness via rotary evaporation. The resultant was purified by flash SiO₂ chromatography (dichloromethane : methanol = 30 : 1), so as to obtain a pale yellow oily liquid (5 mg, yield: 63%). ¹H NMR (400 MHz, chloroform-*d*) δ 7.98 (d, *J* = 8.2 Hz, 1H), 7.93 (d, *J* = 7.9 Hz, 1H), 7.60-7.39 (m, 5H), 4.78-4.69 (m, 2H), 4.11 (d, *J* = 15.0 Hz, 1H), 3.72 (qd, *J* = 6.9, 4.6 Hz, 3H), 1.27 (t, *J* = 7.0 Hz, 4H), 0.87 (s, 3H), 0.78 (s, 3H). ¹³C NMR (101 MHz, chloroform-*d*) δ 147.57, 136.95, 133.08, 130.61, 128.59, 128.23, 127.89, 127.34, 127.07, 126.72, 119.44, 85.21, 70.45, 66.32, 64.60, 55.70, 29.70, 27.69, 14.90.

### Example 64: Synthesis of 1-(2-(ethoxymethyl)-4-iodo-5-(p-tolyl)-1H-imidazole-1-yl)-2-methylpropan-2-ol (Compound 64)

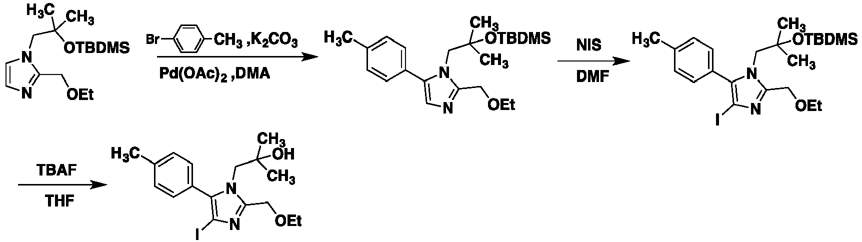

(1) The product 1-{2-[(tert-butyldimethylsilyl)oxy]-2-methylpropyl}-2-(ethoxymethyl)-1H-imidazole (313 mg, 1.00 mmol) synthesized in step (2) of Example 38 was weighed and added to a 25-mL round-bottom flask, m-bromotoluene (513 mg, 3.00 mmol), potassium carbonate (552 mg, 4.00 mmol) and palladium acetate (38 mg, 0.17 mmol) were added, and 10 mL of DMA was added as a solvent. The reaction system was repeatedly purged with nitrogen gas for 3 times and then protected with a nitrogen balloon, heated to 80°C, and stirred and reacted for 12h. Afterwards, a large amount of water was added to the reaction mixture, which was then extracted with ethyl acetate (3 × 100 mL), washed with saturated aqueous sodium chloride solution (200 mL), dried over anhydrous sodium sulfate, filtered and evaporated to dryness via rotary evaporation. The resultant was purified by flash SiO₂ chromatography (petroleum ether : ethyl acetate = 3 : 1), so as to obtain 1-(2-((tert-butyldimethylsilyl)oxy)-2-methylpropyl)-2-(ethoxymethyl)-5-(p-tolyl)-1H-imidazole as an orange liquid (20 mg, yield: 5%). ¹H NMR (400 MHz, chloroform-*d*) δ 7.28-7.19 (m, 4H), 6.97 (s, 1H), 4.79 (s, 2H), 4.22 (s, 2H), 3.58 (q, *J* = 7.1 Hz, 2H), 2.40 (s, 3H), 1.25 (t, *J* = 7.0 Hz, 3H), 0.94 (s, 6H), 0.83-0.77 (m, 9H). ¹³C NMR (101 MHz, chloroform-*d*) δ 147.28, 137.48, 134.47, 129.46, 128.80, 128.67, 127.03, 74.40, 65.83, 65.73, 54.91, 28.56, 25.95, 21.22, 18.02, 15.13, -2.05.
(2) 1-(2-((Tert-butyldimethylsilyl)oxy)-2-methylpropyl)-2-(ethoxymethyl)-5-(p-tolyl)-1H-imidazole (20 mg, 0.05 mmol) was weighed and added to a 25-mL round-bottom flask, N-iodosuccinimide (12.4 mg, 0.06 mmol) and DMF (as a solvent) were added successively, and the mixture was heated to 60°C and reacted for 12 hours. After the completion of the reaction was detected, TBAF (230 uL, with a concentration of 1M in tetrahydrofuran, 0.23 mmol) and tetrahydrofuran (5 mL) were added successively. The mixture was stirred at room temperature overnight, tetrahydrofuran was removed via rotary evaporation under reduced pressure, and purified water (50 mL) and ethyl acetate (3 × 50 mL) were added to conduct extraction. The resulting mixture was washed with saturated aqueous sodium chloride solution (100 mL), dried over anhydrous sodium sulfate, filtered and evaporated to dryness via rotary evaporation. The resultant was purified by flash SiO₂ chromatography (petroleum ether : ethyl acetate = 3 : 1), so as to obtain 1-(2-(ethoxymethyl)-4-iodo-5-(p-tolyl)-1H-imidazole-l-yl)-2-methylpropan-2-ol as a yellow solid (15 mg, yield: 72%). ¹H NMR (400 MHz, chloroform-*d*) δ 7.31 (s, 2H), 7.22 (d, *J* = 8.1 Hz, 2H), 4.69 (s, 2H), 4.14 (s, 2H), 3.69 (q, *J* = 7.0 Hz, 2H), 2.44 (s, 3H), 1.26 (t, *J* = 7.0 Hz, 3H), 0.93 (s, 6H). ¹³C NMR (101 MHz, chloroform-*d*) δ 147.22, 138.97, 137.04, 130.76, 129.53, 126.87, 84.33, 70.38, 66.25, 64.54, 55.56, 27.66, 21.42, 14.88.

### Biological Activity Experiment

Reagents, Materials and Instruments
DMEM medium: Gibco, Cat. No.: C11995500BT; RPMI medium: Gibco, Cat. No.: 10-040-CVR
Fetal bovine serum: Gibco, Cat. No.: 10099-141; Penicillin/Streptomycin Solution (100X): Corning, Cat. No.: 30-002-Cl
HEK Blue hTLR2 cell: InvivoGen, Cat. No.: hkb-htlr2
HEK Blue hTLR3 cell: InvivoGen, Cat. No.: hkb-htlr3
HEK Blue hTLR4 cell: InvivoGen, Cat. No.: hkb-htlr4
HEK Blue hTLR5 cell: InvivoGen, Cat. No.: hkb-htlr5
HEK Blue hTLR7 cell: InvivoGen, Cat. No.: hkb-htlr7
HEK Blue hTLR8 cell: InvivoGen, Cat. No.: hkb-htlr8
HEK Blue hTLR9 cell: InvivoGen, Cat. No.: hkb-htlr9
R848: Invivogen, Cat. No.: tlrl-r848-5; ssRNA40/LyoVec: InvivoGen, Cat. No.: tlrl-lrna40
ORN06/LyoVec: InvivoGen, Cat. No.: tlrl-orn6; Quanti-Blue: InvivoGen, Cat. No.: rep-qb2
Cell Counting Kit-8 (CCK-8): Bimake, Cat. No.: B34304
ploy (I:C) (polyribonucleic acid : polycytidylic acid): InvivoGen, Cat. No.: tlrl-pic-5 LPS (lipopolysaccharide): InvivoGen, Cat. No.: tlrl-3pelps
Pam3CSK4 (triacyl lipopeptide): InvivoGen, Cat. No.: tlrl-pms
Pam2CSK4 (diacyl lipopeptide): InvivoGen, Cat. No.: tlrl-pm2s-1
Flagellin: InvivoGen, Cat. No.: tlrl-bsfla; ODN2006: InvivoGen, Cat. No.: tlrl-2006-5 PMA (phorbol-12-myristic acid-13-acetate): Solarbio, Cat. No.: p6741-1 mg
2-mercaptoethanol: Solarbio, Cat. No.: 21985023; FBS buffer: Corning, Cat. No.: 21-040-CVR
Lymphocyte Separation Medium: Solarbio, Cat. No.: p8610-200 ml; RIPA lysis buffer: Biyuntian, Cat. No.: P0013B
Human IL-1β ELISA Kit: BD OptEIA, Cat. No.: 557953
Human IL-6 ELISA Kit: BD OptEIA, Cat. No.: 555220
Human TNF ELISA Kit: BD OptEIA, Cat. No.: 555212
96-well plate: Thermo Scientific, Cat. No.: 167008; 12-well plate: Thermo Scientific, Cat. No.: 150628
Multi-mode plate reader Varioskan Flash: Thermo Scientific, mode: N06354

### Activity Test of Compounds

HEK Blue hTLR8 cells or HEK Blue hTLR7 cells were cultured in DMEM medium supplemented with 10% fetal bovine serum, penicillin (100 U/mL) and streptomycin (100 mg/mL). Thereafter, cells were seeded in 96-well plate (45,000 cells per well), and were cultured for 24 hours in a humidified incubator (37°C, 5% CO₂).

After 24 hours, the supernatant and suspending cells were aspirated and replaced with fresh serum-free DMEM. Cells were treated with 1 µg/mL of R848 (agonist of TLR7/8) or 5 µg/mL of ssRNA40/LyoVec or 5µg/mL of ORN06/LyoVec (InvivoGen) and compounds with specified concentrations, and were placed in a humidified incubator (37°C, 5% CO₂) for co-culture.

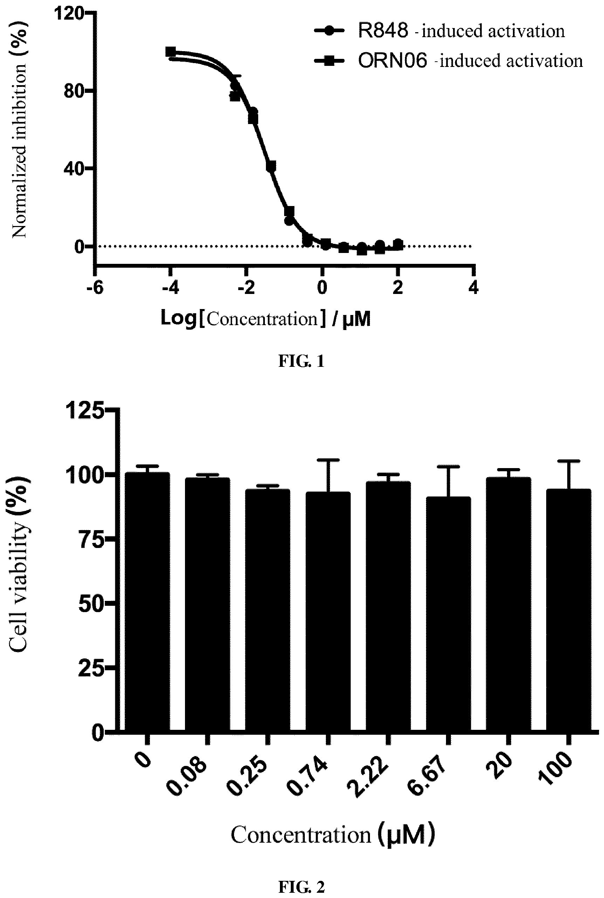

After 24 hours, 50 µL of the medium was aspirated from each well and then added to a new 96-well plate, followed by the addition of 50 µL of Quanti-Blue in each well and the incubation at 37°C in darkness. Changes in color would occur in the 96-well plate within 60 minutes, and the absorbance was measured at 620 nm by using the multi-mode plate reader Varioskan Flash. Inhibition rate (%) = [R848 (OD₆₂₀) - Compound (OD₆₂₀)]/[R848 (OD₆₂₀) - Control (OD₆₂₀)]×100%. Among these, the control group was only added with the same amount of cells. Finally, the software Prism 6.0 was utilized to plot a graph and calculate IC₅₀ values. Or, the activity of the cells treated with ligand and DMSO was defined as 100% activity, and the activity of the untreated cells was defined as 0% activity. The test results were as shown in Table 1, Table 2, Figure 1, Figure 10, and Figure 12 to Figure 14.

### Toxicity Test of Compounds

In the 96-well plate used for the above-mentioned activity test of compounds (remaining 50 µL of culture medium containing cells), 5 µL of CellCountingKit-8 (CCK-8) Supermix was added to each well. Note that the introduction of air bubbles into the wells should be avoided since the air bubbles would interfere with the detection of OD value.

The culture plate was incubated in an incubator for 1 to 4 hours.

The edge of the 96-well plate could be tapped gently before reading the plate, and then the absorbance at 450 nm was measured by using the multi-mode plate reader.

Cell viability (%) was determined using the following formula: cell viability (%) =

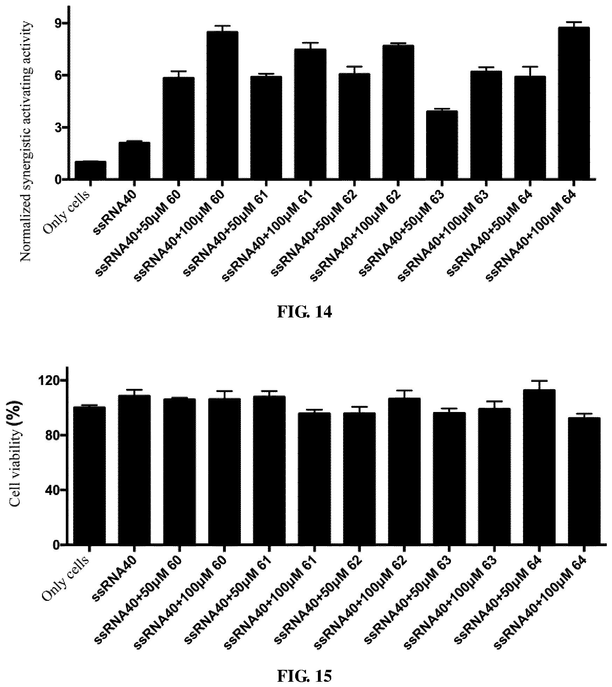

([Compound well (OD₄₅₀) - Background well (OD₄₅₀)]/[Control well (OD₄₅₀) - Background well (OD₄₅₀)]) ×100%. Among these, the background well was only added with the medium and CCK-8 reagent, the control well was only added with the medium, cells, R848 and CCK-8, and the compound wells were added with the medium, cells, R848, compounds in different concentrations, and CCK-8. The test results were as shown in Table 2, Figure 2 and Figure 15.

### Specificity Test of Compounds

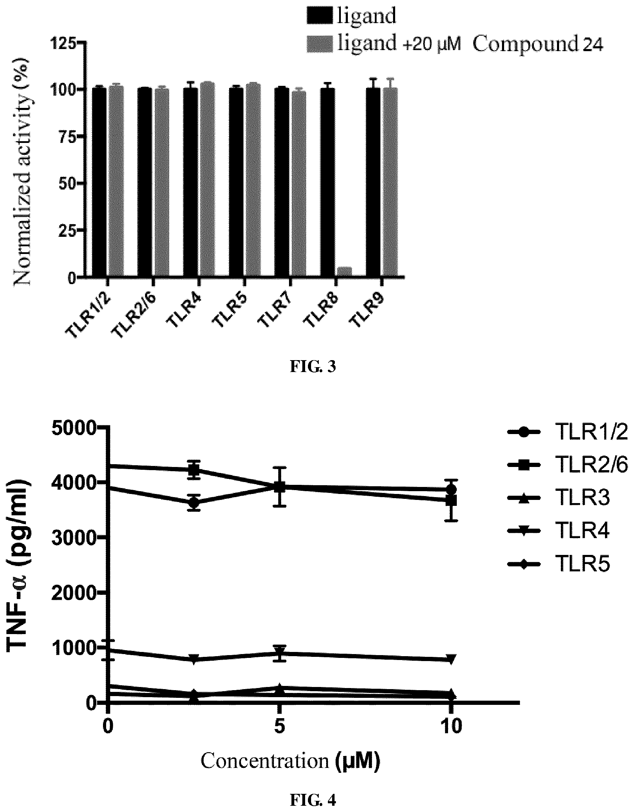

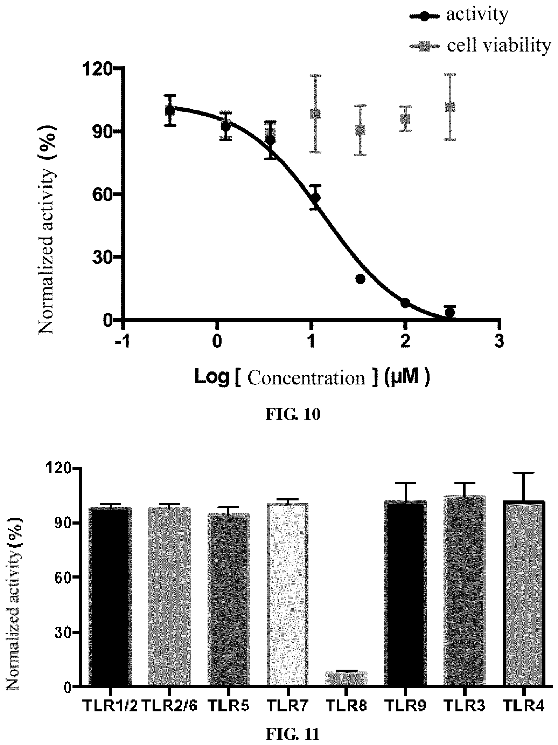

The selectivity of compounds for receptors in TLR family was tested in HEK-Blue cells overexpressing various TLR_{X} (x = 1 to 9) respectively. The steps of this test method were the same as those mentioned above in "Activity Test of Compounds", except that the ligands corresponding to different TLRs were used to activate the downstream signaling in the corresponding cells. Among these, ploy (I:C) (20 µg/mL), LPS (40 ng/mL), Pam3CSK4 (100 ng/mL), Pam2CSK4 (100 ng/mL), flagellin (200 ng/mL), R848 (1 µg/mL) and ODN2006 (0.5 µM) were used to selectively activate HEK-Blue hTLR3-, hTLR4-, hTLR1/2-, hTLR2/6-, hTLR5-, hTLR7- and hTLR9-overexpressing cells, respectively. The test results were as shown in Figure 3 and Figure 11.

### Enzyme-linked immunosorbent assay (ELISA)

THP-1 cells treated with 50 ng/mL of PMA were suspended in RPMI medium (containing 10% fetal bovine serum, penicillin (100 U/mL), streptomycin (100 mg/mL) and 0.05 mM 2-mercaptoethanol), seeded in a 12-well plate at a density of 1×10⁶ cells/well and 1 mL/well, and incubated in a cell incubator (37°C, 5% CO₂).

After 48 hours, suspending cells would adhere onto the surface of the 12-well plate, non-adhered cells and the medium were aspirated, and cells were washed with sterile FBS buffer (3 × 1 mL). RPMI medium was added (1 mL/well), and R848 (1 µg/mL) and various concentrations of Compound **24** or Compound **10** (as a negative control) were further added to treat cells. Or, the same amount of peripheral blood mononuclear cells derived from healthy human were used instead of THP-1 cells for subsequent experiments.

After 24 hours, the supernatant of the medium was collected and stored in a freezer at -80°C. Cells were washed with PBS (3 × 1 mL), the 12-well plate was placed on ice, and then 400 µL of lysis buffer (containing protease inhibitor) was added to each well, during which the cells were pipetted every five minutes. After 20 minutes, the mixture was transferred to the corresponding 1.5-mL tube and centrifuged at 13.2K rpm and 4°C for 20 minutes. 350 µL of the supernatant was collected into a new centrifuge tube and frozen at -80°C.

Cytokines interleukin-1β (IL-1β), interleukin-6 (IL-6) and tumor necrosis factor (TNF-α) were quantified by enzyme-linked immunosorbent assay (ELISA) according to the instructions of the kit provided by the manufacturer. The test results were as shown in Figure 4 to Figure 9.

### Test of samples collected from rheumatoid arthritis patients

Approximately 8 mL of blood was collected from untreated rheumatoid arthritis patients.

Peripheral blood mononuclear cells (PBMCs) were isolated from the whole blood derived from 6 rheumatoid arthritis patients using the lymphocyte separation medium, and the cells were seeded in a 96-well round-bottom plate at a density of 1×10⁶ cells/mL immediately after the isolation (cultured in RPMI 1640 (0.25 mL/well)).

Afterwards, cells were treated with 0, 10, 20, 40 and 80 µM Compound **24,** Compound **10** was used as a negative control, and cells were placed in a cell incubator (37°C, 5%CO₂) for incubation.

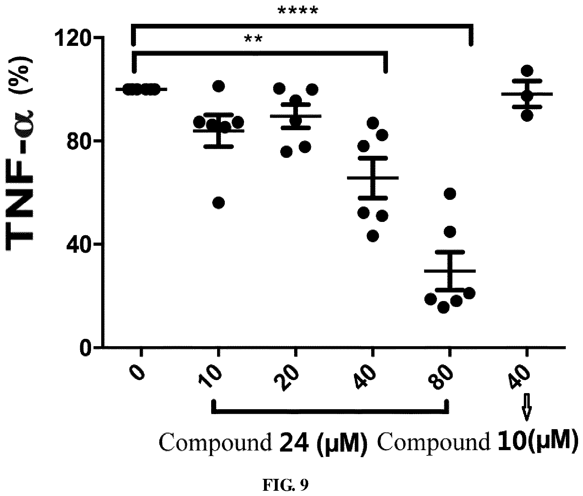

After 24 hours, the resultant was centrifuged at 4,000 rpm/minute for 10 minutes, and then the supernatant was collected, frozen to -80°C and used for the determination of TNF-α. Quantification was conducted using a human TNF ELISA Kit. The test results were as shown in Figure 9. Table 1 showed the data of the inhibitory effects of Compounds **1 to 37** on R848-induced signaling in HEK-Blue hTLR8 cell line, wherein IC₅₀ value and the corresponding standard deviations were at least biologically determined in triplicate, and N.T. denoted untested.

**Table 1**

| **Compound** | **Structural Formula** | **IC₅₀[µM]** | **Compound** | **Structural Formula** | **IC₅₀[µM]** |
|---|---|---|---|---|---|
| **1** | 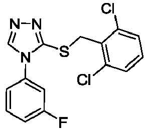 | 1.95 ± 0.19 | **2** | 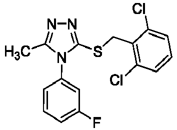 | 6.18 ± 0.29 |
| **3** | 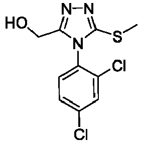 | 0.55 ± 0.07 | **4** | 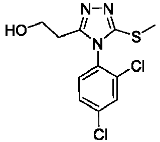 | 0.59 ± 0.03 |
| **5** | 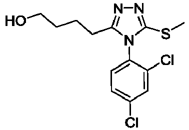 | 1.06 ± 0.10 | 6 | 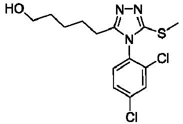 | 0.52 ± 0.05 |
| **7** | 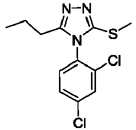 | 0.63 ± 0.02 | **8** | 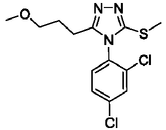 | 1.18 ± 0.14 |
| **9** | 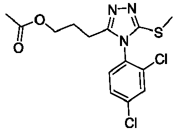 | 2.08 ± 0.10 | **10** | 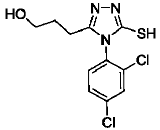 | N.T. |
| **11** | 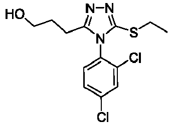 | 0.39 ± 0.04 | **12** | 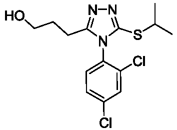 | 10.1 ± 0.8 |
| **13** | 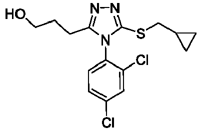 | 8.01 ± 0.13 | **14** | 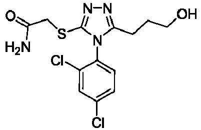 | N.T. |
| **15** | 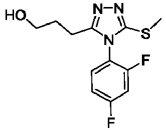 | 2.13 ± 0.56 | **16** | 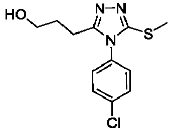 | 1.61 ± 0.12 |
| **17** | 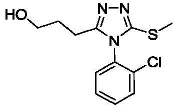 | 0.98 ± 0.14 | **18** | 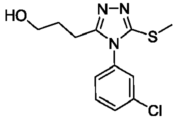 | 0.35 ± 0.02 |
| **19** | 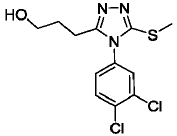 | 0.19 ± 0.01 | **20** | 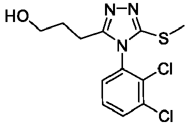 | 0.80 ± 0.07 |
| **21** | 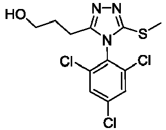 | 2.81 ± 0.44 | **22** | 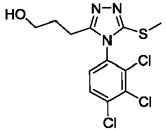 | 0.60 ± 0.10 |
| **23** | 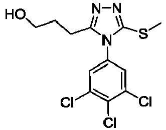 | 0.11 ± 0.01 | **24** | 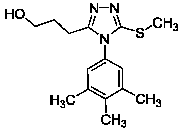 | 0.030±0.002 |
| **25** | 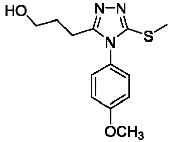 | 3.86 ± 0.31 | **26** | 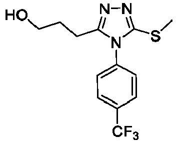 | 4.84 ± 0.82 |
| **27** | 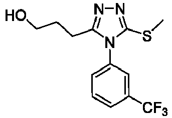 | 0.21 ± 0.02 | **28** | 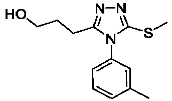 | 0.29 ± 0.03 |
| **29** | 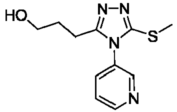 | N.T. | **30** | 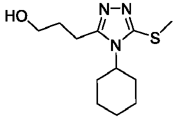 | N.T. |
| **31** | 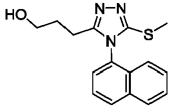 | 0.39 ± 0.06 | **32** | 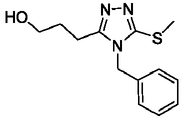 | N.T. |
| **33** | 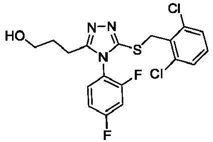 | 8.44 ± 0.78 | **34** | 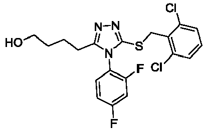 | 11.4 ± 0.7 |
| **35** | 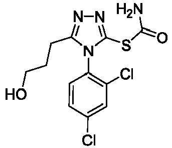 | N.T. | **36** | 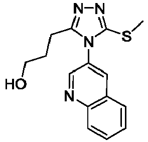 | 0.74 ± 0.02 |
| **37** | 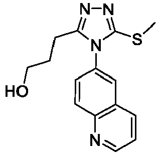 | 0.65 ± 0.01 | | | |

Table 2 showed the data of the normalized inhibitory effects of Compounds **38 to 64** on R848-induced signaling and the data of the normalized toxicity effects in HEK-Blue hTLR8 and TLR7 cell lines.

**Table 2**

| Compound | Structural Formula | TLR8 | TLR7 | WST1 |
|---|---|---|---|---|
| 38 | 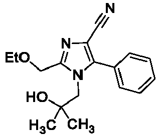 | 1.1±0.2 | 1.0±0.2 | 0.7±0.3 |
| 39 | 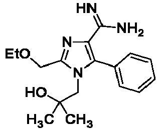 | 1.0±0.2 | 0.8±0.1 | 0.7±0.2 |
| 40 | 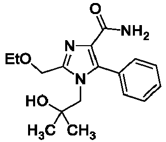 | 0.7±0.1 | 1.0±0.1 | 0.8±0.3 |
| 41 | 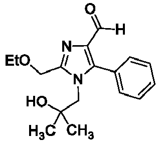 | 1.2±0.2 | 1.1±0.2 | 0.7±0.2 |
| 42 | 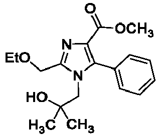 | 0.9±0.2 | 0.9±0.2 | 0.9±0.1 |
| 43 | 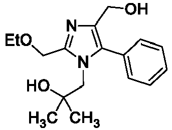 | 1.0±0.2 | 0.9±0.2 | 0.8±0.1 |
| 44 | 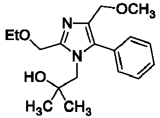 | 1.0±0.3 | 0.9±0.2 | 0.8±0.2 |
| 45 | 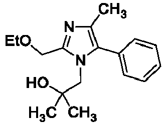 | 0.8±0.2 | 0.9±0.1 | 0.8±0.3 |
| 46 | 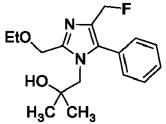 | 1.0±0.2 | 0.8±0.2 | 0.9±0.3 |
| 47 | 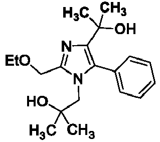 | 0.9±0.1 | 0.8±0.0 | N.T. |
| 48 | 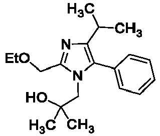 | 0.9±0.2 | 0.7±0.1 | 0.8±0.3 |
| 49 | 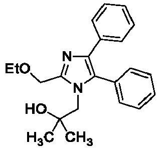 | 0.6±0.0 | 0.8±0.0 | N.T. |
| 50 | 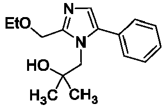 | 1.1±0.3 | 1.0±0.1 | 0.9±0.2 |
| 51 | 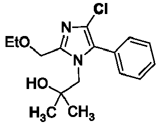 | 0.6±0.1 | 1.1±0.2 | 0.9±0.2 |
| 52 | 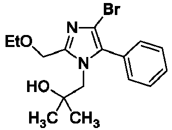 | 0.3±0.2 | 0.8±0.2 | 1.1±0.3 |
| 53 | 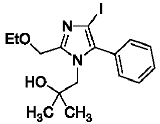 | 0.1±0.1 | 1.0±0.2 | 0.9±0.2 |
| 54 | 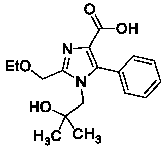 | 0.9±0.2 | 0.9±0.2 | 1.1±0.3 |
| 55 | 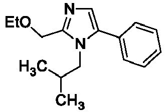 | 0.8±0.1 | 0.6±0.2 | 0.9±0.3 |
| 56 | 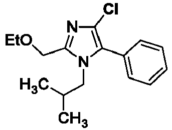 | 0.5±0.2 | 0.6±0.2 | 0.7±0.2 |
| 57 | 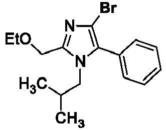 | 0.01±0.01 | 0.1±0.1 | 0.7±0.1 |
| 58 | 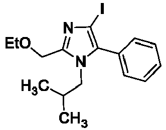 | 0.01±0.01 | 0.1±0.2 | 0.3±0.2 |
| 59 | 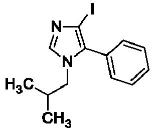 | 0.2±0.1 | 0.2±0.1 | N.T. |
| 60 | 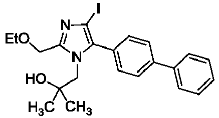 | 0.3±0.1 | 1.0±0.1 | 0.6±0.1 |
| 61 | 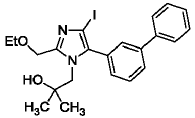 | 0.01±0.01 | 0.9±0.1 | 0.9±0.2 |
| 62 | 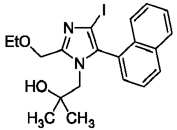 | 0.06±0.1 | 0.9±0.1 | 0.8±0.1 |
| 63 | 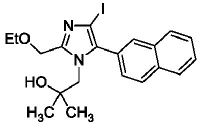 | 0.9±0.1 | 0.9±0.2 | 1.0±0.1 |
| 64 | 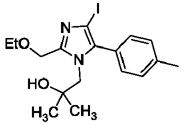 | 0.9±0.1 | 0.9±0.2 | 1.1±0.1 |

Figure 1 showed that Compound **24** had excellent inhibitory effect on both R848-induced signaling and ORN06-induced signaling in HEK-Blue hTLR8 cell line. Figure 2 showed that Compound **24** did not cause significant toxicity in HEK-Blue hTLR8 cell line even at a concentration of 100 µM.

Figure 3 showed that Compound **24** exerted specific inhibitory effect on HEK-Blue hTLR8 cell line at a concentration of 20 µM. Figure 4 showed that Compound **24** had no inhibitory effect on TNF-α in TLR1/2, TLR2/6, TLR3, TLR4 and TLR5 signaling pathways in human peripheral blood mononuclear cells, which also further proved that Compound **24** specifically exerted inhibitory effect on TLR8 signaling pathway.

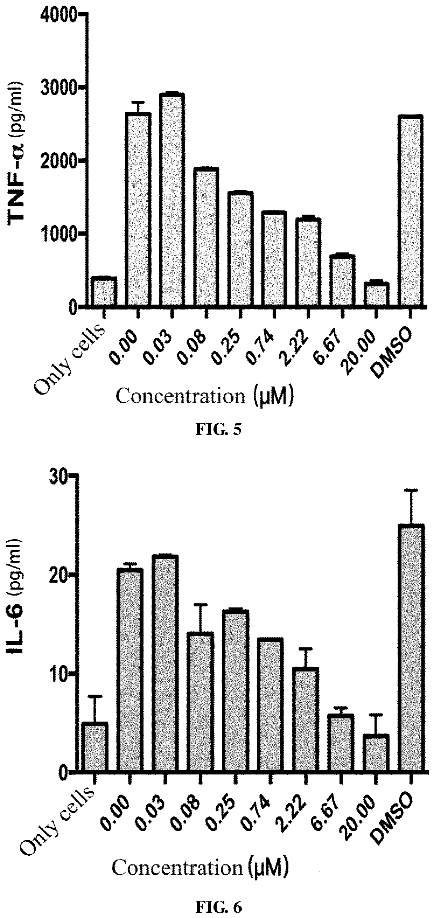

Figure 5 showed that Compound **24** exerted significant inhibitory effect on the inflammatory factor TNF-α in R848-induced signaling pathway in a concentration-dependent manner in THP-1 cells. Figure 6 showed that Compound **24** exerted significant inhibitory effect on the inflammatory factor IL-6 in R848-induced signaling pathway in a concentration-dependent manner in THP-1 cells.

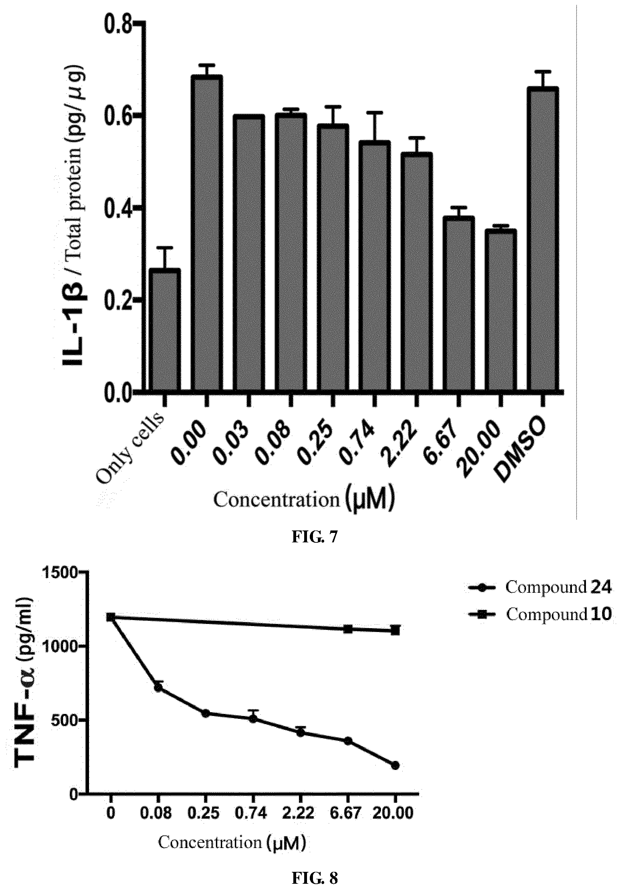

Figure 7 showed that Compound **24** exerted significant inhibitory effect on the inflammatory factor IL-1β in R848-induced signaling pathway in a concentration-dependent manner in THP-1 cells. Figure 8 showed that Compound **24** exerted significant inhibitory effect on the inflammatory factor TNF-α in R848-induced signaling pathway in human peripheral blood mononuclear cells, while Reference Compound **10** did not exert inhibitory effect.

Figure 9 showed that Compound **24** exerted significant inhibitory effect on the inflammatory factor TNF-α in R848-induced signaling pathway in the peripheral blood mononuclear cells derived from patients suffering from rheumatoid arthritis, while Reference Compound **10** did not show such effect.

Figure 10 showed that Compound **53** exerted significant inhibitory effect on R848-induced signaling in HEK-Blue hTLR8 cell line and Compound **53** exerted no influence on cell viability under the test conditions. Figure 11 showed that Compound **53** exerted specific inhibitory effect on TLR8 signaling pathway at a concentration of 100 µM while exerting no influence on other TLR signaling pathways.

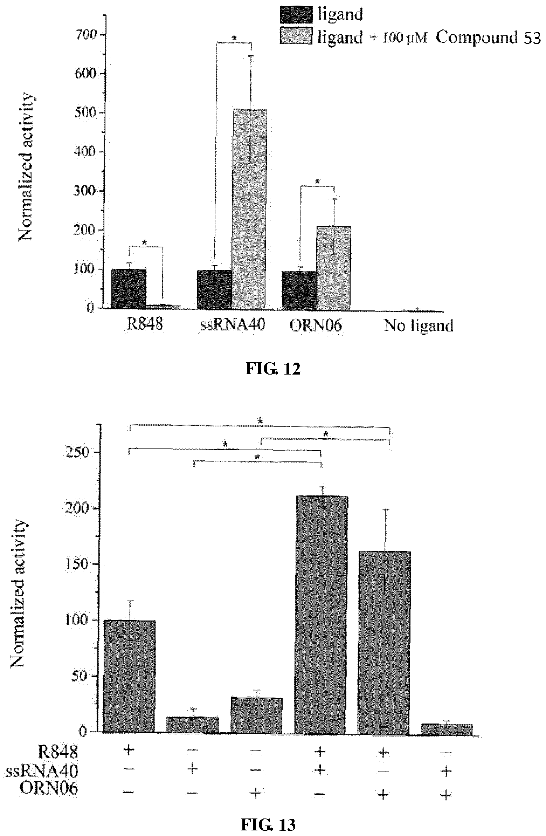

Figure 12 showed that Compound **53** exerted significant inhibitory effect on R848-induced signaling while exerting synergistic activating effect on ssRNA40-induced signaling and ORN06-induced signaling in HEK-Blue hTLR8 cell line at a concentration of 100 µM. Figure 13 showed that Reference Compound R848 was capable of activating the signaling pathway of TLR8 alone, and was also capable of activating the signaling pathway of TLR8 synergistically with ssRNA40 and ORN06.

Figure 14 showed that Compounds **60 to 64** were capable of exerting synergistic activating effect on ssRNA40-activated TLR8 signal at concentrations of 50 µM and 100 µM in HEK-Blue hTLR8 cell line. Figure 15 showed that Compounds **60 to 64** had little toxicity to cells at concentrations of 50 µM and 100 µM in HEK-Blue hTLR8 cell line.

## Claims

1. A compound of formula (I) or a pharmaceutically acceptable salt thereof, 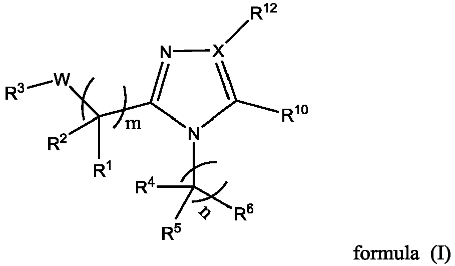 wherein,
X is CH or N;
W is O or CH₂;
m is 0, 1, 2, 3, 4, 5 or 6;
n is 0, 1, 2, 3, 4, 5 or 6;
R¹ and R² are each independently H, C₁-C₆ alkyl, or halogen, wherein said C₁-C₆ alkyl is optionally substituted by one or two or three substituents selected from halogen, hydroxyl, cyano, amino, or nitro;
R³ is H, C₁-C₆ alkyl, C₁-C₆ alkoxy, or C₁-C₆ alkylacyl, wherein said C₁-C₆ alkyl, C₁-C₆ alkoxy or C₁-C₆ alkylacyl is optionally substituted by one or two or three substituents selected from halogen, hydroxyl, cyano, amino, or nitro;
R⁴ and R⁵ are each independently H, C₁-C₆ alkyl, or 3-6 membered cycloalkyl, wherein said C₁-C₆ alkyl or 3-6 membered cycloalkyl is optionally substituted by one or two or three substituents selected from halogen, hydroxyl, cyano, amino, or nitro;
R⁶ is phenyl, pyridyl, naphthyl, quinolyl, isoquinolyl, 3-6 membered cycloalkyl, or CR⁷R⁸R⁹, said phenyl, pyridyl, naphthyl, quinolyl, isoquinolyl or 3-6 membered cycloalkyl is optionally substituted by one or two or three substituents selected from H, C₁-C₆ alkyl, C₁-C₆ alkenyl, C₁-C₆ alkynyl, C₁-C₆ alkoxy, 3-6 membered cycloalkyl, trifluoromethyl, halogen, hydroxyl, cyano, amino, or nitro;
R⁷ and R⁸ are each independently H, C₁-C₆ alkyl, or 3-6 membered cycloalkyl, wherein said C₁-C₆ alkyl or 3-6 membered cycloalkyl is optionally substituted by one or two or three substituents selected from halogen, hydroxyl, cyano, amino, or nitro;
R⁹ is OH or H;
R¹⁰ is phenyl, pyridyl, naphthyl, quinolyl, biphenyl, 3-6 membered cycloalkyl, or SR¹¹, said phenyl, pyridyl, naphthyl, quinolyl, biphenyl or 3-6 membered cycloalkyl is optionally substituted by one or two or three substituents selected from H, C₁-C₆ alkyl, C₁-C₆ alkenyl, C₁-C₆ alkynyl, C₁-C₆ alkoxy, 3-6 membered cycloalkyl, halogen, hydroxyl, cyano, amino, or nitro;
R¹¹ is H, C₁-C₆ alkyl, C₁-C₆ alkenyl, C₁-C₆ alkynyl, C₁-C₆ alkoxy, 3-6 membered cycloalkyl, benzyl, or amide group, wherein said C₁-C₆ alkyl, C₁-C₆ alkenyl, C₁-C₆ alkynyl, C₁-C₆ alkoxy, 3-6 membered cycloalkyl or benzyl is optionally substituted by one or two or three substituents selected from C₁-C₆ alkyl, C₁-C₆ alkenyl, C₁-C₆ alkynyl, C₁-C₆ alkoxy, 3-6 membered cycloalkyl, phenyl, halogen, hydroxyl, cyano, amino, amide group, or nitro;
R¹² is H, C₁-C₆ alkyl, C₁-C₆ alkenyl, C₁-C₆ alkynyl, C₁-C₆ alkoxy, C₁-C₆ alkylacyl, 3-6 membered cycloalkyl, phenyl, halogen, hydroxyl, cyano, amino, nitro, amidino, amide group, aldehyde group, ester group, or carboxyl group, wherein said C₁-C₆ alkyl, C₁-C₆ alkenyl, C₁-C₆ alkynyl, C₁-C₆ alkoxy, C₁-C₆ alkylacyl, 3-6 membered cycloalkyl or phenyl is optionally substituted by one or two or three substituents selected from C₁-C₆ alkyl, C₁-C₆ alkenyl, C₁-C₆ alkynyl, C₁-C₆ alkoxy, C₁-C₆ alkylacyl, 3-6 membered cycloalkyl, halogen, hydroxyl, cyano, amino, or nitro.

2. The compound or the pharmaceutically acceptable salt thereof according to claim 1, wherein R¹ and R² are each independently H, C₁-C₃ alkyl, fluorine, chlorine, bromine, or iodine, wherein said C₁-C₃ alkyl is optionally substituted by one or two or three substituents selected from fluorine, chlorine, bromine, iodine, hydroxyl, cyano, amino, or nitro; preferably, R¹ and R² are each independently H, methyl, ethyl, n-propyl, isopropyl, fluorine, or chlorine.

3. The compound or the pharmaceutically acceptable salt thereof according to claim 1 or 2, wherein R³ is H, C₁-C₃ alkyl, C₁-C₃ alkoxy, or C₁-C₃ alkylacyl, wherein said C₁-C₃ alkyl, C₁-C₃ alkoxy or C₁-C₃ alkylacyl is optionally substituted by one or two or three substituents selected from fluorine, chlorine, bromine, iodine, hydroxyl, cyano, amino, or nitro; preferably, R³ is H, methyl, ethyl, n-propyl, isopropyl, acetyl, or propionyl.

4. The compound or the pharmaceutically acceptable salt thereof according to any one of claims 1 to 3, wherein R⁴ and R⁵ are each independently H, C₁-C₃ alkyl, or 3-6 membered cycloalkyl, wherein said C₁-C₃ alkyl or 3-6 membered cycloalkyl is optionally substituted by one or two or three substituents selected from fluorine, chlorine, bromine, iodine, hydroxyl, cyano, amino, or nitro; preferably, R⁴ and R⁵ are each independently H, methyl, ethyl, n-propyl, isopropyl, cyclopentyl, or cyclohexyl.

5. The compound or the pharmaceutically acceptable salt thereof according to any one of claims 1 to 4, wherein R⁶ is phenyl, pyridyl, naphthyl, quinolyl, isoquinolyl, 3-6 membered cycloalkyl, or CR⁷R⁸R⁹, said phenyl, pyridyl, naphthyl, quinolyl, isoquinolyl or 3-6 membered cycloalkyl is optionally substituted by one or two or three substituents selected from H, C₁-C₃ alkyl, C₁-C₃ alkenyl, C₁-C₃ alkynyl, C₁-C₃ alkoxy, 3-6 membered cycloalkyl, trifluoromethyl, fluorine, chlorine, bromine, iodine, hydroxyl, cyano, amino, or nitro; preferably, R⁶ is phenyl, 2-chlorophenyl, 3-chlorophenyl, 4-chlorophenyl, 3-fluorophenyl, 2,4-difluorophenyl, 2,4-dichlorophenyl, 2,3-dichlorophenyl, 3,4-dichlorophenyl, 2,4,5-trichlorophenyl, 2,3,4-trichlorophenyl, 3,4,5-trichlorophenyl, 3,4,5-trimethylphenyl, 4-methoxyphenyl, 3-methylphenyl, 3-trifluoromethylphenyl, 4-trifluoromethylphenyl, 3-pyridyl, cyclohexyl, 1-naphthyl, 3-quinolyl, 6-quinolyl, 2-hydroxyprop-2-yl, or isopropyl.

6. The compound or the pharmaceutically acceptable salt thereof according to any one of claims 1 to 5, wherein R⁷ and R⁸ are each independently H, C₁-C₃ alkyl, or 3-6 membered cycloalkyl, wherein said C₁-C₃ alkyl or 3-6 membered cycloalkyl is optionally substituted by one or two or three substituents selected from fluorine, chlorine, bromine, iodine, hydroxyl, cyano, amino, or nitro; preferably, R⁷ and R⁸ are each independently H, methyl, ethyl, n-propyl, isopropyl, cyclopentyl, or cyclohexyl.

7. The compound or the pharmaceutically acceptable salt thereof according to any one of claims 1 to 6, wherein R¹⁰ is phenyl, pyridyl, naphthyl, quinolyl, biphenyl, 3-6 membered cycloalkyl, or SR¹¹, said phenyl, pyridyl, naphthyl, quinolyl, biphenyl or 3-6 membered cycloalkyl is optionally substituted by one or two or three substituents selected from H, C₁-C₃ alkyl, C₁-C₃ alkenyl, C₁-C₃ alkynyl, C₁-C₃ alkoxy, 3-6 membered cycloalkyl, fluorine, chlorine, bromine, iodine, hydroxyl, cyano, amino, or nitro; preferably, R¹⁰ is phenyl, p-methylphenyl, pyridyl, naphthyl, quinolyl, biphenyl, mercapto, methylthio, ethylthio, isopropylthio, (cyclopropylmethyl)thio, 2,6-dichlorobenzylthio, formamidethio, or acetamidethio.

8. The compound or the pharmaceutically acceptable salt thereof according to any one of claims 1 to 7, wherein R¹¹ is H, C₁-C₃ alkyl, C₁-C₃ alkenyl, C₁-C₃ alkynyl, C₁-C₃ alkoxy, 3-6 membered cycloalkyl, benzyl, or amide group, wherein said C₁-C₃ alkyl, C₁-C₃ alkenyl, C₁-C₃ alkynyl, C₁-C₃ alkoxy, 3-6 membered cycloalkyl or benzyl is optionally substituted by one or two or three substituents selected from C₁-C₃ alkyl, C₁-C₃ alkenyl, C₁-C₃ alkynyl, C₁-C₃ alkoxy, 3-6 membered cycloalkyl, fluorine, chlorine, bromine, iodine, hydroxyl, cyano, amino, amide group or nitro; preferably, R¹¹ is H, methyl, ethyl, isopropyl, cyclopropylmethyl, carboxamide group, acetamide group, or 2,6-dichlorobenzyl.

9. The compound or the pharmaceutically acceptable salt thereof according to any one of claims 1 to 8, wherein R¹² is H, C₁-C₃ alkyl, C₁-C₃ alkenyl, C₁-C₃ alkynyl, C₁-C₃ alkoxy, C₁-C₃ alkylacyl, 3-6 membered cycloalkyl, phenyl, fluorine, chlorine, bromine, iodine, hydroxyl, cyano, amino, nitro, amidino, amide group, aldehyde group, ester group, or carboxyl group, wherein said C₁-C₃ alkyl, C₁-C₃ alkenyl, C₁-C₃ alkynyl, C₁-C₃ alkoxy, C₁-C₃ alkylacyl, 3-6 membered cycloalkyl or phenyl is optionally substituted by one or two or three substituents selected from C₁-C₃ alkyl, C₁-C₃ alkenyl, C₁-C₃ alkynyl, C₁-C₃ alkoxy, C₁-C₃ alkylacyl, 3-6 membered cycloalkyl, fluorine, chlorine, bromine, iodine, hydroxyl, cyano, amino, or nitro; preferably, R¹² is H, cyano, amidino, amide group, aldehyde group, methoxycarbonyl, hydroxymethyl, methoxymethyl, methyl, fluoromethyl, 2-hydroxyprop-2-yl, isopropyl, phenyl, chlorine, bromine, iodine, or carboxyl group.

10. The compound or the pharmaceutically acceptable salt thereof according to any one of claims 1 to 9, wherein R¹ and R² are independently H, methyl, ethyl, n-propyl, isopropyl, fluorine, or chlorine; R³ is H, methyl, ethyl, n-propyl, isopropyl, acetyl, or propionyl; R⁴ and R⁵ are independently H, methyl, ethyl, n-propyl, isopropyl, cyclopentyl, or cyclohexyl; R⁶ is phenyl, 2-chlorophenyl, 3-chlorophenyl, 4-chlorophenyl, 3-fluorophenyl, 2,4-difluorophenyl, 2,4-dichlorophenyl, 2,3-dichlorophenyl, 3,4-dichlorophenyl, 2,4,5-trichlorophenyl, 2,3,4-trichlorophenyl, 3,4,5-trichlorophenyl, 3,4,5-trimethylphenyl, 4-methoxyphenyl, 3-methylphenyl, 3-trifluoromethylphenyl, 4-trifluoromethylphenyl, 3-pyridyl, cyclohexyl, 1-naphthyl, 3-quinolyl, 6-quinolyl, 2-hydroxyprop-2-yl, or isopropyl; R⁷ and R⁸ are independently H, methyl, ethyl, n-propyl, isopropyl, cyclopentyl, or cyclohexyl; R⁹ is OH or H; R¹⁰ is phenyl, p-methylphenyl, pyridyl, naphthyl, quinolyl, biphenyl, mercapto, methylthio, ethylthio, isopropylthio, (cyclopropylmethyl)thio, 2,6-dichlorobenzylthio, formamidethio, or acetamidethio; R¹¹ is H, methyl, ethyl, isopropyl, cyclopropylmethyl, carboxamide group, acetamide group, or 2,6-dichlorobenzyl; R¹² is H, cyano, amidino, amide group, aldehyde group, methoxycarbonyl, hydroxymethyl, methoxymethyl, methyl, fluoromethyl, 2-hydroxyprop-2-yl, isopropyl, phenyl, chlorine, bromine, iodine, or carboxyl group .

11. The compound or the pharmaceutically acceptable salt thereof according to claim 1, wherein the compound of formula (I) has a structure represented by formula (VII), 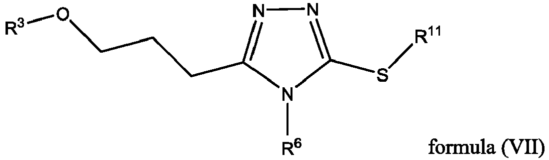 wherein R³, R⁶ and R¹¹ are as defined in claim 1.

12. The compound or the pharmaceutically acceptable salt thereof according to claim 1, wherein the compound of formula (I) has a structure represented by formula (X), 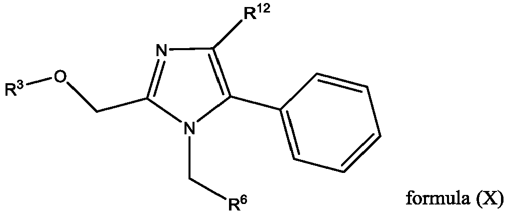 wherein R³, R⁶ and R¹² are as defined in claim 1.

13. The compound or the pharmaceutically acceptable salt thereof according to claim 1, wherein the compound comprises:

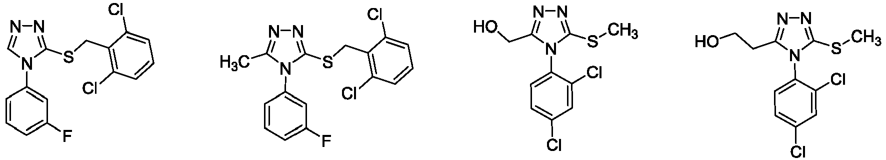

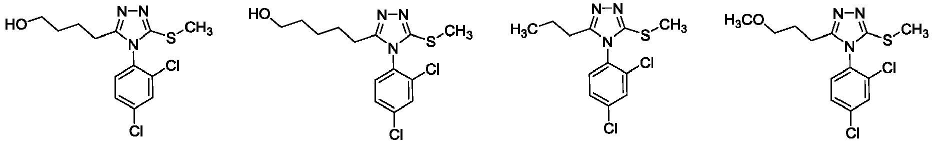

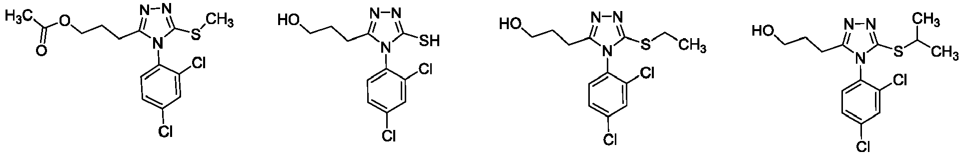

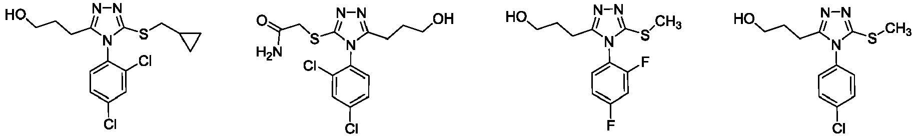

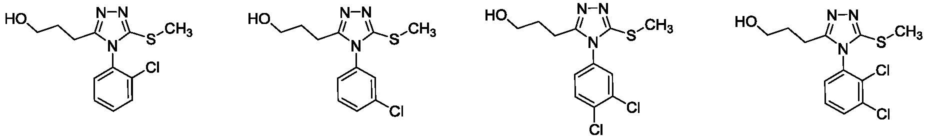

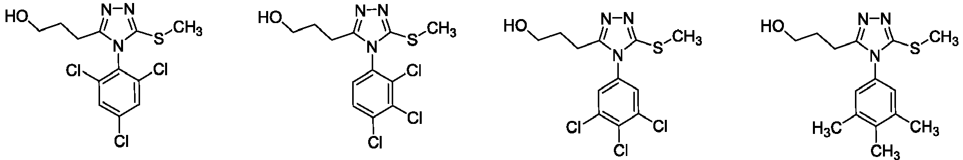

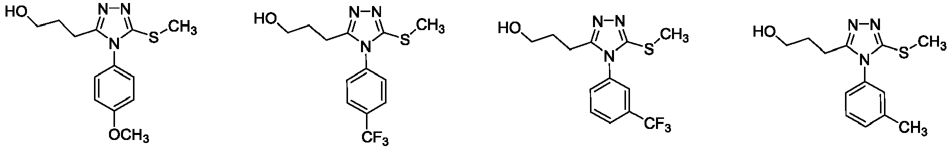

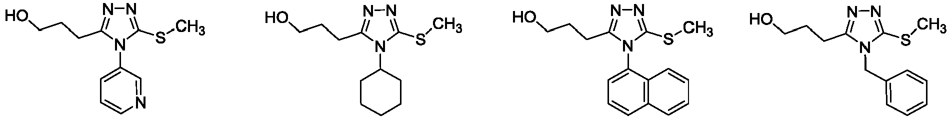

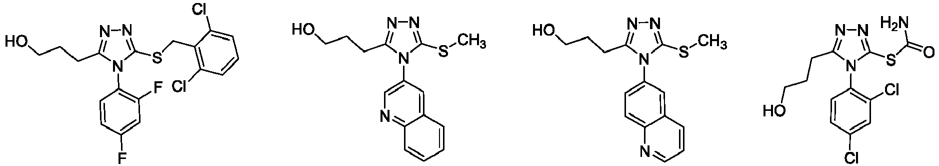

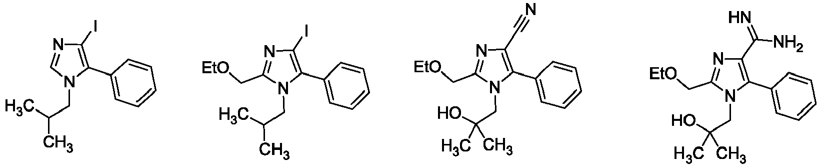

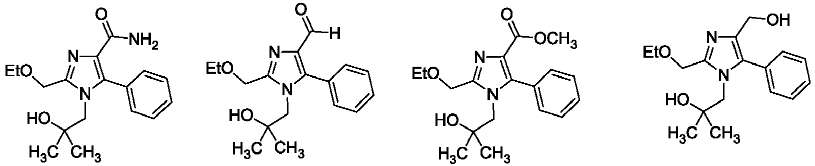

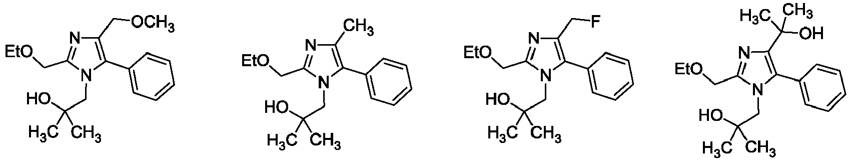

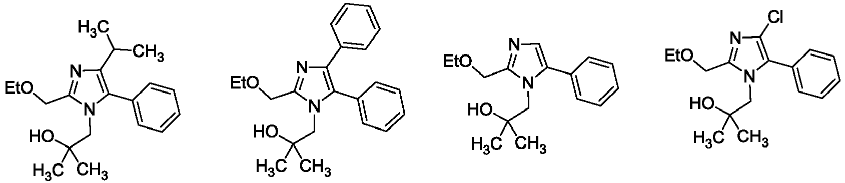

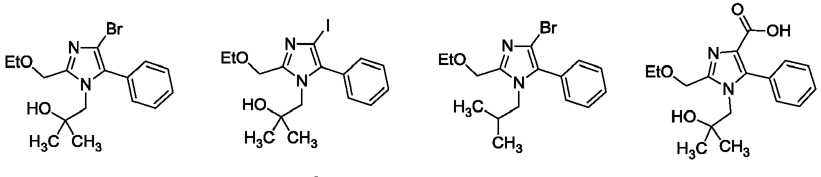

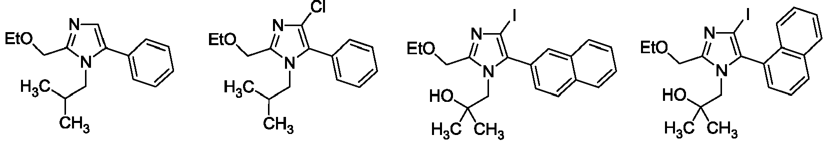

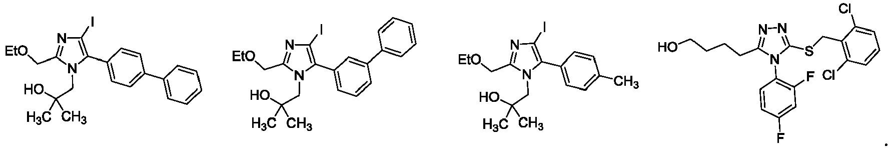

14. A pharmaceutical composition comprising the compound or the pharmaceutically acceptable salt thereof of any one of claims 1 to 12 and a pharmaceutically acceptable excipient.

15. Use of the compound or the pharmaceutically acceptable salt thereof of any one of claims 1 to 12 in preparation of a medicament for preventing or treating a TLR8-mediated disease, preferably, said TLR8-mediated disease is rheumatoid arthritis, inflammatory bowel disease, Alzheimer's syndrome, systemic lupus erythematosus, or Adult Still's disease.
